# EUROPÄISCHE PATENTSCHRIFT

(11) **EP 2 774 988 B1**
(45) Veröffentlichungstag und Bekanntmachung des Hinweises auf die Patenterteilung: **02.05.2018**
(21) Anmeldenummer: 14157651.2
(22) Anmeldetag: 04.03.2014
(51) Int. Cl.: C12N 15/11

(54) **Fluorchinolone-spezifische Aptamere**
Fluoroquinolone-specific aptamers
Aptamères spécifiques au fluorchinolone

(30) Priorität: 08.03.2013 DE 102013204057
(43) Veröffentlichungstag der Anmeldung: 10.09.2014
(73) Patentinhaber: Helmholtz-Zentrum für Umweltforschung GmbH-UFZ, 04318 Leipzig (DE)
(72) Erfinder: Reinemann, Christine, 04275 Leipzig (DE); Strehlitz, Beate, 04279 Leipzig (DE); Stoltenburg, Regina, 04157 Leipzig (DE)
(74) Vertreter: Beyer, Andreas

(56) Entgegenhaltungen:
- WO-A1-2012/130948
- KR-A- 20120 033 801
- LI S ET AL: "Screening of pefloxacin-binding single strand DNA aptamer", JOURNAL OF BIOTECHNOLOGY, ELSEVIER SCIENCE PUBLISHERS, AMSTERDAM, NL, Bd. 136, 1. Oktober 2008 (2008-10-01), Seite S86, XP026830117, ISSN: 0168-1656 [gefunden am 2008-10-01]
- CAO ET AL: "Surface plasmon resonance biosensor for enrofloxacin based on deoxyribonucleic acid", ANALYTICA CHIMICA ACTA, ELSEVIER, AMSTERDAM, NL, Bd. 589, Nr. 1, 18. April 2007 (2007-04-18), Seiten 1-5, XP022209487, ISSN: 0003-2670, DOI: 10.1016/J.ACA.2007.02.034
- BEATE STREHLITZ ET AL: "Aptamers for pharmaceuticals and their application in environmental analytics", BIOANALYTICAL REVIEWS, Bd. 4, Nr. 1, 17. Dezember 2011 (2011-12-17), Seiten 1-30, XP055120300, ISSN: 1867-2086, DOI: 10.1007/s12566-011-0026-1

## Beschreibung

Die vorliegende Erfindung betrifft ein Aptamer, das an eine oder mehrere Verbindungen aus der Gruppe der Fluorchinolone, bindet, Verwendungen des Aptamers sowie Verfahren zum Nachweis und Anreicherung von Verbindungen aus der Gruppe der Fluorchinolone, in denen das Aptamer eingesetzt wird.

Bei den Fluorchinolonen handelt es sich um eine Gruppe synthetisch hergestellter Antibiotika, die seit rund 15 Jahren weltweit eingesetzt werden. Chemisch gesehen leiten sich Chinolone in ihrer Struktur von Chinolin ab, welches am stickstoffhaltigen Ringsystem eine Carbonylgruppe sowie eine Carbonsäuregruppe trägt. Fluorchinolone sind im Grundgerüst fluoriert und weisen zusätzlich einen Piperazinsubstituenten auf. Sie gelten als besonders wertvoll, weil sie bei einem breiten Spektrum von Krankheitskeimen (Shigellen, Salmonellen, E. coli, Klebsiellen und andere Enterobacteriaceen) wirken und vergleichsweise wenig unerwünschte Nebenwirkungen verursachen. Besonders häufig eingesetzte Vertreter der Fluorchinolone sind: Ciprofloxacin, Ofloxacin, Enrofloxacin und Moxifloxacin. Fluorchinolone gelten als "Reserveantibiotika". Seit den 90er Jahren sind Fluorchinolone auch als Tierarzneimittel zugelassen. In Deutschland werden sie bei Schweinen, Rindern und vor allem bei Geflügel eingesetzt. Die Anwendung erfolgt sowohl am Einzeltier als auch für den Bestand über das Trinkwasser. Speziell diese Anwendungsform gilt im Hinblick auf die Resistenzausbildung als kritisch, weil in großem Umfang auch Tiere behandelt werden, die (noch) nicht krank sind. Seit der Zulassung von Fluorchinolonen für die Anwendung am Tier und insbesondere beim Nutzgeflügel ist ein Anstieg resistenter Keime sowohl beim Tier als auch beim Menschen beobachtet worden (siehe: Annual report of the European Antimicrobial Resistance Surveillance Network (EARS-Net) von 2009). Zudem sind Fluorchinolone als umweltproblematisch eingestuft worden.

Fluorchinolone gelangen u.a. über das Abwasser in die Umwelt. Sie werden in Kläranlagen nicht vollständig abgebaut. Daher können sie auch über das Oberflächen- und Grundwasser in das Trinkwasser gelangen. Eine gezielte Entfernung von Antibiotikareststoffen aus Kläranlagenabläufen, Gülle oder Wässern aus z.B. der Tierhaltung erfolgt derzeit nicht. Der Gehalt an Medikamentenreststoffen wird aber zunehmend als Problem erkannt. Die Entfernung von Fluorchinolonen, insbesondere von Ciprofloxacin, Ofloxacin und Enrofloxacin, aus den verschiedenen Wässern, sowie die Bestimmung oder Messung von Fluorchinolon-Kontaminationen in der Umwelt, sind daher sehr wichtig.

Li et al., "Screening of pefloxacin-bindung single strand DNA aptamer", Journal of Biotechnology, Elsevier, Amsterdam, NL, Bd. 136, 1. Oktober 2008, S. 86 (ISSN: 0168-1656) offenbaren ein Pefloxacin-bindendes DNA Aptamer, ohne dass dort Sequenz und Eigenschaften gezeigt sind.

Cao et al., "Surface plasmon resonance biosensor for enrofloxacin based on deoxyribonucleic acid", Analytica Chimica Acta, Elsevier, Amsterdam, NL, Bd. 589, Nr. 1, 18. April 2007, S. 1-5 (ISSN: 0003-2670) zeigt, dass Herings-Sperma DNA an Enrofloxacin bindet. Es wird in dieser Publikation nicht ausgesagt, ob einzelsträngige DNA aus Heringssperma an Enrofloxacin bindet, oder ob doppelsträngige DNA vorliegt.

WO 2012/130948 A1 offenbart Aptamere, die an Aminoglycoside binden.

Aufgabe der vorliegenden Erfindung war es, spezifische Substanzen zur Verfügung zu stellen, die einen schnellen, einfachen und zuverlässigen Nachweis von Verbindungen aus der Gruppe der Fluorchinolone ermöglichen.

Die Aufgabe wird mit einem Aptamer nach Anspruch 1 gelöst, das das an eine oder mehrere Verbindungen aus der Gruppe der Fluorchinolone bindet, Insbesondere ist das erfindungsgemäße Aptamer spezifisch für Verbindungen aus der Gruppe der Fluorchinolone, Das erfindungsgemäße Aptamer ist ein DNA-Aptamer, das heißt ein einzelsträngiges DNA(ssDNA)-Aptamer.

Bevorzugte Verbindungen aus der Gruppe der Fluorchinolone sind Enoxacin, Norfloxacin, Ciprofloxacin, Ofloxacin, Levofloxacin, Dextrofloxacin, Moxifloxacin, Nadifloxacin, Lomefloxacin, Fleroxacin, Gatifloxacin, Grepafloxacin, Pefloxacin, Sparfloxacin, Temafloxacin, Trovafloxacin, Danofloxacin, Enrofloxacin, Ibafloxacin, Marbofloxacin, und Orbifloxacin, mehr bevorzugt Ciprofloxacin, Ofloxacin, Levofloxacin, Dextrofloxacin, Enrofloxacin, Moxifloxacin, Marbofloxacin und Pefloxacin.

Offenbart von der Erfindung sind Aptamere, die besonders spezifisch an eine dieser speziellen Verbindungen binden, oder an eine beliebige Auswahl aus diesen genannten Gruppen von Verbindungen binden. Ofloxacin ist ein Racemat aus den Enantiomeren Levofloxacin und Dextrofloxacin. Angegeben von der Erfindung werden beispielsweise Aptamere, die entweder stärker an Levofloxacin oder stärker an Dextrofloxacin binden.

Die Erfindung stellt unter anderem ein einfaches Testsystem, ein Assay und einen Biosensor auf Basis des erfindungsgemäßen Aptamers für Fluorchinolone bereit. Dadurch ist es möglich, Antibiotika aus dieser Gruppe einfach und schnell vor Ort zu messen. (Bisher erfolgen die Messungen von Antibiotikareststoffen mit aufwändigen Methoden im Labor wie GC/MS oder HPLC, teilweise kombiniert mit fluorimetrischen Messungen, wofür teure Messgeräte und gut ausgebildetes Personal erforderlich sind und die zudem den Transport der Proben in das Labor erfordern.) Biosensoren auf Basis von Fluorchinolon-Aptameren können u.a. zur Verfolgung der Reinigungsleistung von Kläranlagen verwendet werden. Zudem kann eine Anwendung des Fluorchinolon-Aptamers zur Anreicherung, Abtrennung oder Entfernung von Fluorchinolonen in Separations-Systemen (z.B. biomagnetische Separation) oder mit spezifischen Filtermaterialien dazu beitragen den Eintrag dieser Antibiotikaklasse in die Umwelt zu vermindern.

Der allgemeine Begriff "Aptamere" bezeichnet im Stand der Technik kurze einzelsträngige Nukleinsäure-Oligomere, auch bezeichnet als Oligonukleotide, die spezifisch an eine Zielstruktur oder ein Zielmolekül, auch bezeichnet als Target, binden können, beispielsweise an ein Protein, an niedermolekulare Verbindungen, wie organische Substanzen, Aminosäuren und Antibiotika, Nukleinsäuren, Viruspartikel oder (Mikro)Organismen. Die Aptamer-Target-Bindung erfolgt beispielsweise über die Strukturkompatibilität, so genannte "Stacking interactions" bei aromatischen Ringstrukturen (Stapelkräfte durch Elektronenwechselwirkung mit Nachbarbasen), elektrostatische Wechselwirkungen (z.B. Van der Waals-, Ionen-, Dipolkräfte) und Wasserstoffbrückenbindungen.

Auch Aptamere mit Peptidstruktur sind bekannt, wobei sich die vorliegende Erfindung ausschließlich auf Nukleinsäure-Aptamere bezieht. Somit bezeichnet der Begriff "Aptamere" nachfolgend Nukleinsäure-Aptamere. Bei Nukleinsäure-Aptameren unterscheidet man beispielsweise DNA-Aptamere, gebildet aus einzelsträngiger DNA (ssDNA), und RNA-Aptamere. Aptamere zeichnen sich durch die Ausbildung einer spezifischen dreidimensionalen Struktur, die von der Nukleinsäuresequenz abhängt, aus. Diese Struktur befähigt Aptamere, analog einer Antigen-Antikörper-Bindung Zielstrukturen passgenau zu binden. Eine bestimmte Nukleinsäuresequenz eines Aptamers kann bei definierten Bedingungen eine dreidimensionale Struktur aufweisen, die spezifisch für eine definierte Zielstruktur (Target) ist. Die dreidimensionale Struktur eines Aptamers entsteht u.a. infolge von intramolekularen Basenpaarungen nach Watson und Crick und über Hoogsteen-Basenpaarungen (Quadruplex).

Erfindungsgemäße Aptamere können beispielsweise mit dem SELEX-Verfahren (Systematic Evolution of Ligands by Exponential Enrichment) erhalten werden. Grundlegende Arbeiten zum SELEX-Verfahren stammen von Tuerk and Gold, Science 249 (1990) 505-510, sowie Ellington and Szostak, Nature 346 (1990) 818-822. SELEX Verfahren sind weiterhin offenbart in US 5,567,588 und US 5,270,163.

Im SELEX Verfahren wird zunächst eine kombinatorische Zufallsbibliothek, bestehend aus einzelsträngigen DNA (ssDNA)-Oligonukleotiden erzeugt. Die Oligonukleotide weisen einen internen variablen Bereich auf, mit beispielsweise 40-60 Nukleotiden, der am 5' und 3' Ende von Primerregionen flankiert wird. Die Primerregionen dienen als Primerbindungsstellen für eine PCR Amplifikation. Kombinatorische Zufallsbibliotheken können von kommerziellen Anbietern bezogen werden. Die Variabilität einer Bibliothek liegt beispielsweise im Bereich von ca. 10¹⁵ verschiedenen Molekülen. Ausgehend von der einzelstängigen DNA-Oligonukleotid-Bibiothek werden über verschiedene Selektions- und Amplifikationsschritte zyklusweise die Oligonukleotide angereichert, die am besten an das Target binden. Jeder Zyklus besteht aus folgenden Teilschritten:
a) Bindung der Oligonukleotide an das Target,
b) Waschen der Oligonukleotid-Target-Komplexe zur Entfernung von ungebundenen Oligonukleotiden,
c) Elution der am Target gebundenen Oligonukleotide,
d) Amplifikation der eluierten Oligonukleotide mittels PCR,
e) Reinigung der relevanten ssDNA-Oligonukleotide aus dem PCR-Produkt

Nach jedem Zyklus wird der selektierte und angereicherte Oligonukleotidpool als Ausgangsmaterial für einen nächsten Zyklus genutzt. Vorteilhafterweise werden 8 bis 12 Zyklen durchlaufen.

Ein SELEX Verfahren zur Isolierung erfindungsgemäßer Aptamere ist in den beigefügten Beispielen genauer beschrieben. Ein beispielhaftes Verfahren zur Auffindung von erfindungsgemäßen Aptameren ist das sogenannte Capture-SELEX Verfahren, das in Stoltenburg, R.; Nikolaus, N.; Strehlitz, B. (2012) Capture-SELEX - selection of DNA aptamers for aminoglycoside antibiotics, Journal of Analytical Methods in Chemistry, Volume 2012 (2012), Article ID 415697, sowie in den beigefügten Beispielen beschrieben ist.

Nach Analyse der Sequenz können erfindungsgemäße Aptamere sowie Varianten, Mutanten, Fragmente und Derivate davon, welche nachfolgend noch beschrieben werden, mit üblichen Techniken der chemischen DNA- Synthese hergestellt werden, die dem Fachmann bekannt sind. Ist die Sequenz eines DNA Aptamers bekannt, so kann ein RNA Aptamer mit der gleichen Sequenz hergestellt werden. Ferner können die Bindungseigenschaften einzelner Aptamere an das Target untersucht werden.

Vorzugsweise sind die erfindungsgemäßen Aptamere synthetische Oligonukleotide, die vorzugsweise nach dem soeben beschriebenen SELEX Verfahren aus einer zugrunde gelegten synthetischen, kombinatorischen Oligonukleotid-Bibliothek selektiert wurden.

Die Erfindung betrifft ein DNA-Aptamer, das an eine oder mehrere Verbindungen aus der Gruppe der Fluorchinolone, bindet und das ausgewählt ist aus der Gruppe bestehend aus
a) einem Aptamer umfassend, oder bestehend aus, eine(r) Nukleinsäuresequenz, die ausgewählt ist aus der Gruppe bestehend aus SEQ ID NO: 14, 20, 36, 30, 37, 33, 25, 22, 39, 27, 41, 43 und 52,
b) einem Aptamer, dessen Nukleinsäuresequenz eine Identität von mindestens 98% mit der Nukleinsäuresequenz eines Aptamers aus a) aufweist
c) einem Aptamer bei dem gegenüber einem Aptamer aus a) bei der Sequenz mit der SEQ ID NO: 14, 20, 36, 30, 37, 33, 25, 22, 39, 27, 41, 43 oder 52 bis zu 2 Nukleotide substituiert, deletiert und/oder insertiert sind,
d) einem Aptamer bei dem gegenüber einem Aptamer aus a) bei der Sequenz mit einer der SEQ ID NO: 14, 20, 36, 30, 37, 33, 25, 22, 39, 27, 41, 43 und 52 bis zu 20 Nukleotide am 3'-Ende und/oder am 5'-Ende angefügt sind,
e) einem Fragment eines Aptamers nach a), b) oder c), das mindestens 30 Nukleotide aufweist, und
f) einem Derivat eines Aptamers nach a), b), c), d) oder e), wobei ein Aptamer nach a), b), c), d) oder e) an der Nukleobase, an der Pentose oder am Phosphat-Rückgrat chemisch modifiziert ist und eine chemische Struktur aufweist, die in natürlicher DNA oder RNA nicht vorkommt.

Die Funktionalität der Aptamere aus a) - f), also die Bindung an Verbindungen aus der Gruppe der Fluorchinolone kann mit einer Sekundärstrukturanalyse der Aptamere, abgeschätzt werden. Auch die Funktionalität sonstiger in dieser Beschreibung genannter Varianten kann so abgeschätzt werden. Die Modellierung der möglichen Sekundärstruktur der Aptamere kann unter Nutzung des im Internet frei verfügbaren Programms "mfold" (Version 3.1 oder 3.5) erfolgen. Dieses Programm führt eine Analyse der zweidimensionalen Struktur mit Hilfe einer Energieminimierungsmethode aus (Zuker M., 2003, Nucleic Acid Research 31, 3406-3415). Bei der Faltung der Aptamere kann es zu Stems (doppelsträngige Bereiche), Loops, d.h. Ausschleifungen von einzelsträngigen Bereichen, wie Haarnadel- oder interne Schleifen, und Bulbs, d.h. kleine einzelsträngige Ausbuchtungen in doppelsträngigen Bereichen, kommen. Mit dem Programm mfold wird für eine Aptamer-Nukleotidsequenz die hypothetische Sekundärstruktur bei Standardbedingungen bestimmt (Bindungs-/Faltungstemperatur: 21°C, lonenstärke des Bindungspuffers: [Na⁺] 100 mM / [Mg²⁺] 2 mM). Bindungspufferzusammensetzung: 100 mM NaCl, 20 mM Tris-HCl, pH 7.6, 2 mM MgCl₂, 5 mM KCl, 1 mM CaCl₂. Der Fachmann kann sehr leicht eine große Anzahl Varianten der konkret mit Sequenzen beschriebenen erfindungsgemäßen Aptamere entwerfen, beispielsweise durch Substitution, Insertion oder Deletion einzelner Basen. Solche Varianten können per Computer, wie oben beschrieben, in großer Zahl und ohne experimentellen Aufwand auf ihre Sekundärstruktur analysiert werden. Wenn eine Übereinstimmung der Sekundärstruktur einer Variante mit der Sekundärstruktur eines konkret mit Sequenz beschriebenen Aptamers vorliegt, ist eine Funktionalität der Variante wahrscheinlich oder bisweilen sehr wahrscheinlich.

Varianten der Aptamere nach Anspruch 1a) werden nachfolgend erläutert.

Von der Erfindung umfasst sind Aptamere, deren Sequenz eine Identität von mindestens 98%, mit einer der Sequenzen der SEQ ID NO: 14, 20, 36, 30, 37, 33, 25, 22, 39, 27, 41, 43 und 52 aufweisen.

Unter dem Begriff "Identität" soll im Zusammenhang mit der vorliegenden Erfindung die Anzahl der übereinstimmenden Nukleotide (Identität), ausgedrückt in Prozent verstanden werden. Bevorzugt wird die Identität zwischen zwei betreffenden Nukleinsäuresequenzen mit Hilfe von Computerprogrammen ermittelt. Weisen Sequenzen, die miteinander verglichen werden, unterschiedliche Längen auf, ist die Identität so zu ermitteln, dass die Anzahl an Nukleotide, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW2 bestimmt. Auf die Definition von Identität in dem Programm ClustalW2 und die Methode zu ihrer Ermittlung, die öffentlich zugänglich sind, wird in dieser Erfindung ausdrücklich Bezug genommen.

ClustalW2 wird öffentlich zur Verfügung gestellt vom European Bioinformatics Institute (EBI) des European Molecular Biology Laboratory (EMBL) und kann im Internet unter http://www.ebi.ac.uk/Tools/msa/clustalw2/ abgerufen werden. Wenn das ClustalW2 Computerprogramm benutzt wird, um die Identität zwischen z.B. der Nukleotidsequenz der im Rahmen der vorliegenden Erfindung beschriebenen Nukleinsäuremoleküle und der Nukleotidsequenz von anderen Nukleinsäuremolekülen zu bestimmen, werden folgende Parameter eingestellt: DNA Weight Matrix: IUB; GAP OPEN: 10; GAP EXTENSION: 0,20; GAP DISTANCES: 5; NO END GAPS: no; ITERATION: none; NUMITER: 1; CLUSTERING: NJ.

Gegenstand der Erfindung ist auch ein Aptamer, das von einem Aptamer mit einer der Sequenzen der SEQ ID NO: 14, 20, 36, 30, 37, 33, 25, 22, 39, 27, 41, 43 und 52 dadurch abgeleitet ist, dass bei einer dieser Sequenzen bis zu 2 Nukleotide substituiert, entfernt (deletiert), oder innerhalb der Sequenz eingefügt (insertiert) sind, wobei ein solches Aptamer an eine oder mehrere Verbindungen aus der Gruppe der Fluorchinolone bindet. Derart veränderte Aptamere weisen vorzugsweise eine Identität von mindestens 98% mit einer der Sequenzen der SEQ ID NO: 14, 20, 36, 30, 37, 33, 25, 22, 39, 27, 41, 43 und 52 auf. Der Begriff Identität wurde zuvor definiert. Eine Sequenz, bei der ein oder mehr Nukleotide substituiert sind, bezeichnet man auch als Substitutionsmutante, eine Sequenz, bei der ein oder mehr Nukleotide deletiert sind, als Deletionsmutante und eine Sequenz, bei der ein oder mehr Nukleotide insertiert sind, als Insertionsmutante.

Bei einer Anfügung sind am 5' und/oder am 3' Ende des Aptamers bis zu 20 Nukleotide angefügt, insbesondere bevorzugt bis zu 10 Nukleotide oder bis zu 8 Nukleotide, am meisten bevorzugt bis zu 5 Nukleotide.

In einer speziellen Ausführungsform, die mit den vorigen kombiniert werden kann, weisen die in den Punkten b) bis f) beschriebenen Aptamer-Abwandlungen eins oder mehrere der Motive ATACCAGCTTATTCAATT (SEQ ID NO: 83), ACAATCGTAATCAGTTAG (SEQ ID NO: 84) und TGAGGCTCGATC (SEQ ID NO: 85) auf. Vorliegen können somit die oben erwähnten Motive einzeln, oder das Motiv mit der SEQ ID NO: 83 und das Motiv mit der SEQ ID NO: 84, das Motiv mit der SEQ ID NO: 83 und das Motiv mit der SEQ ID NO: 85, das Motiv mit der SEQ ID NO: 84 und das Motiv mit der SEQ ID NO: 85, oder die Motive mit der SEQ ID NO: 83, SEQ ID NO: 84 und SEQ ID NO: 85.

Bei einem Aptamer, bei dem gegenüber einem Aptamer, welches eine Nukleinsäuresequenz umfasst, die ausgewählt ist aus einer der SEQ ID NO: 14, 20, 36, 30, 37, 33, 25, 22, 39, 27, 41, 43 und 52, ein oder mehrere Nukleotide angefügt sind, können Anfügungen am 5'-Ende des Aptamers und/oder am 3'-Ende des Aptamers erfolgen. Bei Anfügungen kann es sich beispielsweise um Oligonukleotide handeln, die als Spacer zwischen der Aptamersequenz und einer Markierung dienen oder um ein Oligonukleotid das eine Sequenz aufweist, die komplementär zu einem gelabelten Oligonukleotid ist, wie beschrieben in WO2005113817. Verschiedene weitere anfügbare Sequenzen, welche die Bindungseigenschaften des Aptamers an sein Target nicht beeinträchtigen, sind dem Fachmann auf dem Gebiet der SELEX Verfahren bekannt, beispielsweise aus Conrad et al., "In Vitro Selection of Nucleic Acid Aptamers That Bind Proteins, "Methods in Enzymology, 267: 336-83 (1996) ; Ciesiolka et al., "Affinity Selection-Amplification from Randomized Ribooligonucleotide Pools, "Methods in Enzymology, 267: 315-35 (1996); and Fitzwater et al., "A SELEX Primer, "Methods in Enzymology, 267: 275-301 (1996).

Fragmente, auch bezeichnet als Teile oder Teilsequenzen, weisen die zuvor beschriebene Funktionalität der erfindungsgemäßen Aptamere auf. Fragmente werden erhalten, indem man am 5'-Ende und/oder am 3'-Ende eines erfindungsgemäßen Aptamers eine oder mehrere Nukleinsäuren entfernt. Fragmente haben eine Länge von mindestens 30 Nukleotiden, noch mehr bevorzugt mindestens 40 Nukleotiden, weiterhin bevorzugt mindestens 50 Nukleotiden, und am meisten bevorzugt mindestens 60 Nukleotiden. Fragmente sind beispielsweise solche Aptamere, bei welchen die Motive ATACCAGCTTATTCAATT (SEQ ID NO: 83) und ACAATCGTAATCAGTTAG (SEQ ID NO: 84) ganz oder teilweise entfernt sind.

Der Begriff "Derivat" bezeichnet im Zusammenhang mit der vorliegenden Erfindung ein Aptamer, das an der Nukleobase, an der Pentose oder am Phosphat-Rückgrat chemisch modifiziert ist und das eine chemische Struktur aufweist, die in natürlicher DNA oder RNA nicht vorkommt.

Insbesondere bezeichnet der Begriff Derivat ein Aptamer, das eine chemische Struktur enthält, die von Desoxyribose, Ribose, Phosphat, Adenin (A), Guanin (G), Cytosin (C), Thymin (T), oder Uracil (U) abweichend ist. Ein Aptamer-Derivat kann an der Nukleobase, an der Pentose oder am Phosphat-Rückgrat modifiziert sein.

Insbesondere bezeichnet der Begriff "Derivat" ein Aptamer,
- bei dem natürlich in DNA und RNA vorkommende Nukleotide teilweise oder vollständig durch chemisch davon abweichende Nukleotide, sogenannte modifizierte Nukleotide, ersetzt sind und/oder
- dessen molekulare Struktur anderweitig modifiziert ist, insbesondere durch Anbindung nicht natürlich in RNA oder DNA vorkommender Strukturen, und/oder
- die ein modifiziertes Rückgrat aufweisen

Spezielle Beispiele für Derivate sind, ohne darauf beschränkt zu sein,
- Aptamere, die an mindestens einem Nukleotid eine Alkylierung, Arylierung oder Acetylierung, Alkoxylierung, Halogenierung, eine Aminogruppe oder eine andere funktionelle Gruppe aufweisen. Beispiele für modifizierte Nukleotide sind 2'-Fluor-Ribonucleotide, 2'-NH₂-, 2'-OCH₃- und 2'-O-methoxyethyl-Ribonukleotide, die für RNA-Aptamere genutzt werden.
- Aptamere, die eine Basenmodifikation aufweisen, wie Bromuridin,
- Markierte Aptamere, auch bezeichnet als gelabelte Aptamere. Bevorzugte Markierungen (Label) sind visuell, optisch, photonisch, elektronisch, akustisch, opto-akustisch, nach Masse, elektrochemisch, elektrooptisch, spektrometrisch, enzymatisch, oder anderweitig chemisch, biochemisch oder physikalisch detektierbar. Label können beispielsweise angebundene Reporter-, Marker- oder Adaptermoleküle sein. Beispiele hierfür sind markierte Aptamere, deren Markierung durch Lumineszenz, UV/VIS-Farbgebung, enzymatisch, elektrochemisch, immunologisch oder radioaktiv nachgewiesen werden kann. Beispiele für Markierungssubstanzen sind weiter unten bei der Erläuterung von erfindungsgemäßen Verfahren angegegeben, bei denen markierte Aptamere eingesetzt werden können.
- Aptamere, die enantiomere Nukleotide aufweisen.
- Aptamere, die ganz oder teilweise als Phosphorthioat-RNA oder -DNA, Phosphordithioat-RNA oder -DNA, Phosphorselenoat-RNA oder -DNA, Phosphordiselenoat-RNA oder -DNA, Phosphoroamidat-RNA oder -DNA, locked nucleic acid (LNA), peptide nucleic acid (PNA), N3'-P5' Phosphoramidat RNA/DNA, Cyclohexennukleinsäure (CeNA), Tricyclo-DNA (tcDNA) oder Spiegelmer vorliegen, oder die Phosphoramidatmorpholin (PMO) Komponenten aufweisen (siehe auch Chan et al., Clinical and Experimental Pharmacology and Physiology (2006) 33, 533-540).

Durch manche der Modifikationen können Aptamere gegen Nukleinsäure-spaltende Enzyme stabilisiert werden. Bei der Stabilisierung der Aptamere kann grundsätzlich zwischen der nachträglichen Modifikation der Aptamere und der Selektion mit bereits modifizierter DNA unterschieden werden. Die Stabilisierung berührt die Affinität der modifizierten DNA-Aptamere nicht, verhindert jedoch die schnelle Zersetzung der Aptamere in einem Organismus oder biologischen Lösungen durch RNasen/DNasen. Ein Aptamer wird im Rahmen der Erfindung als stabilisiert bezeichnet, wenn die Halbwertszeit in biologischen Seren grösser als eine Minute, vorzugsweise grösser als eine Stunde, besonders bevorzugt grösser als ein Tag ist. Die Aptamere können auch mit Reportermolekülen modifiziert sein, die neben der Detektion der markierten Aptamere auch zur Erhöhung der Stabilität beitragen können.

Die Bindungsfähigkeit der erfindungsgemäßen Aptamere wird durch die Affinität (Sensitivität) der Bindung (ausgedrückt durch die Dissoziationskonstante) beschrieben.

In einem weiteren Aspekt betrifft die Erfindung ein Verfahren zum Nachweis einer oder mehrerer Verbindungen aus der Gruppe der Fluorchinolone bei dem
a) ein oder mehrere verschiedene Aptamere wie zuvor beschrieben mit einer Probe, die eine oder mehrere Verbindungen aus der Gruppe der Fluorchinolone enthält, in Kontakt gebracht wird/werden und
b) eine Bindung zwischen dem/den Aptamer(en) und der/den Verbindung(en) aus der Gruppe der Fluorchinolone nachgewiesen wird.

Das Verfahren kann sowohl ein qualitatives als auch ein quantitatives Nachweisverfahren sein. Die Verfahrenschritte a) und b) können je nach konkreter Verfahrensgestaltung gleichzeitig oder im Wesentlichen gleichzeitig erfolgen.

Das Verfahren ist insbesondere zum Einsatz in der Lebensmittel-, Wasser- und Umweltanalytik, der Wasserbehandlung und der Wasseraufbereitung, insbesondere von Trinkwasser und Abwasser, der Diagnostik sowie zur Anwendung in Krankenhäusern und Pflegeeinrichtungen geeignet.

Das Verfahren eignet sich insbesondere zum Nachweis von einer Verbindung aus der Gruppe der Fluorchinolone in beliebigen Medien und Umgebungen, insbesondere in Wasser und anderen Flüssigkeiten, wie beispielsweise in Trink- und Abwasserproben.

Eine Probe im Sinne des obigen Nachweisverfahrens ist ein bereitgestelltes oder durch Probenentnahme erhaltenes Material, von dem angenommen wird, dass es eine oder mehrere Verbindungen aus der Gruppe der Fluorchinolone (zusammenfassend bezeichnet als "Target" oder im Rahmen dieses Nachweisverfahrens auch als "Analyt") umfasst, und das auf das Vorhandensein von Target geprüft werden soll.

Eine Probe kann eine Wasserprobe, insbesondere eine Trinkwasser-, Grundwasser-, Oberflächenwasser- oder Abwasserprobe, eine Probe von anderweitigem, aus der Umwelt entnommenem Material, eine klinische Probe oder eine Lebensmittelprobe sein.

Eine Probe können weiterhin alle biologischen Materialien sein, die von Individuen isoliert worden sind, beispielsweise biologische Gewebe und Flüssigkeiten, zu denen u.a. Blut, Haut, Plasma, Serum, Lymphe, Urin, Gehirnflüssigkeit, Tränen, Abstriche, Gewebeproben, Organe und Tumore zählen. Eine beispielhafte und bevorzugte Lebensmittelprobe ist Milch. Vorliegend sind in Proben auch Bestandteile von Zellkulturen eingeschlossen. Die Probenentnahme erfolgt insbesondere so, dass die entnommene Teilmenge einem Durchschnitt der gesamten Menge entspricht. Die durch Untersuchung der Probe ermittelten Merkmale dienen der Beurteilung der durch die Probe erfassten Menge, die wiederum Rückschlüsse auf die Gesamtmenge, beispielsweise das Blut bzw. die Lymphe in einem Organismus, zulässt. Für die Untersuchung können die Proben vorbehandelt, wie z.B. durch Mischen, Zugabe von Enzymen oder Markern, oder aufgereinigt werden.

Wird das Aptamer mit dem Target in Kontakt gebracht, so bildet sich ein Aptamer-Target-Komplex durch Bindung des Aptamers an das Target. Die Bindung bzw. das Bindungsereignis kann beispielsweise visuell, optisch, photonisch, elektronisch, akustisch, opto-akustisch, nach Masse, elektrochemisch, elektrooptisch, spektrometrisch, enzymatisch, oder anderweitig chemisch, biochemisch oder physikalisch nachgewiesen werden.

Bei einem markierungsfreien Nachweis wird das Aptamer beispielsweise auf einer Oberfläche fixiert und die Änderung der Schichtdicke nach Anlagerung des Targets mit einer der genannten Methoden bestimmt, wie z.B. über eine Änderung der optischen Eigenschaften der Sensorschicht (z.B. Brechungsindex). Ein weiteres Verfahren des markierungsfreien Nachweises ist die Messung des Masseänderung nach Bindung des Aptamers an das Target mit einer Mikrowaage, oder die Messung einer Frequenzänderung eines Schwingquarzes nach Bindung des Aptamers an das Target, welches auf der Oberfläche des Schwingquarzes bereitgestellt wird.

In Fällen, in denen die Komplexierung nicht direkt detektierbar ist, kann der Komplex durch eine Markierung sichtbar gemacht werden, beispielsweise in einer Indikatorreaktion nach einer direkten oder indirekten Kopplung eines Komplexpartners mit einer Markierungssubstanz. Entweder kann das verwendete Aptamer oder das Target mit einer Markierung (Label) versehen sein. Bevorzugt ist das Aptamer markiert.

Bevorzugte Markierungen (Label) sind visuell, optisch, photonisch, elektronisch, akustisch, opto-akustisch, nach Masse, elektrochemisch, elektrooptisch, spektrometrisch, enzymatisch, oder anderweitig physikalisch, chemisch oder biochemisch detektierbar. In einer Ausführungsform des Verfahrens wird die Markierung durch Lumineszenz, UV/VIS-Spektroskopie, enzymatisch, elektrochemisch oder radioaktiv nachgewiesen.

Lumineszenz betrifft die Emission von Licht. Im erfindungsgemäßen Verfahren finden beispielsweise die Photolumineszenz, die Chemilumineszenz und die Biolumineszenz zum Nachweis der Markierung Anwendung. Bei der Photolumineszenz oder Fluoreszenz erfolgt die Anregung durch Absorption von Photonen. Beispielhafte Fluorophore sind, ohne Beschränkung, Bisbenzimidazol, Fluoreszein, Acridinorange, Cy5, Cy3 oder Propidiumiodid, die kovalent an Aptamere gekoppelt werden können, Tetramethyl-6-Carboxyrhodamin (TAMRA), Texas Red (TR), Rhodamin, Alexa Fluor Farbstoffe (u.a. Fluoreszenzfarbstoffe verschiedener Wellenlängen von verschiedenen Firmen). Die Auswertung geschieht visuell oder mit entsprechenden Messgeräten, z.B. im Multilabel Counter, im Fluoreszenzmikroskop, oder durch Durchflusszytometrie, z.B. im Cytofluorimeter. Chemilumineszenz beschreibt die Emission sichtbaren Lichts als Folge einer chemischen Reaktion. Biolumineszenz bezeichnet die Emission sichtbaren Lichts als Folge einer Enzymreaktion, beispielsweise einer durch das Enzym Luciferase katalysierten Redoxreaktion.

Andere Markierungsstoffe sind Katalysatoren, kolloidale metallische Teilchen, z.B. Gold-Nanopartikel, kolloidale nicht metallische Teilchen, Quantum Dots, organische Polymere, Latexteilchen, Nanofasern, insbesondere aus Kohlenstoff, Nanoröhren ("nanotubes"), insbesondere aus Kohlenstoff ("carbon nanotubes"), Dendrimere, Proteine oder Liposome mit signalerzeugenden Stoffen. Kolloidale Teilchen können kolorimetrisch nachgewiesen werden.

Auch einsetzbar sind visuell detektierbare Farbstoffe, wie beispielsweise interkalierende Farbstoffe.

Einsetzbar als Marker sind auch Enzyme, deren enzymatische Reaktion durch den Verbrauch oder die Entstehung detektierbarer Substrate oder Produkte gekennzeichnet ist, wobei ohne Beschränkung eine optische oder elektrochemische Detektion angewandt werden kann. Ein Nachweis kann z.B. mit Enzymen als Markierungssubstanzen geführt werden, die Substrate zu farbigen Produkten umsetzen, vorzugsweise Peroxidase, Green Fluorescent Protein (GFP), Luciferase, [beta]-Galactosidase oder Alkalische Phosphatase. Beispielsweise wird das farblose Substrat X-Gal durch die Aktivität der [beta]-Galactosidase zu einem blauen Produkt umgesetzt, dessen Farbgebung visuell erfasst wird.

Ein oben genanntes enzymatisches Marker- und Nachweissystem verwendet Alkalische Phosphatase. Mit Alkalischer Phosphatase (APP) sind verschiedene Nachweise möglich, wie nachfolgend beispielhaft dargestellt:
- elektrochemischer Nachweis: Substrat Phenylphosphat, enzymatische Reaktion von APP bildet Phenol, wird an Phenolsensor (z.B. mit immobilisierter Tyrosinase) elektrochemisch detektiert
- Messung der Farbreaktion: Substrat p-Nitrophenylphosphat, enzymatische Reaktion von APP bildet p-Nitrophenol, ist gelb gefärbt
- Fluoreszenz-Nachweis: Substrat 4-Methylumbelliferonylphosphat: enzymatische Reaktion von APP bildet Methylumbelliferonyl-Rest, der nach Anregung Fluoreszenz freisetzt
- für Chemilumineszenz-Nachweis: Substrat AMPPD (3-(2'-Spiroadamantan)-4-Methoxy-4-(3"-Phosphoryloxy)phenyl-1,2-Dioxetan): enzymatische Reaktion von APP bildet AMP-D und setzt hv (Chemilumineszenz) frei

Ferner setzt APP 5-Brom-4-Chlor-3-Indolylphosphat (BCIP oder X-Phosphat) und Nitroblau-Tetrazoliumsalz (NBT) farbig um. Die Farbstoffe fallen dabei in unmittelbarer Nähe der AP-Moleküle aus und färben die Umgebung der gebundenen Verbindungen dunkelviolett an.

Die Peroxidase katalysiert z.B. die Oxidation von ABTS (2,2'-Azino-bis-[3-ethylbenzthiazolin-6-sulfonsäure]) in Gegenwart von H₂O₂. Aufgrund der Enzymstabilität und einer Vielzahl an möglichen Substraten ist die Meerettich-Peroxidase bevorzugt. Weitere Enymmarker, die die Erzeugung detektierbarer Produkte katalysieren sind Chloramphenicolacetyltransferase (CAT) und Glutathion-S-Transferase (GST).

Der Nachweis kann auch mittels radioaktiver Isotope erfolgen, mit denen das Aptamer markiert ist, vorzugsweise 3H, 14C, 32P, 33P, 35S oder 125I, besonders bevorzugt 32P, 33P oder 125I. Bei der Szintillationszählung wird die vom radioaktiv-markierten Aptamer-Target-Komplex abgegebene radioaktive Strahlung indirekt gemessen. Eine Szintillatorsubstanz wird durch die radioaktive Strahlung angeregt. Beim Übergang in den Grundzustand wird die Anregungsenergie wieder als Lichtblitze freigesetzt, welche durch einen Photoelektronenvervielfacher (Photomultiplier) verstärkt und gezählt werden.

Die Aptamere können auch mit Digoxigenin oder Biotin markiert sein, die beispielsweise durch Antikörper oder Streptavidin gebunden werden, die wiederum eine Markierung tragen können, wie z.B. ein Enzymkonjugat. Die vorherige kovalente Verknüpfung (Konjugation) eines Antikörpers mit einem Enzym kann auf verschiedene bekannte Arten erfolgen. Der Nachweis der Antikörper-Bindung kann auch radioaktiv in einem RIA (radioactive immunoassay) mit radioaktiven Isotopen, vorzugsweise mit 125I, oder durch Fluoreszenz in einem FIA (Fluoroimmunoassay) mit Fluorophoren, vorzugsweise mit Fluorescein oder FITC, erfolgen.

Die genannten Methoden schließen vorzugsweise Waschschritte ein, um ungebundene und/oder unspezifisch gebundene Aptamere und/oder Nachweisreagenzien abzutrennen. Die Durchführung sämtlicher Nachweisverfahren ist dem Fachmann bekannt. Der direkte Nachweis der Markierung ist im vorliegenden Verfahren der Erfindung bevorzugt, insbesondere der direkte Nachweis durch Fluoreszenz.

In einer weiteren Ausgestaltung des Verfahrens wird der Nachweis in-situ durchgeführt. Diese Umsetzung erfordert einen geeigneten Inkubationsraum, auf dem der Nachweis einfach durchgeführt und sichtbar gemacht werden kann. Hierzu wird vorteilhaft ein fester Träger verwendet, der es ermöglicht, Probe, Aptamere und ggf. Nachweisreagenzien der Komplexe zu fixieren. Die Aptamere können sowohl vor oder nach der Probe auf den festen Träger aufgebracht werden. Methoden der Immobilisierung vorgenannter Aptamere sind dem Fachmann bekannt. Beispiele sind weiter unten angegeben. Für den Nachweis kommen alle Nachweise in Betracht, die zuvor bereits genannt sind, wie markierungsfreie Nachweise und Nachweise mit Verwendung einer Markierung. Bei einem Farbnachweis können die Aptamere so markiert sein, so dass direkt im Anschluss an die Inkubation ein Ablesen und Auswerten realisiert werden kann.

Weitere Nachweismethoden eines Aptamer-Target-Komplexes, sind beschrieben in Stoltenburg, R.; Nikolaus, N.; Strehlitz, B. (2012) Capture-SELEX - selection of DNA aptamers for aminoglycoside antibiotics, Journal of Analytical Methods in Chemistry, Volume 2012 (2012), Article ID 415697, worauf ausdrücklich verwiesen wird. Es sind in der Publikation sowohl direkte als auch indirekte Methoden zur Detektion eines Aptamer-Target-Komplexes beschrieben. In der Publikation erläuterte Methoden sind beispielsweise die sogenannte Förster-Resonanz-Energie-Transfer (FRET)-Methode und die "FP"-Methode. Der Förster-Resonanz-Energie-Transfer (FRET) ist ein physikalischer Prozess, bei dem Energie eines angeregten Donormoleküles strahlungsfrei auf ein Akzeptormolekül im Abstand von etwa 1,5 bis 10 nm übertragen werden kann. Die Moleküle sind dazu so ausgewählt, dass das Emissionsspektrum des Donormoleküls mit dem Anregungsspektrum des Akzeptormoleküls überlappt. Sind sowohl Donor als auch Akzeptor Fluoreszenzfarbstoffe, spricht man von einem Fluorescence resonance energy transfer. Ist der Donor oder Akzeptor hingegen eine Chemilumineszenz- oder eine Biolumineszenzquelle wird der Begriff Chemiluminescence resonance energy transfer (CRET) bzw. Bioluminescence resonance energy transfer (BRET) verwendet. Vorteilhaft kann durch die Bindung an ein erfindungsgemäßes Aptamer ein nachzuweisendes Molekül aus der Gruppe der Fluorochinolone in die Nähe des Donor/Akzeptorpaares gebracht werden, wodurch das FRET-Signal modifiziert wird und die Änderung messtechnisch erfasst werden kann.

In den zuvor erläuterten Verfahren und allen anderen von dieser Erfindung offenbarten Verfahren kann das erfindungsgemäße Aptamer allein, oder eine Mischung verschiedener erfindungsgemäßer Aptamere eingesetzt werden. Erfindungsgemäße Aptamere können auch in Kombination mit anderen Aptameren angewendet werden. Eine Kombination mit anderen Aptameren für andere Targets (d.h. andere Targets als eine Verbindung aus der Gruppe der Fluorchinolone) bietet den Vorteil, dass ein entsprechendes Verfahren gleichzeitig auch zum Nachweis, Anreicherung, Abtrennung und/oder Isolierung anderer Targets anwendbar ist.

Wie bereits erwähnt, können die Probe oder das Aptamer je nach Verfahrensgestaltung an eine feste Phase immobilisiert sein. In dem Verfahren können beispielsweise (Micro)-Arrays des erfindungsgemäßen Aptamers, oder Mikrotiterplatten-Testsysteme eingesetzt werden.

Aptamere binden an das Target ähnlich stark wie Antikörper an ihr Target (Antigen). Daher ist das oben beschriebene Nachweisverfahren analog durchführbar wie bekannte Immunoassays, wobei im Vergleich zu einem bekannten Immunoassay einer oder mehrere Antikörper durch ein erfindungsgemäßes Aptamer ersetzt sind.

Beispielsweise ist es möglich, das Nachweisverfahrens in Form eines kompetitiven Assays oder eines Sandwich-Assays durchzuführen.

Bei einer Ausführungsform eines kompetitiven Assays wird ein für das Target spezifisches erfindungsgemäßes Aptamer an eine feste Phase gebunden. Anschließend erfolgt die Zugabe der Probenlösung, die das zu messende Target enthält und gleichzeitig mit einer bekannten Konzentration an markiertem Target versetzt ist. Sowohl das in der Probenlösung in unbekannter Konzentration vorliegende unmarkierte Target als auch das markierte Target konkurrieren um die Bindung an das gebundene Aptamer. Je höher die Konzentration des Targets in der Probe ist, desto weniger markiertes Target wird demzufolge an das Aptamer gebunden. Über die Erkennung und gegebenenfalls eine quantitative Bestimmung der Markierung sind der Nachweis des Targets in der Probe und die Messung seiner Konzentration möglich.

In einer anderen Ausführungsform eines kompetetiven Assays wird ebenfalls zunächst ein für das Target spezifisches erfindungsgemäßes Aptamer an eine feste Phase gebunden. Daran wird markiertes Target gebunden. Die Messung der Markierung ergibt das Ausgangssignal. Die Zugabe von Probe mit unmarkiertem Target verdrängt gebundenes markiertes Target, und das abnehmende Messignal ist der Probenkonzentration proportional.

Bei einem klassischen immunologischen Sandwich-Assay werden mindestens zwei Antikörper eingesetzt. Zunächst wird einer der beiden Antikörper an eine feste Phase immobilisiert. Dieser wird als Primärantikörper oder auch als Fänger bezeichnet. Nach der Zugabe der Probe bindet das darin enthaltene Antigen an den Primärantikörper. Anschließend wird die Probenlösung entfernt und der als Sekundärantikörper oder Detektor bezeichnete zweite Antikörper in gelöster Form auf die feste Phase gegeben. Der Detektor bindet nun ebenfalls an das durch den Primärantikörper gebundene Antigen. Für den Nachweis und die Quantifizierung ist der Sekundärantikörper entweder selbst markiert, oder er wird über ein markiertes Reagens nachgewiesen. In der vorliegenden Erfindung wird dieses Verfahren dahin gehend abgewandelt, dass entweder der Primärantikörper oder der Sekundärantikörper, oder beide, durch ein erfindungsgemäßes Aptamer ersetzt werden und dass das Antigen ein Target für das Aptamer, also eine Verbindung aus der Gruppe der Fluorchinolone ist. Der Assay kann mit allen bekannten festen Phasen durchgeführt werden, beispielsweise Mikrotiterplatten, Streifen, Membranen, Küvetten etc.

Die Erfindung stellt somit in einer speziellen Ausführungsform ein Verfahren zum Nachweis von Verbindungen aus der Gruppe der Fluorchinolone zur Verfügung, bei dem
a) ein erster Antikörper oder ein erfindungsgemäßes Aptamer, der/das für eine Verbindung aus der Gruppe der Fluorchinolone spezifisch ist, an eine feste Phase immobilisiert wird,
b) die feste Phase mit daran immobilisiertem Antikörper oder Aptamer mit einer Probe, die eine Verbindung aus der Gruppe der Fluorchinolone enthält, inkubiert wird, und
c) die feste Phase mit einem zweiten Antikörper oder einem erfindungsgemäßen Aptamer, der/das für eine Verbindung aus der Gruppe der Fluorchinolone spezifisch ist, inkubiert wird, und
d) eine Bindung zwischen dem zweiten Antikörper oder dem Aptamer und der Verbindung aus der Gruppe der Fluorchinolone nachgewiesen wird, unter der Bedingung, dass mindestens in einem der Schritte a) oder c) ein erfindungsgemäßes Aptamer statt eines Antikörpers eingesetzt wird.

Die Immobilisierung in Schritt a) kann mit allen üblichen Techniken erfolgen, die auch aus immunologischen Assays bekannt sind. Der Nachweis in Schritt d) kann im Falle eines Antikörpers durch die bekannten Methoden erfolgen, die aus immunologischen direkten und indirekten Sandwich-Assays bekannt sind. Entweder kann der Antikörper selbst markiert sein, beispielsweise durch Anbindung eines Enzyms, das eine Farbreaktion zum Nachweis katalysieren kann, oder es kann ein dritter Antikörper zugegeben werden, der seinerseits an den zweiten Antikörper bindet und eine Markierung aufweist. Sofern in Schritt c) ein Aptamer eingesetzt wird, ist dieses vorzugsweise ein markiertes Aptamer, das mit geeigneten Nachweisreaktionen detektiert werden kann, wie zuvor bereits beschrieben. Zwischen den genannten Schritten a) -d) werden vorzugsweise Waschschritte durchgeführt, z.B. mit üblichen und bekannten Waschpuffern. Der Begriff Immobilierung bedeutet bei diesem Verfahren, dass der in Schritt a) immobilisierte Antikörper oder das Aptamer nicht durch einen Waschschritt von der festen Phase entfernt wird.

In einem weiteren Aspekt betrifft die Erfindung ein Verfahren zur Anreicherung, Abtrennung und/oder Isolierung einer oder mehrerer Verbindungen aus der Gruppe der Fluorchinolone bei dem
a) ein oder mehrere verschiedene Aptamere wie zuvor beschrieben mit einer Probe, die eine oder mehrere Verbindungen aus der Gruppe der Fluorchinolone enthält, in Kontakt gebracht wird/werden, wobei ein Komplex oder Komplexe aus dem/den Aptamer(en) und der/den Verbindung(en) aus der Gruppe der Fluorchinolone gebildet wird,
b) der/die Komplex(e) und die übrige Probe voneinander getrennt werden, und
c) optional die Verbindung(en) aus der Gruppe der Fluorchinolone aus dem Komplex isoliert wird/werden.

Eine "Probe" im Sinne dieses Verfahrens ist ebenso definiert wie eine Probe im Sinne des zuvor beschriebenen Nachweisverfahrens. Eine Probe ist insbesondere eine Wasserprobe, wie zum Beispiel eine Trinkwasser-, Grundwasser-, Oberflächenwasser- oder Abwasserprobe, eine Probe von anderweitigem aus der Umwelt entnommenem Material, oder eine Lebensmittelprobe.

Auch dieses Verfahren kommt insbesondere zum Einsatz in der Lebensmittel-, Wasser- und Umweltanalytik, der Wasserbehandlung und der Wasseraufbereitung, insbesondere von Trinkwasser und Abwasser, der Diagnostik sowie zur Anwendung in Krankenhäusern und Pflegeeinrichtungen.

Eine "Abtrennung" von einer oder mehrerer Verbindungen aus der Gruppe der Fluorchinolone (zusammengefasst bezeichnet als "Target") aus einer Probe kann, im Rahmen der analytischen Nachweisgrenze, vollständig sein oder nicht vollständig sein. Eine vollständige Abtrennung wird hierin auch als "Entfernung" bezeichnet.

Eine "Anreicherung" wird in Schritt b) erzielt, wenn der gebildete Aptamer-Target-Komplex und die übrige Probe voneinander getrennt werden. Es werden zwei Fraktionen erhalten, wobei in der Fraktion, die den Komplex enthält, der Komplex angereichert ist. Der Begriff "Anreicherung" kann sich auf den Komplex beziehen, der aus Aptamer/en und einer oder mehrerer Verbindungen aus der Gruppe der Fluorchinolone gebildet ist, oder auf die Verbindung/en aus der Gruppe der Fluorchinolone selbst, d.h. das Target ohne Aptamer. Zur Anreicherung des Targets kann der Verfahrensschritt c) durchgeführt werden. Eine Anreicherung bedeutet mit anderen Worten eine Erhöhung der Konzentration des Aptamer-Target-Komplexes oder des Targets selbst.

Der Begriff "Isolierung" kann sich auf den Komplex beziehen, der aus Aptamer/en und einer oder mehrerer Verbindungen aus der Gruppe der Fluorchinolone gebildet ist, oder auf die Verbindung/en aus der Gruppe der Fluorchinolone selbst. Daher ist der letzte Verfahrensschritt optional.

Die oben beschriebene Abtrennung, Anreicherung, und Isolierung können mit dem Verfahren gleichzeitig verwirklicht werden. Vorzugsweise wird das Verfahren aber zu einem dieser Zwecke durchgeführt.

Der Begriff "Komplex" bezeichnet die Struktur, die bei der Bindung des Aptamers an sein Target gebildet wird. Somit bezeichnet der Begriff "Komplex" eine Aptamer-Target-Struktur, bei der das Aptamer an das Target gebunden ist. Wie einleitend erläutert, erfolgt die Aptamer-Target-Bindung vorwiegend, aber nicht unbedingt ausschließlich, über die Strukturkompatibilität, so genannte "Stacking interactions" bei aromatischen Ringstrukturen (Stapelkräfte durch Elektronenwechselwirkung mit Nachbarbasen), elektrostatische Wechselwirkungen (z.B. Van der Waals-, Ionen-, Dipolkräfte) und Wasserstoffbrückenbindungen.

Die genannten Verfahrensschritte a), b) und c) können je nach Verfahrensgestaltung gleichzeitig erfolgen, und insbesondere können die Verfahrensschritte a) und b) gleichzeitig erfolgen.

Bei der Kontaktierung von Target und Aptamer kann die Bildung des Aptamer-Target-Komplexes durch Temperierung auf die optimale Aptamer-Target-Bindungstemperatur, bevorzugt 20 - 25°C, und/oder Rühren der Probe befördert werden. Nach einer Inkubationszeit werden die Aptamer-Target-Komplexe, von der restlichen Probenlösung abgetrennt. Geeignete Methoden sind dem Fachmann bekannt, wie z.B. Dialyse, Ultrafiltration, Gelfiltration, Chromatographie, magnetische Trennung oder Waschschritte, sofern der Komplex immobilisiert vorliegt.

Durch Veränderungen der für die Aptamerbindung erforderlichen definierten Bedingungen wird im optionalen Schritt c) der Aptamer-Target-Komplex getrennt. Hierzu sind physikochemische Einwirkungen, wie z.B. eine Variation von Salzkonzentration oder pH oder eine hitzebedingte Denaturierung, denkbar. Abschließend ist eine weitere Reinigung des Targets möglich, wobei das Aptamer zuvor abgebaut werden kann, z.B. durch saure (thermische) Hydrolyse oder DNasen. Sofern das Aptamer immobilisiert ist, kann das Target eluiert werden, wie z.B. von einer Säule, die anschließend regeneriert und wiederverwendet wird.

Erfindungsgemäß können die Aptamere auch an einer festen Phase, genauer gesagt an der Oberfläche einer festen Phase, immobilisiert sein, vorzugsweise über ein Spacer-Molekül, das beispielsweise eine Linker-Nukleinsäure sein kann. Geeignete Methoden der Immobilisierung sind dem Fachmann bekannt, die sowohl mit einer kovalenten Kopplung als auch einer nicht-kovalenten Kopplung mittels geeigneter Affinitätspaare, wie z.B. Biotin/Streptavidin, einhergehen können. Die feste Phase können beispielsweise Platten, Streifen, Membranen, Filme, Gele, Perlen, Mikro- und Nanopartikel sein. Beispielhafte Trägermaterialien sind anorganische und organische Polymere, Keramiken, Gläser, Metalle, insbesondere Edelmetalle. Hierzu zählen Kunststoffe, beispielsweise auf Basis von Polystyrol. Des Weiteren sind Biopolymere, vorzugsweise Cellulose, Dextran, Agar, Agarose und Sephadex, die funktionalisiert sein können, insbesondere als Nitrocellulose oder Cyanbromid-Sephadex, als Polymere im erfindungsgemäßen Verfahren einsetzbar. Aptamere können an Magnetic Beads gebunden werden, deren Oberfläche z.B. mit Tosyl- oder Epoxy-Gruppen, mit Amino- oder Carboxyl-Gruppen oder mit Streptavidin funktionalisiert sind. Weiterhin ist eine Kopplung von Magnetic Beads an die Desoxyribose der Aptamere, wie z.B. über eine Hydrazon-Bindung, möglich. Die Beispiele für Polymere sind nicht limitierend und andere sind denkbar. Bevorzugte Kombinationen von geometrischer Form und Material sind Gele aus Biopolymeren, insbesondere Agarose, und Gelmatrices aus Dextran und Sephadex, die - beispielsweise in Säulen verpackt - Hohlräume definierter Porengröße ausbilden. In einer speziellen Ausgestaltung ist das soeben beschriebene Abtrennungs-, Anreicherungs-, und/oder Isolierungsverfahren ein Affinitätschromatographie-Verfahren, bei dem ein erfindungsgemäßes Aptamer an einer festen Phase immobilisiert ist, vorzugsweise an Kügelchen (Beads) oder einem anderweitigen chromatographischen Säulenmaterial, wobei alle geometrische Formen einer festen Phase verwendbar und alle oben genannten Substanzen beispielhaft einsetzbar sind.

Mit Aptamer-modifizierten Oberflächen können die entsprechenden Targets ausgehend von geringen Konzentrationen angereichert werden.

Die Erfindung betrifft auch die Verwendung des zuvor beschriebenen Aptamers zum Nachweis, Anreicherung, Abtrennung und/oder Isolierung einer oder mehrerer Verbindungen aus der Gruppe der Fluorchinolone. Insbesondere kann das Aptamer in den zuvor erläuterten Verfahren eingesetzt werden. Der Begriff Anreicherung schließt die Aufreinigung des Targets mit ein.

In einem weiteren Aspekt betrifft die Erfindung auch eine Aptamersonde zur Detektion einer oder mehrerer Verbindungen aus der Gruppe der Fluorchinolone, wobei die Sonde ein erfindungsgemäßes Aptamer und ein Markierungsmittel (Label) umfasst. Das Markierungsmittel kann auf verschiedene Art und Weise an das Aptamer gebunden sein, beispielsweise, und nicht beschränkend, durch kovalente Bindung, Komplexierung, DNA-Hybridisierung. Beispielsweise kann ein Markierungsmittel an ein erfindungsgemäßes Aptamer gebunden werden, wie in WO2005113817 beschreiben. WO2005113817 beschreibt ein Aptamer mit einem angefügten Oligonukleotidschwanz (oligonucleotide tail) und einem Linker-Oligonukleotid, der ein Label trägt, wobei die Sequenz des Linker-Oligonukleotid komplementär zu der Sequenz des Oligonukleotidschwanzes des Aptamers ist, sodass das Linker-Oligonukleotid an den Oligonukleotidschwanz des Aptamers hybridisiert und das Aptamer mit dem Label markiert wird. Eine weitere Möglichkeit ist die Bindung von Biotin an das Aptamer und die Bindung von Markierungsmittel an Steptavidin. Durch eine Biotin-Streptavidin Bindung wird das Markierungsmittel an das Aptamer gebunden.

Alle Markierungsmittel sind grundsätzlich einsetzbar, die sich an DNA binden lassen. Konkrete Beispiele für Markierungssubstanzen wurden weiter oben bei der Erläuterung von erfindungsgemäßen Verfahren angegeben, bei denen markierte Aptamere eingesetzt werden können. Beispielsweise ist die Markierung ein Farbstoff. In einer speziellen Variante ist das Markierungsmittel eine Substanz, die eine Bilderzeugung durch das markierte Aptamer in einem bildgebenden Verfahren ermöglicht. Das Bild kann durch Strahlung erzeugt sein, die ein für das menschliche Auge direkt sichtbares Bild erzeugt, oder durch Strahlung, die nicht direkt sichtbar ist, beispielsweise Röntgenstrahlung oder radioaktive Strahlung, die anderweitig sichtbar gemacht wird, beispielsweise auf einem Film. Markierungsmittel können beispielsweise Lumineszenz-, Phosphoreszenz, Fluoreszenz-, radioaktive, oder UV/VIS- Markierungssubstanzen sein. Die erfindungsgemäße Sonde ist besonders für die in dieser Beschreibung erläuterten Nachweis-, Anreicherungs-, Abtrennungs- und/oder Isolierungsverfahren einsetzbar, insbesondere in Assays zur Detektion von Verbindungen aus der Gruppe der Fluorchinolone. Assays können z.B. direkte und indirekte Sandwich-Assays sein.

In einem weiteren Aspekt betrifft die Erfindung einen Biosensor, der ein erfindungsgemäßes Aptamer aufweist.

Ein solcher Biosensor weist gemäß der vorliegenden Erfindung vorzugsweise die folgenden Elemente auf:
- einen Rezeptor, aufweisend oder bestehend aus einem erfindungsgemäßen Aptamer, und
- einen Transducer, der das Bindungsereignis zwischen Aptamer und Target in ein elektrisch quantifizierbares Signal wandelt.

Außerdem können weitere Elemente, wie beispielsweise eine Signalverarbeitungseinrichtung, eine Ausgabeelektronik, eine Anzeigevorrichtung, eine Datenverarbeitungseinrichtung, ein Datenspeicher sowie Schnittstellen zu anderen Geräten vorhanden sein.

Der biologische Rezeptor des Aptamer-Biosensors weist das erfindungsgemäße Aptamer vorzugsweise in immobilisierter Form auf oder besteht nur aus dem Aptamer in immobilisierter Form. Die Funktion des Rezeptors ist die auf einem biochemischen Mechanismus beruhende Erkennung des Analyten, hier die Bindung des erfindungsgemäßen Aptamers an sein Target. Aus einer Probe, die man mit dem Biosensor in Kontakt bringt, beispielsweise einem komplexen Gemisch, das Target enthält, kann das Target über die spezifische Anbindung des Aptamers identifiziert werden.

Die Spezifität des Biosensors wird durch den Rezeptor vorgegeben, während die Sensitivität des Sensors vorwiegend durch den verwendeten Transducer beeinflusst wird. Die am Rezeptor stattfindende Aptamer-Target Bindung wird durch den Transducer in ein elektronisch verwertbares Signal umgesetzt. Der Transducer wandelt das Signal aus der selektiven Erkennungsreaktion des Rezeptors (Bindung Target an Aptamer), das der Konzentration des Targets in der Probe proportional ist, in ein elektrisch quantifizierbares Messsignal um. Die Signalbildung erfolgt auf Grund der molekularen Interaktion zwischen dem Target und dem Aptamer.

Mit einem erfindungsgemäßen Biosensor können vorzugsweise qualitative, quantitative und/oder halb-quantitative analytische Informationen gewonnen werden. Er ist insbesondere zum Einsatz in der Lebensmittel-, Wasser- und Umweltanalytik, der Wasserbehandlung und der Wasseraufbereitung, insbesondere von Trinkwasser und Abwasser, in der Diagnostik sowie zur Verwendung in Krankenhäusern und Pflegeeinrichtungen geeignet.

Die Kopplung zwischen Rezeptor und Transducer erfolgt vorzugsweise durch die Immobilisierung des Aptamers auf der Transduceroberfläche, beispielsweise über eine Straptavidin-Biotin-Bindung, wobei vorzugsweise das Aptamer mit Biotin verbunden ist und die Oberfläche des Transducers immobilisiertes Streptavidin aufweist.

Die Bindung des Targets an das erfindungsgemäße Aptamer kann beispielsweise über optische, mikrogravimetrische, thermische oder akustische Transducer gemessen werden, vorzugsweise über optische oder mikrogravimetrische Transducer.

Die Messung in optischen Transducern kann auf Prinzipien der Photometrie beruhen, wobei beispielsweise Farb- oder Lumineszenz- Intensitätsänderungen erfasst werden. Zu den optischen Methoden zählen die Messung von Fluoreszenz, Phosphoreszenz, Biolumineszenz und Chemolumineszenz, Infrarotübergängen und die Lichtstreuung. Zu den optischen Methoden zählt auch die Messung von Schichtdickenänderungen wenn Target an Aptamer gebunden wird. Die Schichtdickenänderung kann z.B. mittels Oberflächenplasmonenresonanz (SPR) oder reflektometrischer Interferenzspektroskopie (RIfS) durchgeführt werden. Ferner kann die Interferenz an dünnen Schichten (reflektrometrische Interferenzspektroskopie) sowie die Änderung des evaneszenten Feldes gemessen werden.

Mikrogravimetrische Transducer sind zum Beispiel QCM-Sensoren (Quartz Crystal Microbalance), die eine Masseänderung detektieren, wenn Target an Aptamer gebunden wird.

Akustische Transducer nutzen die Frequenzänderungen eines piezoelektrischen Schwingquarzes, der Masseänderungen hochempfindlich nachweist, die auftreten wenn Target an Aptamer bindet. Der verwendete Quarzkristall wird in einem oszillierenden elektrischen Feld platziert und die Resonanzfrequenz des Kristalls gemessen. Eine Masseänderung auf der Oberfläche des Schwingquarzes, z.B. durch Reaktion des Analyten mit dem vorher auf der Kristalloberfläche immobilisierten Rezeptor, bewirkt eine Änderung der Resonanzfrequenz, welche quantifiziert werden kann.

Elektrochemische Transducer können z.B. die Änderung der Konzentration redoxaktiver Marker an der Elektrodenoberfläche messen, oder die Änderung der Konzentration redoxaktiver Substrate oder Produkte, die z.B. in der enzymatischen Reaktion eines Enzym-Markers verbraucht werden oder entstehen.

Thermische Transducer messen die Wärmetönung der Aptamer-Target-Bindungsreaktion.

Ein beispielhafter Biosensor ist im beigefügten Ausführungsbeispiel erläutert. Anleitung zur Konstruktion weiterer Biosensoren findet der Fachmann in Cho, E. J., Lee, J.-W., Ellington, A. D. (2009): Applications of Aptamers as Sensors, Annual Review of Analytical Chemistry 2: 241-264; Mok, W and Li, Y. (2008): Recent Progress in Nucleic Acid Aptamer-Based Biosensors and Bioassays, Sensors 8: 7050-7084; Song, S., Wang, L., Li, J., Fan, C., Zhao, J. (2008): Aptamer-based biosensors, TrAC Trends in Analytical Chemistry 27: 108-117.

Die Erfindung betrifft in einem weiteren Aspekt eine feste Phase, auch bezeichnet als fester Träger, insbesondere zur Verwendung beim Nachweis, Anreicherung, Abtrennung und/oder Isolierung einer oder mehrerer Verbindungen aus der Gruppe der Fluorchinolone an dem ein erfindungsgemäßes Aptamer immobilisiert ist.

Feste Phasen sind in allen möglichen geometrischen Formen denkbar. Beispiele für feste Träger sind Platten, Streifen, Membranen, Mikrotiterplatten, Filme, Gele, Mikropartikel, Nanopartikel, Nanofasern, insbesondere aus Kohlenstoff, Nanoröhren ("nanotubes"), insbesondere aus Kohlenstoff ("carbon nanotubes"), oder Perlen.

Die Immobilisierung eines erfindungsgemäßen Aptamers an eine feste Phase kann direkt auf der Oberfläche der festen Phase, durch physikalische oder chemische Anbindung erfolgen.

In einer anderen Variante wird ein erfindungsgemäßes Aptamer indirekt, beispielsweise über einen soganannten Linker, an die feste Phase angebunden. In einer speziellen Variante ist der Linker ein Protein, insbesondere ein sogananntes S-Layer Protein. Die Besonderheit von S-Layer-Proteinen besteht in ihrer Fähigkeit, sich selber zu hochgeordneten Nanostrukturen, den sog. S-Layern, zu organisieren. In dieser Art sich selbst organisierende S-Layer-Proteine können eine Schicht auf der Oberfläche eines Substrats bzw. oder festen Phase bilden. Die Proteinschicht ist bevorzugt eine Monolayer (d. h. hat die Dicke eines Proteinmoleküls) oder auch eine Doppelschicht (d. h. hat die Dicke von zwei Proteinmolekülen). Beispielhafte S-Layer-Proteine sind bakterielle oder archaeale Hüllproteine, wie S-Layer-Proteine der Bakteriengattungen Bacillus, Lactobacillus, Lysinibacillus, Geobacillus, Deinococcus, Sporosarcina. Die Bindung der erfindungsgemäßen Aptamere an das selbstorganisierende Protein erfolgt bevorzugt kovalent an freie Aminogruppen oder Carboxylgruppen der Aminosäurenseitenketten (Lysin oder Aspartat- oder Glutamat-Reste) des Proteins. Alternativ erfolgt die Anbindung von Aptameren durch genetische Modifizierung des selbstorganisierenden Proteins, durch Einführung von nukleinsäurebindenden Sequenzen in das Protein, wie z. B. Zinkfingermotiven. Die Anbindung von erfindungsgemäßen Aptameren erfolgt bevorzugt mittels Crosslinker an freie Aminogruppen oder Carboxylgruppen der Aminosäurenseitenketten (Lysin- oder Aspartat- oder Glutamat-Reste) des Proteins. Alternativ kann diese Anbindung jedoch auch durch die Herstellung eines Fusionsproteins, d. h. durch genetische Modifizierung des selbstorganisierenden Proteins erfolgen. Es werden also in dieser Variante erfindungsgemäße Aptamere an Proteine gebunden, die wiederum auf einer Oberfläche eines Trägers oder einer festen Phase eine strukturierte Anordnung bilden. Mit dieser Variante kann eine strukturierte, regelmäßige Anordnung erfindungsgemäßer Aptamerer auf einer Oberfläche realisiert werden.

Besonders geeignete Materialien, aus denen eine feste Phase hergestellt werden kann, sind anorganische Polymere, organische Polymere, Gläser, organische und anorganische Kristalle, Mineralien, Oxide, Keramiken, Metalle, insbesondere Edelmetalle, Kohlenstoff und Halbleiter. Ein besonders geeignetes organisches Polymer ist ein Polymer auf Basis von Polystyrol. Desweiteren sind Biopolymere, vorzugsweise Cellulose, Dextran, Agar, Agarose und Sephadex, die funktionalisiert sein können, insbesondere als Nitrocellulose oder Cyanbromid-Sephadex, als Polymere einsetzbar. Aptamere können an magnetische Kügelchen (Magnetic Beads) gebunden sein, die z.B. Carboxy-terminierte oder Epoxyaktivierte Seitenketten tragen. Des Weiteren ist eine Kopplung von Magnetic Beads an die Ribose oder Desoxyribose der Aptamere, wie z.B. über eine Hydrazon-Bindung, möglich. Die Beispiele für Polymere sind nicht limitierend und andere Moleküle denkbar. Bevorzugte Kombinationen von geometrischer Form und Material sind Gele aus Biopolymeren, insbesondere Agarose, und Gelmatrices aus Dextran und Sephadex, die - beispielsweise in Säulen verpackt - Hohlräume definierter Porengröße ausbilden. In einer speziellen Ausführungsform ist der feste Träger mit immobilisiertem Aptamer eine stationäre Phase für die Affinitätschromatographie, wobei der Träger vorzugsweise die Form von Kugeln oder anderweitig geformten Partikeln hat.

In einer speziellen Ausführungsform ist die feste Phase ein Filtermaterial. Ein Filtermaterial, woran ein erfindungsgemäßes Aptamer immobilisiert ist, kann als biokatalytisch aktives Filtersystem zwecks Nachweis, Anreicherung, Abtrennung und/oder Isolierung einer oder mehrerer Verbindungen aus der Gruppe der Fluorchinolone eingesetzt werden. Eine mögliche Anwendung ist beispielsweise die Behandlung von Wasser, insbesondere Abwasser, zwecks Nachweis, Abtrennung und/oder Isolierung einer oder mehrerer Verbindungen aus der Gruppe der Fluorchinolone.

In einer speziellen Ausführungsform ist die feste Phase ein Teststreifen, der ein oder mehrerere verschiedene erfindungsgemäße Aptamere enthält. Der Teststreifen ist insbesondere einsetzbar für qualitative, halb-quantitative und quantitative Assays, die mit visuellen Nachweisverfahren arbeiten, insbesondere für Lateralfluss-Assays.

Ein Lateralfluss-Assay arbeitet wie folgt: Eine flüssige Probe, die mutmaßlich ein Target für das erfindungsgemäße Aptamer enthält, wird an einer Stelle auf den Streifen aufgebracht bzw. der Streifen an einer Stelle mit der Probe benetzt. Diese Stelle ist die sogenannte Probeauftragszone des Streifens. Der Streifen enthält ein Matrixmaterial, durch welches das flüssige Testmedium und das darin suspendierte oder gelöste Target durch Kapillarwirkung aus einer Probeauftragszone in eine Nachweiszone strömen kann, wo ein nachweisbares Signal oder die Abwesenheit eines solchen Signals auf das Vorhandensein des Targets hinweist.

Der Streifen, der für Lateralfluss-Assays einsetzbar ist, enthält ein oder mehrere erfindungsgemäße Aptamere, beispielsweise in der Probeauftragszone oder zumindest noch örtlich vor der Nachweiszone. Wenn Target in der Testprobe vorhanden ist bildet es mit dem im Streifen vorhandenen Aptamer einen Komplex, der zur Nachweiszone strömt und dort detektiert wird. Anders ausgedrückt strömt das Target innerhalb des Streifens zu dem Aptamer, bildet mit diesem einen Komplex, welcher anschließend zur Nachweiszone strömt.

Die Bindungsreaktion kann auch direkt auf der Auftragsstelle stattfinden, wenn z.B. der Teststreifen das Aptamer enthält und die Aptamer-Target-Bindung direkt mit einem entsprechenden Marker sichtbar gemacht wird.

Vorzugsweise wird ein markiertes Aptamer eingesetzt, wobei jede bereits weiter oben beschriebene Markierung einsetzbar ist. Vorzugsweise wird eine Markierung eingesetzt, die in der Nachweiszone des Teststreifens zu einem visuell detektierbaren Signal führt. Grundsätzlich kann das Vorhandensein oder die Abwesenheit von Target in der Probe durch Nachweis oder fehlenden Nachweis eines markierten Aptamers in der Nachweiszone ermittelt werden.

In einer Ausführungsform eines Teststreifens wird ein Enzym-markiertes Aptamer eingesetzt. In der Probe vorhandenes Target bildet mit dem Enzym-markierten Aptamer einen Komplex, der entlang des Streifens zu einer Nachweiszone strömt, die ein Substrat für den Enzymmarker enthält, der in Anwesenheit des Enzymmarkers eine farbige Reaktion zu erzeugen vermag. Der Streifen enthält vorzugsweise eine weitere Zone, in der Target immobilisiert ist, so dass markiertes Aptamer, das sich aufgrund der Abwesenheit von ausreichendem Target in der Probe nicht mit dem Target vereinigt, erfasst wird und dadurch gehindert wird, die Nachweiszone zu erreichen.

In einer weiteren Ausführungsform funktioniert der Teststreifen nach dem Prinzip eines immunologischen Lateralfluss-Assays, beispielsweise nach dem Prinzip eines Schwangerschaftsteststreifens, wobei ein dort verwendetes markiertes Immunglobulin durch ein erfindungsgemäßes, vorzugsweise markiertes, Aptamer ersetzt ist.

Eine spezielle Variante funktioniert nach folgendem Prinzip: Ein Teststreifen wird mit Probe benetzt und in der Probe vorhandenes Target bindet an ein Farbstoff-markiertes Aptamer. Der Target-Aptamer-Farbstoff-Komplex wandert zur Nachweiszone, in der ein Antikörper fixiert ist, welcher ebenfalls an das Target bindet. Der immobilisierte Antikörper bindet den wandernden Target-Aptamer-Farbstoff-Komplex in der Nachweiszone und diese wird angefärbt. Überschüssiges Farbstoff-markiertes Aptamer wandert weiter zu einer Kontrollzone, in der eine Substanz fixiert ist, beispielsweise eine Verbindung aus der Gruppe der Fluorchinolone, die das Farbstoff-markierte Aptamer bindet, und dadurch angefärbt wird.

Der Teststreifen ist aus jedem Material herstellbar, das mit einer Probe benetzbar ist und in das ein erfindungsgemäßes Aptamer eingebracht werden kann. Insbesondere bevorzugt sind Materialien durch die eine Probe und ein darin enthaltenes Target durch Kapillarwirkung strömen können. Beispiele sind Nitrozellulose, Nitrozellulosemischungen mit Polyester oder Zellulose, unbeschichtetes Papier, poröses Papier, Viskosefilament, Glasfaser, Acrylnitrilcopolymer oder Nylon, sowie alle weiteren Materialien, die bei Lateralfluss-Assays üblich sind.

Der Teststreifen kann bereits als solcher verwendet werden, um das Vorhandensein von Target in einer Probe festzustellen. Zur Anwendung kann der Streifen in eine Probe eingetaucht werden, im Falle eines Lateralfluss-Assays beispielsweise mit nur einem Ende, das dann als Probeauftragszone dient.

Gegenstand der Erfindung ist auch eine Lateralfluss-Assay-Vorrichtung, die den oben beschriebenen Teststreifen aufweist. Die Vorrichtung kann neben dem Teststreifen beispielsweise ein Gehäuse aufweisen, in das der Teststreifen eingebettet ist und das eine, vorzugsweise verschließbare, Öffnung zur Probenauftragszone des Teststreifens aufweist, sowie Aussparungen zur Beobachtung einer Nachweis und ggf. Kontrollzone. Ein solcher Aufbau ist aus dem Gebiet der Schwangerschaftstests bekannt und entsprechend sind bekannte Aufbauten von Lateralfluss-Assay-Vorrichtung auch in der vorliegenden Erfindung verwendbar.

In einer anderen speziellen Ausführungsform bilden der feste Träger und das Aptamer ein Microarray oder einen sogenannten "DNA-Chip", der einkanalig oder mehrkanalig sein kann. Im Microarray können auf mehreren Array-förmig angeordneten Messpunkten ein oder mehrere Aptamere sowie Referenzmaterialien zur Grundsignalkompensation bzw. Funktionstestung angeordnet sein. In einem Mehrkanal-Chip erfolgt diese Anordnung in den einzelnen Kanälen. Dadurch ist die parallele Mehrfachmessung einer Probe, oder bei verschiedenen Aptameren, die parallele Messung unterschiedlicher Analyte in einer Probe möglich.

Die Immobilisierung von Aptamer an fester Phase kann auf verschiedene Art und Weise erfolgen und auf jegliche Art und Weise, die dem Fachmann zur Immobilisierung von DNA an Feststoffen bekannt ist. Bekannte Möglichkeiten wurden bereits zuvor in dieser Beschreibung genannt. Die Immobilisierung von Aptameren auf Nanopartikeln ist z.B. beschrieben in WO2005/13817. Eine feste Phase aus Papier oder einem porösen Material kann mit dem Aptamer in flüssiger Phase benetzt und die flüssige Phase anschließend verflüchtigt werden, so dass das Aptamer in dem Papier bzw. dem porösen Material zurückbleibt.

In einem weiteren Aspekt betrifft die vorliegende Erfindung auch ein Kit umfassend ein erfindungsgemäßes Aptamer.

Das Kit umfasst vorzugsweise ein erfindungsgemäßes Aptamer, insbesondere ein markiertes erfindungsgemäßes Aptamer bzw. eine erfindungsgemäße Aptamersonde, sowie weitere Komponenten für die mit dem Kit beabsichtigte Reaktion oder das mit dem durchzuführende Verfahren, beispielsweise Komponenten für ein beabsichtigtes Nachweis Anreicherungs-, Abtrennungs- und/oder Isolierungsverfahren. Beispiele sind Pufferlösungen, Substrate für eine Farbreaktion, Farbstoffe oder enzymatische Substrate. Das Aptamer und/oder weitere Komponenten können an einer festen Phase immobilisiert sein. Beispielhafte Formen und Materialien für feste Phasen wurden bereits an anderer Stelle in dieser Beschreibung genannt. Die feste Phase kann beispielsweise in Form eines (Micro)-Arrays oder einer Mikrotiterplatte-bereitgestellt werden. Die feste Phase kann im konkreten Fall eine immobilisierte Fängersubstanz für das Target aufweisen, insbesondere einen immobilisierten Antikörper, der zur Anbindung von Target dient. Die immobilisierte Fängersubstanz ist vorzugsweise in Form eines (Micro)-Arrays, in einer Mikrotiterplatte oder in Chips angeordnet.

Das Kit dient vorzugsweise zur Durchführung eines erfindungsgemäßen Nachweis-, Anreicherungs, Abtrennungs- und/oder Isolierungsverfahrens, oder zur Durchführung anderweitiger Assays unter Zuhilfenahme eines erfindungsgemäßen Aptamers. Insofern wird auf die vorangehende Offenbarung Bezug genommen.

In dem Kit kann das Aptamer in verschiedensten Formen bereitgestellt werden, beispielsweise gefriergetrocknet oder in einem flüssigen Medium.

Die Erfindung betrifft auch ein Messgerät zum Nachweis einer oder mehrerer Verbindungen aus der Gruppe der Fluorchinolone. Das Messgerät weist ein oder mehrere verschiedene der zuvor beschriebenen Aptamere, eine Aptamersonde wie zuvor beschrieben oder einen Biosensor wie zuvor beschrieben auf. Darüber hinaus kann das Messgerät unter anderem eine Probeentnahmeeinrichtung, eine Signalverarbeitungseinrichtung, eine Anzeigevorrichtung zum Ablesen von Messergebnissen oder Messwerten, eine Datenverarbeitungseinrichtung, einen Datenspeichereinrichtung und Schnittstellen zur Verbindung mit externen Geräten oder Speichermedien aufweisen.

Mit dem Messgerät können je nach Ausgestaltung qualitative, quantitative und/oder halb-quantitative analytische Informationen über das zu messende Target gewonnen werden. Mit dem Messgerät können die zuvor erläuterten Nachweisverfahren durchgeführt werden. Es ist insbesondere zum Einsatz in der Lebensmittel-, Wasser- und Umweltanalytik, der Wasserbehandlung und der Wasseraufbereitung, insbesondere von Trinkwasser und Abwasser, in der Diagnostik sowie zur Verwendung in Krankenhäusern und Pflegeeinrichtungen geeignet.

Das Messgerät ist insbesondere ein transportables Messgerät, das vor Ort eingesetzt werden kann, beispielsweise ein leichtes Messgerät im Taschenformat.

Die Probeentnahmeeinrichtung des Messgeräts kann auf verschiedene Art und Weise aufgebaut sein. In einer Variante ist die Probeentnahmeeinrichtung eine Kapillare oder ein poröser Streifen, im dem Flüssigkeiten aufgesaugt werden können. Eine solche Probeentnahmevorrichtung wird zur Probeentnahme üblicherweise in eine flüssige Probe eingetaucht. Durch Kapillarkraft wird die Probe zu dem gewünschten Ort im Messgerät transportiert, an dem sich das Aptamer befindet und wo die Nachweisreaktion stattfinden kann. Beispielsweise wird die Probe zu einem das Aptamer aufweisenden Biosensor transportiert, welcher seinerseits ein Messsignal erzeugt.

In einer anderen Variante weist die Probeentnahmeeinrichtung einen Schlauch oder ein Röhrchen sowie eine Pumpe auf. Mit der Pumpe wird eine flüssige Probe angesaugt und die Probe zu dem gewünschten Ort im Messgerät transportiert, an dem sich das Aptamer befindet und wo die Nachweisreaktion stattfinden kann. Beispielsweise wird die Probe zu einem das Aptamer ausweisenden Biosensor transportiert, welcher seinerseits ein Messsignal erzeugt.

Die Erfindung betrifft in einem Aspekt auch die Verwendung einer zuvor beschriebenen Sonde, einer zuvor beschriebenen festen Phase, eines zuvor beschriebenen Biosensors, eines zuvor beschriebenen Teststreifens, einer zuvor beschriebenen Lateralfluss-Assay-Vorrichtung, eines zuvor beschriebenen Kits, oder eines zuvor beschriebenen Messgeräts zum Nachweis einer oder mehrerer Verbindungen aus der Gruppe der Fluorchinolone, oder zur Anreicherung, Abtrennung und/oder Isolierung einer oder mehrerer Verbindungen aus der Gruppe der Fluorchinolone. Hierbei wird auf alle zuvor beschriebenen Verfahrensvarianten Bezug genommen.

### KURZBESCHREIBUNG DER FIGUREN

- Fig. 1: Aufbau und Messprinzip eines Biosensors;
- Fig. 2: Sensorgramm, Änderungen des Resonanzwinkels gegen die Zeit;
- Fig. 3: Schema des angewandten Capture SELEX-Prozesses zur Aptamergewinnung;
- Fig. 4: Bindungsversuch, Targets: Einzelsubstanzen aus Pharma-Mischung 1;
- Fig. 5: Ergebnis der SELEX Runden 2-5 (R2-R5);
- Fig. 6: Ergebnis der SELEX Runden 6-9 (R6-R9);
- Fig. 7: Ergebnis der SELEX Runde 10 (R10);
- Fig. 8a: Ergebnis der SELEX Runden 11 und 12 (R11, R12);
- Fig. 8b: Ergebnis der SELEX Runden 11' und 12' (R11', R12');
- Fig. 9: Bindung der Fluorchinolon-Aptamere an verschiedene Antibiotika;
- Fig. 10: Bindung ausgewählter Aptamere an Ofloxacin-Enantiomere und Negativkontrollen;
- Fig. 11: Aptamer-Target-Bindung in Realproben;

### KURZBESCHREIBUNG DER SEQUENZEN

| | |
|---|---|
| SEQ ID NO: 1-13 | Einzelaptamere der Gruppe Q1; |
| SEQ ID NO: 14 | zusammenfassende Gruppensequenz der Gruppe Q1; |
| SEQ ID NO: 15-19 | Einzelaptamere der Gruppe Q2; |
| SEQ ID NO: 20 | zusammenfassende Gruppensequenz der Gruppe Q2; |
| SEQ ID NO: 21 | Einzelaptamer der Gruppe Q8; |
| SEQ ID NO: 22 | zusammenfassende Gruppensequenz der Gruppe Q8; |
| SEQ ID NO: 23-24 | Einzelaptamere der Gruppe Q7; |
| SEQ ID NO: 25 | zusammenfassende Gruppensequenz der Gruppe Q7; |
| SEQ ID NO: 26 | Einzelaptamer der Gruppe Q10; |
| SEQ ID NO: 27 | zusammenfassende Gruppensequenz der Gruppe Q10; |
| SEQ ID NO: 28-29 | Einzelaptamere der Gruppe Q4; |
| SEQ ID NO: 30 | zusammenfassende Gruppensequenz der Gruppe Q4; |
| SEQ ID NO: 31-32 | Einzelaptamere der Gruppe Q6; |
| SEQ ID NO: 33 | zusammenfassende Gruppensequenz der Gruppe Q6; |
| SEQ ID NO: 34-35 | Einzelaptamere der Gruppe Q3; |
| SEQ ID NO: 36 | zusammenfassende Gruppensequenz der Gruppe Q3; |
| SEQ ID NO: 37 | Einzelaptamer und Gruppensequenz der Gruppe Q5; |
| SEQ ID NO: 38 | Einzelaptamer der Gruppe Q9; |
| SEQ ID NO: 39 | zusammenfassende Gruppensequenz der Gruppe Q9; |
| SEQ ID NO: 40 | Einzelaptamer der Gruppe Q11; |
| SEQ ID NO: 41 | zusammenfassende Gruppensequenz der Gruppe Q11; |
| SEQ ID NO: 42 | Einzelaptamer der Gruppe Q12; |
| SEQ ID NO: 43 | zusammenfassende Gruppensequenz der Gruppe Q12; |
| SEQ ID NO: 44-45 | Einzelaptamere der Gruppe Q14; |
| SEQ ID NO: 46 | zusammenfassende Gruppensequenz der Gruppe Q14; |
| SEQ ID NO: 47 | Einzelaptamer der Gruppe Q15; |
| SEQ ID NO: 48 | zusammenfassende Gruppensequenz der Gruppe Q15; |
| SEQ ID NO: 49-50 | Einzelaptamere der Gruppe Q16; |
| SEQ ID NO: 51 | zusammenfassende Gruppensequenz der Gruppe Q16; |
| SEQ ID NO: 52 | Einzelaptamer und Gruppensequenz der Gruppe Q13; |
| SEQ ID NO: 53 | Einzelaptamer der Gruppe Q17; |
| SEQ ID NO: 54 | zusammenfassende Gruppensequenz der Gruppe Q17; |
| SEQ ID NO: 55-56 | Einzelaptamere der Gruppe Q18; |
| SEQ ID NO: 57 | zusammenfassende Gruppensequenz der Gruppe Q18; |
| SEQ ID NO: 58-59 | Einzelaptamere der Gruppe Q19; |
| SEQ ID NO: 60 | zusammenfassende Gruppensequenz der Gruppe Q19; |
| SEQ ID NO: 61 | Einzelaptamer der Gruppe Q20; |
| SEQ ID NO: 62 | zusammenfassende Gruppensequenz der Gruppe Q20; |
| SEQ ID NO: 63-64 | Einzelaptamere der Gruppe Q21; |
| SEQ ID NO: 65 | zusammenfassende Gruppensequenz der Gruppe Q21; |
| SEQ ID NO: 66 | Einzelaptamer der Gruppe Q22; |
| SEQ ID NO: 67 | zusammenfassende Gruppensequenz der Gruppe Q22; |
| SEQ ID NO: 68 | Einzelaptamer der Gruppe Q23; |
| SEQ ID NO: 69 | zusammenfassende Gruppensequenz der Gruppe Q23; |
| SEQ ID NO: 70-71 | Einzelaptamere der Gruppe Q24; |
| SEQ ID NO: 72 | zusammenfassende Gruppensequenz der Gruppe Q24; |
| SEQ ID NO: 73 | Einzelaptamer der Gruppe Q25; |
| SEQ ID NO: 74 | zusammenfassende Gruppensequenz der Gruppe Q25; |
| SEQ ID NO: 75 | Einzelaptamer der Gruppe Q26; |
| SEQ ID NO: 76 | zusammenfassende Gruppensequenz der Gruppe Q26; |
| SEQ ID NO: 77 | Einzelaptamer und Gruppensequenz der Gruppe Q27; |
| SEQ ID NO: 78 | Einzelaptamer und Gruppensequenz der Gruppe Q28; |
| SEQ ID NO: 79 | Einzelaptamer der Gruppe Q29; |
| SEQ ID NO: 80 | zusammenfassende Gruppensequenz der Gruppe Q29; |
| SEQ ID NO: 81 | Einzelaptamer der Gruppe Q30; |
| SEQ ID NO: 82 | zusammenfassende Gruppensequenz der Gruppe Q30; |
| SEQ ID NO: 83 | Sense-Primersequenz (Forward Primer, 5'-Primer); |
| SEQ ID NO: 84 | komplementäre Sequenz zu Antisense-Primersequenz; |
| SEQ ID NO: 85 | komplementäre Sequenz zu Fängersequenz für Capture SELEX; |
| SEQ ID NO: 86 | Antisense-Primersequenz (Reverse Primer, 3'-Primer); |
| SEQ ID NO: 87 | Fängersequenz für Capture SELEX |

Im Folgenden wird die Erfindung anhand von nicht limitierenden Beispielen für konkrete Ausführungsformen näher erläutert. Die Beispiele, die sich direkt oder indirekt auf die SEQ ID NO: 14, 20, 36, 30, 37, 33, 25, 22, 39, 27, 41, 43 und 52 beziehen, sind erfindungsgemäß, auch Fragmente davon, die Beispiele zu anderen Sequenzen sind Vergleichsbeispiele. In den Beispielexperimenten werden Standardreagenzien und Puffer verwendet, die frei von Kontaminationen sind.

### BEISPIEL 1: Präparation der Magnetic Beads.

Dynabeads® M-280 Streptavidin Magnetic Beads (Dynal Biotech, Norwegen) wurden nach Herstellerangaben verwendet. Alle für die folgenden Schritte notwendigen 1,5 mL-Reaktionsgefäße wurden vor ihrer Verwendung gewaschen: 1 x mit Bindungspuffer mit Detergenz-Zusatz: (BB+(C): 20 mmol L⁻¹ Tris-HCl pH 7.6, 100 mmol L⁻¹ NaCl, 2 mmol L⁻¹ MgCl₂, 1 mmol L⁻¹ CaCl₂, 0,02 % Tween 20 sowie 2 x mit Bindungspuffer ohne Zusatz von Tween (BB(C)). 4 × 10⁹ Magnetic Beads wurden 1 h bei 21 °C und leichtem Schütteln mit 600 pmol der Fängersequenzen in 700 µL Puffer inkubiert. Danach folgten Waschschritte: 3 x in 10 mmol L⁻¹ Tris-HCl pH 7.5, 1 mmol L⁻¹ EDTA, 2 mol L⁻¹ NaCl und 3 x in BB(C). Die Abtrennung der Beads von den Waschlösungen erfolgte jeweils unter Verwendung eines Magnet-Separationsstandes (Promega, Deutschland). Die mit den Fängersequenzen modifizierten Beads wurden dann in 4200 µL BB(C) resuspendiert und bei 4 °C aufbewahrt.BEISPIEL 2: In-vitro Selektion (Capture-SELEX).

Die DNA-Aptamer-Selektion wurde unter Anwendung des Capture-SELEX-Prozesses durchgeführt. Eingefügte Fluoreszenz-Marker erlauben die Quantifizierung der DNA nach verschiedenen Schritten des Prozesses mittels Fluoreszenz-Messung. Die Bindung der ssDNA-Oligomeren aus der Ausgangsbibliothek bzw. ihrer Derivate an Magnetic Beads erfolgt über die Fängersequenz (Capture sequence). Die Fängersequenz ist 12 Basen lang und zu einem Bereich in der ssDNA der Ausgangsbibliothek komplementär. Mit Hilfe der Fängersequenz findet eine reversible Bindung zwischen Fänger und ssDNA statt. An ihrem 5'-Ende trägt die Fängersequenz über einen Hexaethylenglycol(HEGL)-Rest gebundenes Biotin, welches für die Ankopplung der Fängersequenz an die Streptavidinmodifizierten Magnetic Beads verantwortlich ist.

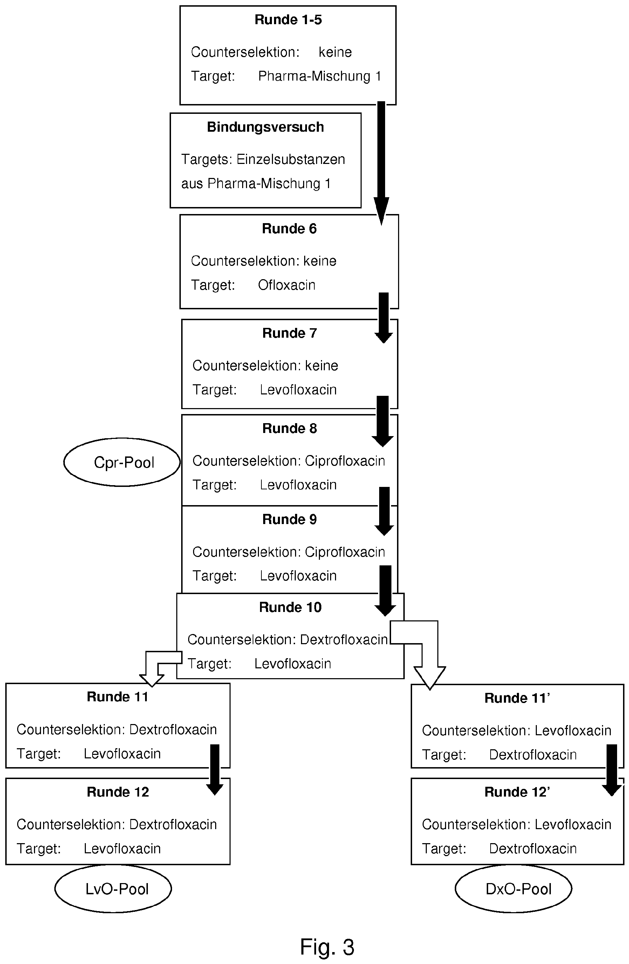

Die Targetsubstanzen (Ofloxacin, Sulfamethoxazol, Metoprolol, und Bronopol, in Fig. 3 bezeichnet als "Pharma-Mischung 1 "), für welche DNA-Aptamere selektiert werden sollten, lagen frei in Lösung in einer Mischung mit einer Konzentration von je 1 mM vor und standen für eine Bindung mit der ssDNA zur Verfügung. Ofloxacin ist ein Vertreter der Fluorchinolone. Sulfamethoxazol, Metoprolol, Bronopol sind Vertreter anderer Wirkstoffklassen. Targetspezifische Oligomere sollten sich durch Ausbildung einer definierten zwei- oder dreidimensionalen Struktur von der Fängersequenz abkoppeln und zusammen mit dem Target in Lösung zu finden sein.

Ausgangspunkt des Selektionsprozesses war eine ssDNA-Bibliothek mit der grundsätzlichen Struktur: 5'-ATACCAGCTTATTCAATT-N₁₀-TGAGGCTCGATC-N₄₀-ACAATCGTAATCAGTTAG -3', wobei N für eine beliebige der vier Basen A, T, C oder G steht. Folgende modifizierte Primer wurden in der Amplifizierung der Oligomere mittels PCR eingesetzt: Primer AP60 mit der Sequenz: 5' Fluorescein-ATACCAGCTTATTCAATT-3' und Primer TER-AP30 mit der Sequenz: 5' dA₂₀-HEGL-CTAACTGATTACGATTGT-3'. (Alle chemisch synthetisiert von Microsynth, Schweiz.). Die Bibliothek wurde mittels PAGE gereinigt. Primer wurden mit HPLC gereinigt. Die Fängersequenz hatte folgende Struktur: 5' Bio-GTC-HEGL-GATCGAGCCTCA-3' und wurde von IBA (Deutschland) hergestellt sowie HPLC gereinigt.

Es wurden aufeinanderfolgende Runden des SELEX-Prozesses durchgeführt, die sich aus den Schritten
(i) Bindung der ssDNA-Bibliothek bzw. der in der vorhergehenden Runde selektierten ssDNA an die mit der Fängersequenz modifizierten Beads,
(ii) Abtrennen der ungebundenen ssDNA,
(iii) Ablösen von nur schwach gebundenen Oligomeren in einem Temperaturschritt, Bedingungen: 15 min, 28°C, Puffer
(iv) Hintergrund-Elution in Puffer bei gleichen Bedingungen wie bei der nachfolgenden Target-Elution jedoch ohne Target, Bedingungen: 45 min, 21 °C, Puffer,
(v) Target-Bindung und Elution in Anwesenheit der Mischung der Targetsubstanzen, Bedingungen: 45 min, 21 °C, Puffer und Target,
(vi) Vervielfältigung der eluierten ssDNA mittels PCR,
(vii) Trennung des Doppelstranges aus der PCR und Gewinnung des relevanten Einzelstranges, zusammensetzten. In jeder Runde wurden 10⁸ mit der Fängersequenz modifizierten Magnetic Beads eingesetzt (Ausnahme: 1. Runde, 10⁹ Beads).

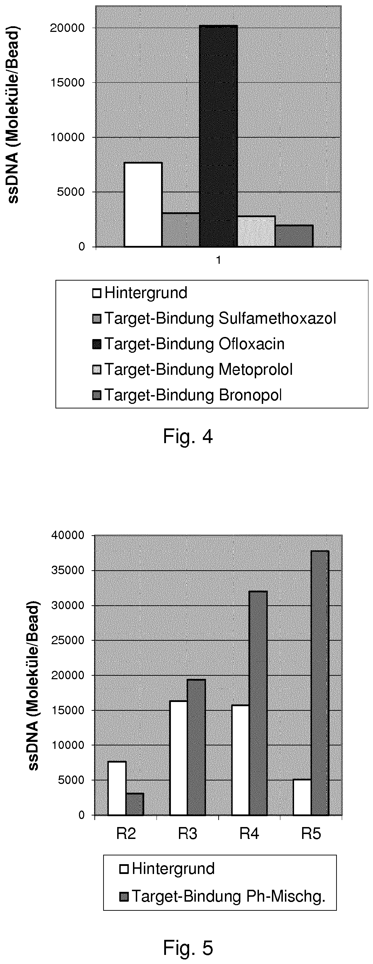

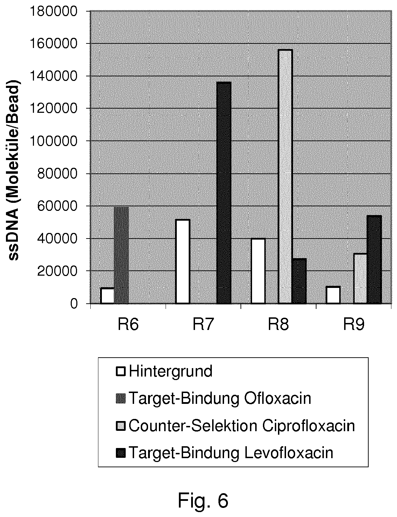

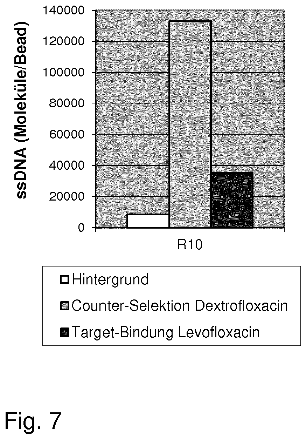

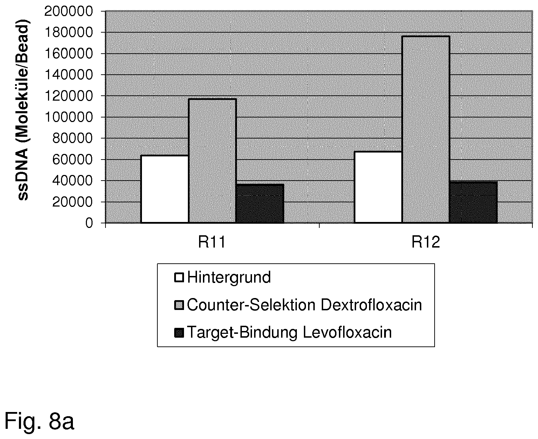

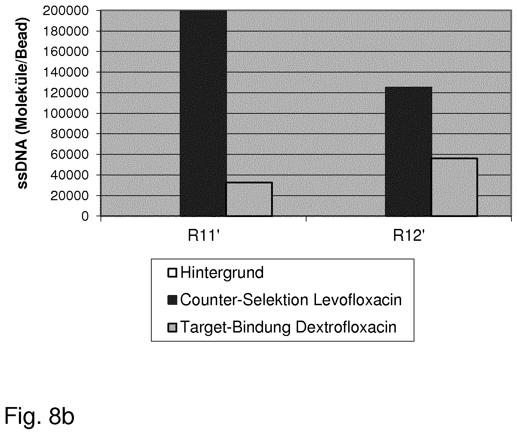

Eine Übersicht über die Vorgehensweise im SELEX-Prozess ist in Fig. 3 angegeben. Nach der 5. Runde wurden die selektierten Oligonukleotide auf ihre Bindungsspezifität für die vier in der Mischung enthaltenen Targetsubstanzen getestet. Dabei wurde eine eindeutige Anreicherung von Ofloxacin -bindender ssDNA gefunden. Daraufhin wurde in der 6. Runde Ofloxacin, in der 7. Runde Levofloxacin als Target einsetzt (je 1 mM in BB(C)). In Runde 8 wurde 1 mM Ciprofloxacin für die Targetelution in einer Counterselektion, auch bezeichnet als Vorselektion, eingesetzt. Der somit erhaltene ssDNA-Pool (Cpr-Pool) wurde kloniert und sequenziert (siehe Beispiel 3). Die modifizierten Beads wurden innerhalb der Runde 6 jedoch weiter behandelt. Es erfolgten 3 weitere Waschschritte mit BB(C) und eine weitere Targetelution mit 1 mM Levofloxacin. Runde 9 war ebenso gestaltet wie Runde 8. In Runde 10 erfolgte zunächst eine Targetelution (Counterselektion) mit Dextrofloxacin. Der eluierte ssDNA-Pool wurde für die Runden 11' und 12' aufbewahrt. Die modifizierten Beads wurden innerhalb dieser Runde jedoch weiter behandelt. Es erfolgten 3 weitere Waschschritte mit BB(C) und eine weitere Targetelution mit 1 mM Levofloxacin. In zwei weiteren Runden (Runde 11 und 12) wurde ebenso verfahren wie in Runde 10. Der mit Levofloxacin eluierte ssDNA-Pool der Runde 12 (LvO-Pool) wurde kloniert und sequenziert (siehe Beispiel 3). Der aufbewahrte ssDNA-Pool aus Runde 10 (Elution mit Dextrofloxacin) wurde in zwei weiteren Runden (Runden 11' und 12') wie folgt behandelt: Es erfolgte zunächst eine Negativelution mit 1 mM Levofloxacin, danach eine Targetelution mit 1 mM Dextrofloxacin. Die mit Dextrofloxacin eluierten ssDNA der Runde 12' (DxO-Pool) wurde kloniert und sequenziert (siehe Beispiel 3). Fig. 4 zeigt das Resultat des Bindungsversuchs an die Einzelsubstanzen aus der Pharma-Mischung 1, nämlich Ofloxacin, Sulfamethoxazol, Metoprolol, und Bronopol. Die Fig. 5, 6, 7, 8a und 8b zeigen die Ergebnisse der einzelnen Selektionsrunden.

### BEISPIEL 3: Klonierung and Sequenzierung.

Nach den jeweils letzten SELEX-Runden für jeden ssDNA-Pool (8. Runde für Cpr-Pool; 12. Runde für LvO-Pool; 12'. Runde für DxO-Pool, vgl. Fig. 3) wurde die selektierte ssDNA in der PCR mit unmodifizierten Primern amplifiziert, um sie danach in den Vektor pCR2.1-TOPO zu klonieren. *E. coli* TOP10 Zellen (TOPO TA Cloning Kit; Invitrogen, U.K.) wurden durch Aufnahme dieses Vektor-Konstruktes transformiert. Unter Verwendung des QIAprep 96 Turbo Miniprep Kit (Qiagen, Deutschland) wurde die Plasmid-DNA aus 96 der erhaltenen Klone präpariert und zur Sequenzierung gegeben (Microsynth, Schweiz). Zusätzlich erfolgte eine Sequenzierung für alle drei Pools (Cpr-, LvO-, DxO-Pool) mittels Next Generation Sequenzierung (GS FLX Titanium Amplicon Sequencing, 1/8 Picotiterplatte), unter Zuhilfenahme der Methode der Emulsions-PCR für eine Vereinzelung der einzelnen ssDNA-Moleküle in den ssDNA-Pools durch die Firma Eurofins MWG Operon GmbH. Sequenz-Analysen und Alignments wurden unter Verwendung der Webseite ClustalW2 (http://www.ebi.ac.uk/Tools/msa/clustalw2/) durchgeführt. Die Analyse der Sekundärstruktur einiger Aptamere erfolgte mittels des im Internet verfügbaren Faltungsprogramms mfold, das auf einem Energie-Minimierungs-Algorithmus beruht.

Eine Übersicht der aus der Klonierung erhaltenen Aptamersequenzen ist in der Tabelle 1 angegeben. Die Primersequenz (Sense) am 5'-Ende ist ATACCAGCTTATTCAATT (SEQ ID NO: 83) und die komplementäre Sequenz für den Antisense-Primer am 3'-Ende ist ACAATCGTAATCAGTTAG (SEQ ID NO: 84). Die zur Fängersequenz komplementäre Sequenz (Docking Sequenz) für den Capture-SELEX Prozess ist TGAGGCTCGATC (SEQ ID NO: 85). Die zur Fängersequenz komplementäre Sequenz wird bisweilen auch als "Fängersequenz" bezeichnet. In der vorliegenden Anmeldung ist die Fängersequenz die Sequenz des Nukleotids, das an ein Magnetic Beads angekoppelt ist. Diese Sequenzen, und davon abgewandelte Sequenzen, sind jeweils in Fettschrift dargestellt. Die Sequenzen der SEQ ID Nos: 83, 84 und 85 sind Bestandteil der Aptamere, vorbehaltlich verkürzter Sequenzen, wie in der vorangehenden allgemeinen Beschreibung definiert.

Einige Aptamere lassen sich aufgrund ihrer Sequenzen in Gruppen einteilen, bezeichnet mit Q1, Q2 etc. Die jeweils bei einer Gruppe erste genannte Sequenz ist die am häufigsten aufgetretene und wird als Stellvertreter der Gruppe bezeichnet. Wie ersichtlich, bestehen einige sogenannte Gruppen nur aus einer einzelnen Sequenz, werden aber dennoch als "Gruppe" bezeichnet. In diesem Fall ist die einzige Sequenz der Gruppe gleichzeitig der Stellvertreter der Gruppe. Innerhalb einer Gruppe im Vergleich zum Stellvertreter einer Gruppe abweichende Nukleotide sind unterstrichen gekennzeichnet. Die in einer Gruppe zusammengefassten Sequenzen sind bis auf Punktmutationen und Deletionen identisch bzw. vom Stellvertreter abzuleiten. Die Aptamernummer des Stellvertreters einer Gruppe ist unterstrichen gekennzeichnet (siehe z.B. Aptamer Nr. Cpr4/Cp1 für Gruppe Q1). Unterstrichene Nukleotide oder Deletionen (-) innerhalb der komplementären Sequenz zur Fängersequenz bedeuten eine Abwandlung gegenüber der ursprünglichen komplementären Sequenz TGAGGCTCGATC (SEQ ID NO: 85) zur Fängersequenz. Unterstrichene Nukleotide oder Deletionen (-) innerhalb der Primersequenz (Sense) oder innerhalb der komplementären Sequenz für den Antisense-Primer bedeuten eine Abwandlung gegenüber den ursprünglichen Sequenzen (SEQ ID Nos 83 und 84). Doppelt unterstrichene Nukleotide zeigen eine Sequenzveränderung, die nur bei dem Next Generation Sequencing festgestellt wurde. Diese Veränderungen wurden bei den Gruppensequenzen (Tab. 3) nicht berücksichtigt.

**Tabelle 1: Variabilität innerhalb der gefundenen Sequenzgruppen**

| Oligonukleotid (Aptamer) | Sequenz | SEQ ID NO |
|---|---|---|
| **Gruppe Q1** | | |
| Cpr4/Cp1 | **ATACCAGCTTATTCAATT**CGATGGTAAG**TGAGGTTCG-TC**CCTTTAATAAACTCGATTAGGATCTCGTGAGGTGTGCTCT**ACAATCGTAATCAGTTAG** | 1 |
| Cpr37 | **ATACCAGCTTATTCAATT**CGATGGTAAG**TGAGGTTCG-TC**CCTTTAATAAACTCGATTAGGATCTCGTGAGGTGTGCTCT**ACAATCGTAATCAG-TAG** | 2 |
| Cpr41 | **ATACCAGCTTATTCAATT**CGATGGTAAG**TGAGGTTCG-TC**CCTTTAATAAACTCGATTAGGATCTCGTGAGGTGTGCTCT**ACAATCGTAATCAGTTNG** | 3 |
| Cpr13 | **ATACCAGCTTATTCAATT**CGATGGTAAG**TGAGGTTCG-TC**CCTTTAATAAACTCGATTAGGATCTCGTGAGGTGTGCTCC**ACAATCGTAATCAGTTAG** | 4 |
| Cpr29 | **ATACCAGCTTATTCAATT**CGATGGTAAG**TGAGGTTCG-TC**CCTTTAATAAACTCGATTAGGATCTCGTGAGGTGTGCTTT**ACAATCGTAATCAGTTAG** | 5 |
| Cpr85 | **ATACCAGCTTATTCAATT**CGATGGTAAG**TGAGGTTCG-TC**CCTTTAATAAACTCGATTAAGATCTCGTGAGGTGTGCTCT**ACAATCGTAATCAGTTAG** | 6 |
| LvO16 | **ATACCAGCTTATTCAATT**CGATGGTAAG**TGAGGTTCG-TC**CCTTTAATAAACTCGATTAGGATCTCGTGAGGTGTGTTCC**ACAATCGTAATCAGTTAG** | 7 |
| LvO24 | **ATACCAGCTTATTCAATT**CGATGGTA-T**TGAGGTTCG-TC**CCTTTAATAAACTCGATTAGGATCTCGTGAGGTGTGCTCT**ACAATCGTAATCAGTTAG** | 8 |
| LvO91 | **ATACCAGCTTATTCAATT**AGATGGTAAG**TGAGGTTCG-TC**CCTTTAATAAACTCGATTAGGATCTCGTGAGGTGTGCTCT**ACAATCGTAATCAGTTNG** | 9 |
| *LvO Clust49* | **ATACCAGCTTATTCAATT**CGATGGTAAG**TGAGGTTCG-TC**CCTTTTAATAAAACTCGATTAGGATCTCGTGAGGTGTGCTCT**ACAATCGTAATCAGTTAG** | 10 |
| *DxO Clust12* | **ATACCAGCTTATTCAATT**CGATGGTAAG**TGAGGTTCG-TC**CCTTTAATAAACTCGATTAGGATCTCGTGAGGTGTGCTCT**ACAATC**C**GTAATCAGTTAG** | 11 |
| DxO7 | **ATACCAGCTTATTCAATT**CGATGGTAAG**TGAGGTTCG-TC**CCTTTAATAAACTCGATTAGGATCTCGTGAGGTGTGCTCT**ACAATCGTAATCAGTTAG** | 12 |
| DxO71 | **ATACCAGCTTATTCAATT**GGATGGTAAG**TGAGGTTCG-TC**CCTTTAATAAACTCGATTAGGATCTCGTGAGGTGTGCTCT**ACAATCGTAATCAGTTAG** | 13 |
| | | |
| **Gruppe Q2** | | |
| LvO3/Lv1 | **ATACCAGCTTATTCAATT**GCAGGGTATC**TGAGGCTTGATC**TACTAAATGTCGTGAGGCATTGCTATTGGCGTTGACACGT**ACAATCGTAATCAGTTAG** | 15 |
| LvO1 | **ATACCAGCTTATTCAATT**GCAGGGTATC**TGAGGCTTGATC**TACTAAATGTCGTGAGGCATTGCTATTGGCGTTGACACGT**ACAATCGTAATCAGTTAG** | 16 |
| LvO52 | **ATACCAGCTTATTCAATT**GCAGGGTATC**TGAGGCTTGATC**TACTAAATGTCGTGGGGCATTGCTATTGACGTTGACACGT**ACAATCGTAATCAGTTAG** | 17 |
| *LvO Clust10* | **ATACCAGCTTATTCAATT**GCAGGGTATC**TGAGGCTTGATC**TACTAAAATGTCGTGGAGGCATTGCTATTGGCGTTGACACGT**ACAATCGTAATCAGTTAG** | 18 |
| Cpr93 | **ATACCAGCTTATTCAATT**GCAGGGTATC**TGAGGCTTGATC**TACTAAATGTCGTGGGGCATTACTATTGGCGTTGACACGT**ACAATCGTAATCAGTTAG** | 19 |
| | | |
| **Gruppe Q3** | | |
| DxO20/Dx4 | **ATACCAGVTTATTCAATT**CAGGAGGCTC**TGAGGTTCGATC**CTACTGGCTGATAAGCTTGGTGTCGGTTGCATTCACCGTGG**ACAATCGTAATCAGTTAG** | 34 |
| Cpr68 | **ATACCAGCTTATTCAATT**CAGGAGGCTC**TGAGGTTCGATC**CTACTGGCTGATAGCTTGGTGTCGGTTGCATTCACCGTGC**ACAATCGTAATCAGTTAG** | 35 |
| **Gruppe Q4** | | |
| LvO25/Lv2 | **ATACCAGCTTATTCAATT**GGCCACAATT**TGAGGCT-GATC**GCATTTTGCTCGATTGAGGCTGTTACTGAGGCGTTCTTGT**ACAATCGTAATCAGTTAG** | 28 |
| LvO89 | **ATACCAGCTTATTCAATT**GTCCACAATT**TGAGGCT-GATC**GCATTTTGCTCGATTGAGGCTGTTACTGAGGCGTTCTTGT**ACAATCGTAATCAGTTAG** | 29 |
| | | |
| **Gruppe Q5** | | |
| Cpr2/Cp5 | **ATACCAGCTTATTCAATT**AGAGGTGAGA**TGAGGCTCGATC**TCCAACCTTCTGTGAAATGCGGCCGGAAGGGTATGTTTCG**ACAATCGTAATCAGTTAG** | 37 |
| | | |
| **Gruppe Q6** | | |
| LvO7/Lv7 | **ATACCAGCTTATTCAATT**AAGCGGGGGA-**TGAGGTTCGATC**TAGTTTAGGCTCGTTACATAGTGTTTCAAGTGTTCGGTCG**ACAATCGTAATCAGTTAG** | 31 |
| *LVO Clust45* | **ATACCAGCTTATTCAATT**AAGCGGGGGAG**TGAGGTTCGATC**TAGTTTAGGTCTCGTTACATAGTGTTTCAAGTGTTCGGTCG**ACAATCGTAATCAGTTAG** | 32 |
| | | |
| **Gruppe Q7** | | |
| LvO93/Lv12 | **ATACCAGCTTATTCAATT**CCCGCCAGGG**TGAGGTTCGATC**TGATGGCTGGTTCTGGTATTCGCTATGTTTCCCCGTTTGT**ACAATCGTAATCAGTTAG** | 23 |
| *Lv*O *Clust6* | **ATACCAGCTTATTCAATT**CCCGCCCAGGG**TGAGG****T**T**TCGATC**TGATGGCTGGTTCTGGTATTCGCTATGTTTCCCCGTTTTGT**ACAATCGTAATCAGTTAG** | 24 |
| | | |
| **Gruppe Q8** | | |
| DxO4/Dx1 | **ATACCAGCTTATTCAATT**AGTTGTGTAT**TGAGGTTTGATC**TAGGCATAGTCAACAGAGCACGATCGATCTGGCTTGTTCT**ACAATCGTAATCAGTTAG** | 21 |
| | | |
| **Gruppe Q9** | | |
| Cpr50/Cp3 | **ATACCAGCTTATTCAATT**CAGGTGGTGT**T-AGGCTTGATC**TGCCGATCTGTCGAGGCTCGGGTTTAAGCTCCGCTTTTGT**ACAATCGTAATCAGTTAG** | 38 |
| | | |
| **Gruppe Q10** | | |
| DxO62/Dx14 | **ATACCAGCTTATTCAATT**GGCCAGGTAT**TGAGGTTCGATC**TGTGGCTCGTAAGCTGTCTGCCATGCTAGCATTATCGGGT**ACAATCGTAATCAGTTAG** | 26 |
| | | |
| Gruppe **Q11** | | |
| LvO18/Lv3 | **ATACCAGCTTATTCAATT**CACAAATGAC**TGAGGCTTGATC**ATGTGGTTCGATCTGAGTATCTACTGCATTTGAATGGTGT**ACAATCGTAATCAGTTAG** | 40 |
| | | |
| **Gruppe Q12** LvO29/Lv11 | **ATACCAGCTTATTCAATT**AGGGTCGATA**TGAGGCTTGATC**TTATCTTGTTACTGGTCGAACTGTTTGTTACTGCGTGCAT**ACAATCGTAATCAGTTAG** | 42 |
| **Gruppe Q13** | | |
| DxOClust9/Dx16 | **ATACCAGCTTATTCAATT**CCATATTGTA**TGAGGCTCGATC**AAGTATGGACTTTGAGGCGTGGATCTTCTGATTTCTTTGC**ACAATCGTAATCAGTTAG** | 52 |
| | | |
| **Gruppe Q14** | | |
| DxO28/Dx6 | **ATACCAGCTTATTCAATT**GGTCGAAAAG**TGAGGCTTGATC**TAAGTAGTGGCCTAACATTGATTTCGTCGTGAGGCATTCT**ACAATCGTAATCAGTTAG** | 44 |
| DxO33 | **ATACCAGCTTATTCAATT**GGTCGAAAAG**TGAGGCTTGATC**TAAGTAGTGGCCTAACATTGATTTCGTCGTGAGGTATTCT**ACAATCGTAATCAGTTAG** | 45 |
| | | |
| **Gruppe Q15** | | |
| LvO51/Lv10 | **ATACCAGCTTATTCAATT**GTATCCAGAG**TGAGGATCGATC**AGGTTGTGAGGCTCGGATATCATCTGTCATTCTCTGGCAT**ACAATCGTAATCAGTTAG** | 47 |
| | | |
| **Gruppe Q16** | | |
| LvO04/Lv4 | **ATACCAGCTTATTCAATT**ACAGGTGGTA**TGAGACTCGATC**ACTGTTATTTGTCTTTGGCTCGATCCCGATCCGGACTGGT**ACAATCGTAATCAGTTAG** | 49 |
| LvO48 | **ATACCAGCTTATTCAATT**ACAGGTGGTA**TGAGACTCGATC**ACTGTTATTTGTCTTTGGCTCGATCCCGATCCGGACTGGT**ACAATCGTAATCAGTTAG** | 50 |
| | | |
| **Gruppe Q17** | | |
| Cpr52/Cp6 | **ATACCAGCTTATTCAATT**CCCATTGGGA**TGAGGCTTGATC**AGTTTGTGAGGCTACTGGCTTGGTCGTATGCACTGGTTCG**ACAATCGTAATCAGTTAG** | 53 |
| | | |
| **Gruppe Q18** | | |
| Cpr47/Cp7 | **ATACCAGCTTATTCAATT**CCGACCAGGA**TGAGGCTTGATC**GTGTGAGAGTGAGTGGAGGCGTGGATTATGTTGTTATTGT**ACAATCGTAATCAGTTAG** | 55 |
| Cpr Clust24 | **ATACCAGCTTATTCAATT**CCGACCAGGA**TGAGGCTTGA**C**TC**GTGTGAGAGTGAGTGGAGGCGTGGATTATGTTGTTATTGT**ACAATCGTAATCAGTTAG** | 56 |
| | | |
| **Gruppe Q19** | | |
| Cpr55/Cp9 | **ATACCAGCTTATTCAATT**GTATCCAGAG**T**G**GAGGATCGATC**AGGTTGTGAGGCTCGGATATCATCTGTCATTCTCTGGCAT**ACAATCGTAATCAGTTAG** | 58 |
| Cpr Clust.5 | **ATACCAGCTTATTCAATT**GTATCCAGAG**T**G**GAGGATCGATC**AGGTTGTGAGGCTCGGATATCATCTGTCATTCTCTGGCAT**ACAATCGTAATCAGTTAG** | 59 |
| | | |
| **Gruppe Q20** | | |
| LvO12/Lv8 | **ATACCAGCTTATTCAATT**AGGGTCGATC**TGAGGCTCCATC**ACTTCTAATATGTAGCGTCTACGTGGTTGTTCCTAGTCGG**ACAATCGTAATCAGTTAG** | 61 |
| | | |
| **Gruppe Q21** | | |
| DxO69/Dx8 | **ATACCAGCTTATTCAATT**GGAGGCACGA**TGAGGCTCGATC**GCCTGTCAACTGATAAAGCCTGTGCTGCTGTCGTTTTGCT**ACAATCGTAATCAGTTAG** | 63 |
| DxO Clust167 | **ATACCAGCTTATTCAATT**GGAGGTACGA**TGAGGCTCGATC**GCCTGTCAACTGATAAAGCCTGTGCTGCTGTCGTTTTTGGT**ACAATCGTAATCAGTTAG** | 64 |
| | | |
| **Gruppe Q22** | | |
| DxO53/Dx11 | **ATACCAGCTTATTCAATT**AGTCCAATAC**TGAGGCTTGATC**TGGCGAGATAAATAACTGTGCTACTATTCGTTGCGTCCCC**ACAATCGTAATCAGTTAG** | 66 |
| | | |
| **Gruppe Q23** | | |
| DxO84/Dx12 | **ATACCAGCTTATTCAATT**CGCGTTGAAT**TGAGGCTTGATC**TGAATGGCTCGTGTATCTATTTGGTATTGGTTTTGTTTGT**ACAATCGTAATCAGTTAG** | 68 |
| | | |
| **Gruppe Q24** | | |
| DxO59/Dx15 | **ATACCAGCTTATTCAATT**CGCGCCAGGG**TGAGGTTCGATC**TGATGGCTGGTTCTGGTATTCGCTATGTTTCCCCGTTTGT**ACAATCGTAATCAGTTAG** | 70 |
| DxO54 | **ATACCAGCTTATTCAATT**CGCGCCAGGG**TGAGGTTCGATC**TGATGGCTGGTTCTGGTATTCGCTATGTTTCCCCGTTTGT**ACAATCGTAATCAGTTAG** | 71 |
| | | |
| **Gruppe Q25** | | |
| Dx20 | **ATACCAGCTTATTCAATT**GGTCTGGCAT**TGAGGCTCAATC**TGTTGCTCGATTTGGAGTGGCTCGTAGTTAGGGCTCTTTTCT**ACAATCGTAATCAGTTAG** | 73 |
| | | |
| **Gruppe Q26** | | |
| Dx21 | **ATACCAGCTTATTCAATT**GGATGGGAGG**TGAGGCTTGATC**TATGAGGGTCGTGGTGGCNTTTATTATGGCTGTACTCATGG**ACAATCGTAATCAGTTAG** | 75 |
| | | |
| **Gruppe Q27** | | |
| Lv20 | **ATACCAGCTTATTCAATT**CGAGGGGGAGA**TGAGGCTCGATC**ACCTTTCGATTAATTGCTCGATCTAGATGTGTACGTTTGCT**ACAATCGTAATCAGTTAG** | 77 |
| | | |
| **Gruppe Q28** | | |
| Lv21 | **ATACCAGCTTATTCAATT**CACAGCAATTT**TGAGGTTCGATC**GCGGAGGATAGGCTCGGTCAATCCTGGGGTAGTTTTTGTTGT**ACAATCGTAATCAGTTAG** | 78 |
| | | |
| **Gruppe Q29** | | |
| LvO62/Q2vari1 | **ATACCAGCTTATTCAATT**GCAGGGTCGATA**TGAGGCTTGATC**TACTAAATGTCGTGAGGCATTGCTATTGGCGTTG**ACACGTACAATCGTAATCAGTTAG** | 79 |
| | | |
| **Gruppe Q30** | | |
| LvO22/Q2vari2 | **ATACCAGCTTATTCAATT**GGGATGGTAT**TGAGGCTTGATC**TACTAAATGTCGTGGGGCATTGCTATTGGCGTTGATACGT**ACAATCGTAATCAGTTAG** | 81 |

Die Tabelle 2 zeigt die Häufigkeit der Aptamer-Sequenzen, die je Gruppe aufgetreten sind.

Die Sequenzen wurden in dem oben beschriebenen dreigeteilten SELEX-Prozess aus den Pools Cpr, LvO und DxO gewonnen, wie oben anhand der Fig. 3 beschrieben. Daher stammen die unterschiedlichen Bezeichnungen der Klongruppen (Sanger Sequenzierung) bzw. der Cluster (Next Generation Sequencing).

Die so bezeichneten "Klongruppen" in Spalte 2 der Tabelle wurden mit Sanger-Sequenzierung sequenziert, die so bezeichneten "Cluster" in Spalte 2 mit Next Generation Sequencing,

Die Klongruppen in den einzelnen Pools sind durchnummeriert. Jede Klongruppe weist Aptamere einer bestimmten Aptamergruppe auf. Je kleiner die Nummer der Klongruppe ist, desto höher die Anzahl Klone von Aptameren, die zu einer bestimmten Aptamergruppe gehören. Beispielsweise bedeutet in Spalte 2 der Tabelle Q1 - "Cpr Gr. 1" - Anzahl 20,dass aus dem Cpr-Pool Aptamere gewonnen wurden, die der Aptamergruppe Q1 zuzurechnen sind, und die in der Klongruppe 1 vorliegen, und dass in der Klongruppe 1 20 Klone gefunden wurden. Die stellvertretende Sequenz der Gruppe Q1 ist angegeben, aber die Klone beschränken sich nicht nur auf diese Sequenz.

Die Cpr Gruppe 1 ist die Gruppe mit den meisten Klonen im Cpr-Pool, die einer Aptamergruppe zuzurechnen sind. Die Klongruppe Cpr Gr. 2 weist die zweithöchste Anzahl Klone (12) auf, die in diesem Beispiel der Aptamergruppe Q2 zuzurechnen sind. Analog ist die Bezeichnung und Nummerierung von Klongruppen aus anderen Pools. Im Fall von Next Generation Sequencing wird statt von Klongruppen von sog. Clustern gesprochen.

Zu den Häufigkeiten des Auftretens einer Sequenz: Nicht mit Unterstreichung markiert sind die Resultate aus der Sanger-Sequenzierung (insgesamt ca. 250 lesbare Sequenzen), unterstrichen markiert sind diejenigen aus dem "Next Generation Sequencing" (insgesamt > 78.000 reads).

Aptamere mancher Aptamergruppen werden nicht in allen Pools gefunden. Beispielsweise werden Aptamer der Gruppe Q5 nur in dem Cpr-Pool als Klongruppe 5 gefunden. Ein Nicht-Auffinden in anderen Pools gibt keinen Hinweis auf eine nicht vorhandene Spezifität oder Bindungseigenschaft für die Substanzen anderer Pools. Wenn also Aptamere der Gruppe Q5 nur in dem Cpr-Pool, nicht aber in den LvO- und DxO-Pools aufgefunden werden, so kann dennoch eine Bindungseigenschaft an Dextrofloxacin oder Levofloxacin vorhanden sein. Bindungseigenschaften werden gesondert getestet, wie im Beispiel 4 beschrieben.

**Tabelle 2: Sequenzen und Häufigkeit**

| **Gruppen-Name** | ***Klongruppe, Cluster*** | **Anzahl** | **Stellvertretende Sequenz** | **SEQ ID NO** |
|---|---|---|---|---|
| **Q1** | Cpr Gr.1 | 20 | **ATACCAGCTTATTCAATT**CGATGGTAAG**TGAGGTTCGTC**CCTTTAATAAACTCGATTAGGATCTCGTGAGGTGTGCTCT**ACAATCGTAATCAGTTAG** | 1 |
| | LvO Gr.5 | 5 | | |
| | DxO Gr.2 | 7 | | |
| | *Cpr Cl.1* | *1503* | | |
| | *DxO Cl.12* | *334* | | |
| | *LvO Cl.49* | 43 | | |
| **Q2** | LvO Gr.1 | 10 | **ATACCAGCTTATTCAATT**GCAGGGTATC**TGAGGCTTGATC**TACTAAATGTCGTGGGGCATTGCTATTGGCGTTGATACGT**ACAATCGTAATCAGTTAG** | 15 |
| | Cpr Gr.2 | 12 | | |
| | DxO Gr.9 | 3 | | |
| | *LvO Cl.7+10* | *690* | | |
| | *Cpr Cl. 0+6+11* | *2479* | | |
| | *DxO Cl.0* | *2410* | | |
| **Q3** | DxO Gr.4 | 5 | **ATACCAGCTTATTCAATT**CAGGAGGCTC**TGAGGTTCGATC**CTACTGGCTGATAGCTTGGTGTCGGTTGCATTCACCGTGG**ACAATCGTAATCAGTTAG** | 34 |
| | Cpr Gr.4 | 3 | | |
| | LvO Gr.9 | 3 | | |
| | *DxO Cl.1+13* | *2462* | | |
| | *Cpr Cl.2* | *602* | | |
| | *LvO Cl.9* | *415* | | |
| **Q4** | LvO Gr.2 | 9 | **ATACCAGCTTATTCAATT**GGCCACAATT**TGAGGCTGATC**GCATTTTGCTCGATTGAGGCTGTTACTGAGGCGTTCTTGT**ACAATCGTAATCAGTTAG** | 28 |
| | DxO Gr.3 | 5 | | |
| | *LvO Cl.1* | *2129* | | |
| | *DxO Cl.7* | *749* | | |
| | *Cpr Cl.8* | *240* | | |
| **Q5** | Cpr Gr.5 | 2 | **ATACCAGCTTATTCAATT**AGAGGTGAGA**TGAGGCTCGATC**TCCAACCTTCTGTGAAATGCGGCCGGAAGGGTATGTTTCG**ACAATCGTAATCAGTTAG** | 37 |
| **Q6** | LvO Gr.7 | 3 | **ATACCAGCTTATTCAATT**AAGCGGGGGA**TGAGGTTCGATC**TAGTTTAGGCTCGTTACATAGTGTTTCAAGTGTTCGGTCG**ACAATCGTAATCAGTTAG** | 31 |
| | *LvO Cl.45* | *47* | | |
| **Q7** | LvO93 Einzel | 1 | **ATACCAGCTTATTCAATT**CCCGCCAGGG**TGAGGTTCGATC**TGATGGCTGGTTCTGGTATTCGCTATGTTTCCCCGTTTGT**ACAATCGTAATCAGTTAG** | 23 |
| | *LvO Cl.6* | 699 | | |
| **Q8** | DxO Gr.1 | 10 | **ATACCAGCTTATTCAATT**AGTTGTGTAT**TGAGGTTTGATC**TAGGCATAGTCAACAGAGCACGATCGATCTGGCTTGTTCT**ACAATCGTAATCAGTTAG** | 21 |
| | *DxO Cl.12* | *334* | | |
| **Q9** | Cpr Gr.3 | 4 | **ATACCAGCTTATTCAATT**CAGGTGGTGT**TAGGCTTGATC**TGCCGATCTGTCGAGGCTCGGGTTTAAGCTCCGCTTTTGT**ACAATCGTAATCAGTTAG** | 38 |
| | *Cpr Cl.4* | *425* | | |
| | *DxO Cl.14* | *242* | | |
| **Q10** | DxO Gr.14 | 2 | **ATACCAGCTTATTCAATT**GGCCAGGTAT**TGAGGTTCGATC**TGTGGCTCGTAAGCTGTCTGCCATGCTAGCATTATCGGGT**ACAATCGTAATCAGTTAG** | 26 |
| | LvO86 | 1 | | |
| | *DxO Cl.4* | *1569* | | |
| | *Cpr Cl.3* | *588* | | |
| | *LvO Cl.11* | *230* | | |
| **Q11** | LvO Gr.3 | 7 | **ATACCAGCTTATTCAATT**CACAAATGAC**TGAGGCTTGATC**ATGTGGTTCGATCTGAGTATCTACTGCATTTGAATGGTGT**ACAATCGTAATCAGTTAG** | 40 |
| | DxO Gr.5 | 5 | | |
| | *LvO CL.0* | *2367* | | |
| | *DxO Cl.8* | *736* | | |
| **Q12** | LO29 Einzel | 1 | **ATACCAGCTTATTCAATT**AGGGTCGATA**TGAGGCTTGATC**TTATCTTGTTACTGGTCGAACTGTTTGTTACTGCGTGCAT**ACAATCGTAATCAGTTAG** | 42 |
| | *LvO Cl.3* | *1379* | | |
| | *DxO Cl.6* | *817* | | |
| **Q13** | *DxO Cl.*9 | *686* | **ATACCAGCTTATTCAATT**CCATATTGTA**TGAGGCTCGATC**AAGTATGGACTTTGAGGCGTGGATCTTCTGATTTCTTTGC**ACAATCGTAATCAGTTAG** | 52 |
| | *LvO Cl.12* | *224* | | |
| **Q14** | DxO Gr.6 | 4 | **ATACCAGCTTATTCAATT**GGTCGAAAAG**TGAGGCTTGATC**TAAGTAGTGGCCTAACATTGATTTCGTCGTGAGGCATTCT**ACAATCGTAATCAGTTAG** | 44 |
| | Cpr Gr.8 | 2 | | |
| | LvO9 | 1 | | |
| | *DxO Cl.3* | *1613* | | |
| | *Cpr Cl.7* | *251* | | |
| | *LvO Cl.8* | *415* | | |
| **Q15** | LvO51 Einzel | 1 | **ATACCAGCTTATTCAATT**GTATCCAGAG**TGAGGATCGATC**AGGTTGTGAGGCTCGGATATCATCTGTCATTCTCTGGCT**ACAATCGTAATCAGTTAG** | 47 |
| | Cpr55 Einzel | 1 | | |
| | *LvO Cl.2* | *1780* | | |
| | *Cpr Cl.5* | *385* | | |
| | *DxO Cl. 2* | *1664* | | |
| **Q16** | LvO Gr.4 | 6 | **ATACCAGCTTATTCAATT**GCAGGTGGTA**TGAGACTCGATC**ACTGTTATTTGTCTTTGGCTCGATCCCGATCCGGACTGGC**ACAATCGTAATCAGTTAG** | 49 |
| | DxO Gr.10 | 3 | | |
| | *LvO CL.5* | *853* | | |
| | *DxO Cl.5* | *893* | | |
| | *Cpr Cl.9* | *231* | | |
| **Q17** | Cpr Gr.6 | 2 | **ATACCAGCTTATTCAATT**CCCATTGGGA**TGAGGCTTGATC**AGTTTGTGAGGCTACTGGCTTGGTCGTATGCACTGGTTCG**ACAATCGTAATCAGTTAG** | 53 |
| | *Cpr Cl.44* | 40 | | |
| **Q18** | Cpr Gr.7 | 2 | **ATACCAGCTTATTCAATT**CCGACCAGGA**TGAGGCTTGATC**GTGTGAGAGTGAGTGGAGGCGTGGATTATGTTGTTATTGT**ACAATCGTAATCAGTTAG** | 55 |
| | *Cpr Cl.24* | 44 | | |
| **Q19** | Cpr55 | 2 | **ATACCAGCTTATTCAATT**GTATCCAGAG**TGAGGATCGATT**AGGTTGTGAGGCTCGGATATCATCTGTCATTCTCTGGCAT**ACAATCGTAATCAGTTAG** | |
| | *Cpr Cl.5* | 40 | | |
| **Q20** | LvO Gr.8 | 3 | **ATACCAGCTTATTCAATT**AGGGTCGATC**TGAGGCTCCATC**ACTTCTAATATGTAGCGTCTACGTGGTTGTTCCTAGTCGG**ACAATCGTAATCAGTTAG** | 61 |
| | *LvO Cl.52* | *38* | | |
| **Q21** | DxO Gr.8 | 3 | **ATACCAGCTTATTCAATT**GGAGGCACGA**TGAGGCTCGATC**GCCTGTCAACTGATAAAGCCTGTGCTGCTGTCGTTTTGGT**ACAATCGTAATCAGTTAG** | 63 |
| | LVO31 | 1 | | |
| | *DXO Cl.167* | *13* | | |
| | *LvO Cl.35* | 59 | | |
| **Q22** | DxO Gr.11 | 2 | **ATACCAGCTTATTCAATT**AGTCCAATAC**TGAGGCTTGATC**TGGCGAGATAAATAACTGTGCTACTATTCGTTGCGTCCCC**ACAATCGTAATCAGTTAG** | 66 |
| | *DxO Cl.108* | *23* | | |
| | *LvO Cl.53* | *38* | | |
| **Q23** | DxO Gr.12 | 2 | **ATACCAGCTTATTCAATT**CGCGT**TGAATTGAGGCTTGATC**TGAATGGCTCGTGTATCTATTTGGTATTGGTTTTGTTTGT**ACAATCGTAATCAGTTAG** | 68 |
| | *DxO Cl.55* | *41* | | |
| | *LvO Cl.107* | *15* | | |
| | *Cpr Cl.177* | 10 | | |
| **Q24** | DxO Gr.15 | 2 | **ATACCAGCTTATTCAATT**CCCGCCAGGG**TGAGGTTCGATC**TGATGGCTGGTTCTGGTATTCGCTATGTTTCCCCGTTTGT**ACAATCGTAATCAGTTAG** | 70 |
| | *DxO Cl.17* | *203* | | |
| | *LvO Cl.119* | *14* | | |
| | *Cpr Cl.68* | *26* | | |
| **Q25** | *DxO Cl.11+50* | *394* | **ATACCAGCTTATTCAATT**GGTCTGGCAT**TGAGGCTCAATC**TGTTGCTCGATTTGGAGTGGCTCGTAGTTAGGGCTCTTTTCT**ACAATCGTAATCAGTTAG** | 73 |
| | *Cpr Cl.69* | *26* | | |
| | *LvO Cl.131* | *12* | | |
| **Q26** | | *228* | **ATACCAGCTTATTCAATT**GGATGGGAGG**TGAGGCTTGATC**TATGAGGGTCGTGGTGGCNTTTATTATGGCTGTACTCATGG**ACAATCGTAATCAGTTAG** | 75 |
| | *DxO Cl.15* | *35* | | |
| **Q27** | *LvO Cl.13* | *218* | **ATACCAGCTTATTCAATT**CGAGGGGGAGA**TGAGGCTCGATC**ACCTTTCGATTAATTGCTCGATCTAGATGTGTACGTTTGCT**ACAATCGTAATCAGTTAG** | 77 |
| | *DxO Cl.37+44* | *104* | | |
| **Q28** | *LvO Cl.14* | *200* | **ATACCAGCTTATTCAATT**CACAGCAATTT**TGAGGTTCGATC**GCGGAGGATAGGCTCGGTCAATCCTGGGGTAGTTTTTGTTGT**ACAATCGTAATCAGTTA G** | 78 |
| | *Cpr Cl.10* | *166* | | |
| | *DxO Cl.19* | *159* | | |
| **Q29** | *LvO62* | *1* | **ATACCAGCTTATTCAATT**GCAGGGTCGATA**TGAGGCTTGATC**TACTAAATGTCGTGAGGCATTGCTATTGGCGTTGACACGT**ACAATCGTAATCAGTTAG** | 79 |
| **Q30** | *LvO22* | *1* | **ATACCAGCTTATTCAATT**GGGATGGTAT**TGAGGCTTGATC**TACTAAATGTCGTGGGGCATTGCTATTGGCGTTGATACGT**ACAATCGTAATCAGTTAG** | 81 |

In der Tabelle 3 sind zusammengefasste Sequenzen der einzelnen Aptamer-Gruppen (Q1, Q2 etc.) dargestellt, auch bezeichnet als "Gruppensequenz".

Fett gedruckte Nukleotide in Tabelle 3 waren vordefinierte Teilsequenzen (Primersequenz, zum Antisense-Primer komplementäre Sequenz, zur Fängersequenz komplementäre Sequenz), variable Nukleotide sind unterstrichen markiert und entsprechend Tabelle 4 bezeichnet. Die Tabelle 4 zeigt eine Übersicht der Nukleotidsymbole, die für eines oder mehrere der Nukleotide A, G, C oder T stehen.

Variable Nukleotide in der Primersequenz, der zum Antisense-Primer komplementären Sequenz oder der zur Fängersequenz komplementären Sequenz zeigen ein geändertes Nukleotid im Vergleich zu der jeweiligen ursprünglichen Sequenz.

In der Sequenz zu Q1, SEQ ID NO: 14, steht als Nukleotid an Position 19, hinter der Primersequenz, ein N. Aus der Analyse ergibt sich zunächst ein V (A, C oder G), aber es liegen Hinweise vor, dass auch mit T die Aptamerfunktion erfüllt wird.

In der Sequenz zu Q1, SEQ ID NO: 14, bedeuten X₁ und X₂ entweder A oder L, wobei L für kein Nukleotid, also eine Deletion steht.

In der Sequenz zu Q4, SEQ ID NO: 30, bedeutet X₃ entweder C oder L, wobei L für kein Nukleotid, also eine Deletion steht.

In der Sequenz zu Q9, SEQ ID NO: 39, bedeutet X₄ entweder G oder L, wobei L für kein Nukleotid, also eine Deletion steht.

Bei den Gruppen Q5 (SEQ ID NO: 37), Q13 (SEQ ID NO: 52), Q27 (SEQ ID NO: 77) und Q28 (SEQ ID NO: 78) entspricht die Gruppensequenz der Sequenz des einzigen Vertreters der Gruppe. Aus diesem Grund ist die SEQ ID NO unterstrichen gekennzeichnet.

**Tabelle 3: Aptamer-Gruppensequenzen**

| **Gruppen -Name** | **Gruppensequenz** | **Nukleotid Anzahl** | **SEQ ID NO** |
|---|---|---|---|
| **Q1** | **ATACCAGCTTATTCAATT**NGATGGTAX₁K**TGAGGYTCGX₂**TCCCTTTAATAAACTCGATTARGATCTCGTGAGGTGTGYTYY**ACAATCGTAATCAGTTAG** | 97 nt | 14 |
| **Q2** | **ATACCAGCTTATTCAATT**GCAGGGTATC**TGAGGCTYGATC**TACTAAATGTCGTGRGGCATTRCTATTGRCGTTGAYACGT**ACAATCGTAATCAGTTAG** | 98 nt | 20 |
| **Q3** | **ATACCAGCTTATTCAATT**CAGGAGGCTC**TGAGGYTCGATC**CTACTGGCTGATAGCTTGGTGTCGGTTGCATTCACCGTGS**ACAATCGTAATCAGTTAG** | 98 nt | 36 |
| **Q4** | **ATACCAGCTTATTCAATT**GKCCACAATT**TGAGGCTX₃GATC**GCATTTTGCTCGATTGAGGCTGTTACTGAGGCGTTCTTGT**ACAATCGTAATCAGTTAG** | 97 nt | 30 |
| **Q5** | **ATACCAGCTTATTCAATT**AGAGGTGAGA**TGAGGCTCGATC**TCCAACCTTCTGTGAAATGCGGCCGGAAGGGTATGTTTCG**ACAATCGTAATCAGTTAG** | 98 nt | 37 |
| **Q6** | **ATACCAGCTTATTCAATT**AAGCGGGGGA**TGAGGYTCGATC**TAGTTTAGGCTCGTTACATAGTGTTTCAAGTGTTCGGTCG**ACAATCGTAATCAGTTAG** | 98 nt | 33 |
| **Q7** | **ATACCAGCTTATTCAATT**CCCGCCAGGG**TGAGGYTCGATC**TGATGGCTGGTTCTGGTATTCGCTATGTTTCCCCGTTTGT**ACAATCGTAATCAGTTAG** | 98 nt | 25 |
| **Q8** | **ATACCAGCTTATTCAATT**AGTTGTGTAT**TGAGGYTYGATC**TAGGCATAGTCAACAGAGCACGATCGATCTGGCTTGTTCT**ACAATCGTAATCAGTTAG** | 98 nt | 22 |
| **Q9** | **ATACCAGCTTATTCAATT**CAGGTGGTGT**TX₄AGGCTYGATC**TGCCGATCTGTCGAGGCTCGGGTTTAAGCTCCGCTTTTGT**ACAATCGTAATCAGTTAG** | 97 nt | 39 |
| **Q10** | **ATACCAGCTTATTCAATT**GGCCAGGTAT**TGAGGYTCGATC**TGTGGCTCGTAAGCTGTCTGCCATGCTAGCATTATCGGGT**ACAATCGTAATCAGTTAG** | 98 nt | 27 |
| **Q11** | **ATACCAGCTTATTCAATT**CACAAATGAC**TGAGGCTYGATC**ATGTGGTTCGATCTGAGTATCTACTGCATTTGAATGGTGT**ACAATCGTAATCAGTTAG** | 98 nt | 41 |
| **Q12** | **ATACCAGCTTATTCAATT**AGGGTCGATA**TGAGGCTYGATC**TTATCTTGTTACTGGTCGAACTGTTTGTTACTGCGTGCAT**ACAATCGTAATCAGTTAG** | 98 nt | 43 |
| **Q13** | **ATACCAGCTTATTCAATT**CCATATTGTA**TGAGGCTCGATC**AAGTATGGACTTTGAGGCGTGGATCTTCTGATTTCTTTGC**ACAATCGTAATCAGTTAG** | 98 nt | 52 |
| **Q14** | **ATACCAGCTTATTCAATT**GGTCGAAAAG**TGAGGCTYGATC**TAAGTAGTGGCCTAACATTGATTTCGTCGTGAGGYATTCT**ACAATCGTAATCAGTTAG** | 98 nt | 46 |
| **Q15** | **ATACCAGCTTATTCAATT**GTATCCAGAG**TGAGGMTCGATC**AGGTTGTGAGGCTCGGATATCATCTGTCATTCTCTGGCAT**ACAATCGTAATCAGTTAG** | 98 nt | 48 |
| **Q16** | **ATACCAGCTTATTCAATT**RCAGGTGGTA**TGAGRCTCGATC**ACTGTTATTTGTCTTTGGCTCGATCCCGATCCGGACTGGY**ACAATCGTAATCAGTTAG** | 98 nt | 51 |
| **Q17** | **ATACCAGCTTATTCAATT**CCCATTGGGA**TGAGGCTYGATC**AGTTTGTGAGGCTACTGGCTTGGTCGTATGCACTGGTTCG**ACAATCGTAATCAGTTAG** | 98 nt | 54 |
| **Q18** | **ATACCAGCTTATTCAATT**CCGACCAGGA**TGAGGCTYGATC**GTGTGAGAGTGAGTGGAGGCGTGGATTATGTTGTTATTGT**ACAATCGTAATCAGTTAG** | 98 nt | 57 |
| **Q19** | **ATACCAGCTTATTCAATT**GTATCCAGAG**TGAGGMTCGATY**AGTTTGTGAGGCTCGGATATCATCTGTCATTCTCTGGCAT**ACAATCGTAATCAGTTAG** | 98 nt | 60 |
| **Q20** | **ATACCAGCTTATTCAATT**AGGGTCGATC**TGAGGCTCSATC**ACTTCTAATATGTAGCGTCTACGTGGTTGTTCCTAGTCGG**ACAATCGTAATCAGTTAG** | 98 nt | 62 |
| **Q21** | **ATACCAGCTTATTCAATT**ACAGGTGGTA**TGAGGCTCGATC**ACTGTTATTTGTCTTTGGCTCGATCCCGATCCGGACTGGC**ACAATCGTAATCAGTTAG** | 98 nt | 65 |
| **Q22** | **ATACCAGCTTATTCAATT**AGTCCAATAC**TGAGGCTYGATC**TGGCGAGATAAATAACTGTGCTACTATTCGTTGCGTCCCC**ACAATCGTAATCAGTTAG** | 98 nt | 67 |
| **Q23** | **ATACCAGCTTATTCAATT**CGCGTTGAAT**TGAGGCTYGATC**TGAATGGCTCGTGTATCTATTTGGTATTGGTTTTGTTTGT**ACAATCGTAATCAGTTAG** | 98 nt | 69 |
| **Q24** | **ATACCAGCTTATTCAATT**CSCGCCAGGG**TGAGGYTCGATC**TGATGGCTGGTTCTGGTATTCGCTATGTTTCCCCGTTTGT**ACAATCGTAATCAGTTAG** | 98 nt | 72 |
| **Q25** | **ATACCAGCTTATTCAATT**GGTCTGGCAT**TGAGGCTCRATC**TGTTGCTCGATTTGGAGTGGCTCGTAGTTAGGGCTCTTTTCT**ACAATCGTAATCAGTTAG** | 100 nt | 74 |
| **Q26** | **ATACCAGCTTATTCAATT**GGATGGGAGG**TGAGGCTYGATC**TATGAGGGTCGTGGTGGCNTTTATTATGGCTGTACTCATGG**ACAATCGTAATCAGTTAG** | 99 nt | 76 |
| **Q27** | **ATACCAGCTTATTCAATT**CGAGGGGGAGA**TGAGGCTCGATC**ACCTTTCGATTAATTGCTCGATCTAGATGTGTACGTTTGCT**ACAATCGTAATCAGTTAG** | 100 nt | 77 |
| **Q28** | **ATACCAGCTTATTCAATT**CACAGCAATTT**TGAGGTTCGATC**GCGGAGGATAGGCTCGGTCAATCCTGGGGTAGTTTTTGTTG**TACAATCGTAATCAGTTAG** | 101 nt | 78 |
| **Q29** | **ATACCAGCTTATTCAATT**GCAGGGTCGATA**TGAGGCTYGATC**TACTAAATGTCGTGAGGCATTGCTATTGGCGTTGACACGT**ACAATCGTAATCAGTTAG** | 100 nt | 80 |
| **Q30** | **ATACCAGCTTATTCAATT**GGGATGGTAT**TGAGGCTYGATC**TACTAAATGTCGTGGGGCATTGCTATTGGCGTTGATACGT**ACAATCGTAATCAGTTAG** | 98 nt | 82 |

**Tabelle 4**

| **Symbol** | **Bedeutung** |
|---|---|
| R | G oder A |
| Y | T/U oder C |
| M | A oder C |
| K | G oder T/U |
| S | G oder C |
| W | A oder T/U |
| B | G oder C oder T/U |
| D | A oder G oder T/U |
| H | A oder C oder T/U |
| V | A oder G oder C |
| N | A, C, G oder T/U |

### BEISPIEL 4: Bestimmung der Spezifität einzelner Aptamere.

Die erhaltenen Sequenzen der Aptamer-Klone konnten anhand ihrer Ähnlichkeit in 30 Gruppen eingeteilt werden. Aus 10 Gruppen wurde je ein Stellvertreter ausgewählt und bei Microsynth (Schweiz) chemisch hergestellt und PAGE gereinigt. Diese Stellvertreter wurden auf ihre individuelle Bindungsfähigkeit an mehrere Substanzen (Fluorchinole, Antibiotika anderer Gruppen, andere Pharmaka) getestet. Die Substanzen lagen dabei gelöst entweder in Bindungspuffer oder in Realproben (z.B. Leitungswasser oder Kläranlagenabläufen) vor. Als Negativkontrollen wurde parallel Bindungspuffer (oder die entsprechende Realprobe) ohne enthaltene Targetsubstanz verwendet.

Für jeden Bindungstest wurde ein frisches Aliquot von 3 x 10⁸ der mit der Fängersequenz modifizierten Magnetic Beads (1 x 10⁷ Beads für jede Bindungsreaktion) zunächst 3 x in Bindungspufferlösung (BB(C): 20 mmol L⁻¹ Tris-HCl pH 7.6, 100 mmol L⁻¹ NaCl, 2 mmol L⁻¹ MgCl₂, 1 mmol L⁻¹ CaCl₂) gewaschen. Die Aptamer-ssDNA wurde vorbereitet, indem ca. 300 pmol in 500 µL BB(C) für 8 min bei 90 °C inkubiert, danach 10 min auf Eis und 5-6 min bei Raumtemperatur äquilibriert wurden. Das vorbereitete Aptamer wurde mit dem gewaschenen Aliquot Fängersequenz-modifizierter Magnetic Beads über Nacht bei 21 °C unter leichtem Schütteln inkubiert. Nach der Bindungsreaktion wurden die ungebundenen Aptamere entfernt und wie alle folgenden Fraktionen gesammelt.

Der Bindungskomplex wurde 9 x in jeweils 500 µL BB(C) gewaschen. Die Magnetic Beads mit den gebundenen Aptameren wurden nun einem Temperaturschritt wie während des Selektionsprozesses unterworfen: Inkubation in 500 µL BB(C) bei 28 °C für 15 min bei leichtem Schütteln. Aptamere, die sich während dieses Schrittes von den Magnetic Beads gelöst hatten, wurden entfernt. Anschließend wurden die Magnetic Beads 7 x mit je 500 µL BB(C) gewaschen. Im Hintergrund-Elutionsschritt wurden die Magnetic Beads in 300 µL BB(C) bei 21 °C für 45 min leicht geschüttelt. Anschließend wurden gelöste Aptamere entfernt. Die Beads wurden 6 x in je 500 µL BB(C) gewaschen, dann in 600 µL BB(C) aufgenommen und auf 6 vorgespülte Reaktionsgefäße gleichmäßig verteilt. Der Überstand wurde abgenommen und die einzelnen Aliquots in je 300 µL Targetsubstanz-Lösung, deren Mischung sowie Puffer ohne Targetsubstanz bei 21 °C 45 min inkubiert. Anschließend wurde die Lösung abgenommen. In der so entstandenen Fraktion der Target-Elution sollten sich diejenigen Aptamere befinden, welche sich durch Trennung der Fängersequenz-Aptamer-Bindung bei Anwesenheit der Targetsubstanz(en) von den Magnetic Beads gelöst hatten. Die danach noch an den Magnetic Beads gebundenen Aptamere wurden durch Hitze eluiert (2 x 300 µL BB(C), 95 °C, 10 min).

Die DNA-Mengen in allen so gewonnenen Fraktionen (inklusive der Waschschritte) wurden mittels Fluorometrie (Wallac Victor²V Multilabel Counter; PerkinElmer life sciences, Deutschland) bei folgenden Bedingungen gemessen: Excitation 485 nm/Emission 535 nm; time 1 s; CW-lamp energy 22500. Das Messvolumen war 100 µL/well in 96 well black Mikrotiterplatten (NUNC, Deutschland). Die Berechnung der ssDNA-Konzentration jeder Fraktion erfolgte unter Verwendung einer Kalibrierkurve, welche aus der jeweiligen Aptamer-DNA hergestellt wurde, die den gleichen thermischen Bedingungen bei Äquilibrierung und Über-Nacht-Inkubation ausgesetzt war wie die bei den Spezifitätstests eingesetzte Aptamer-DNA.

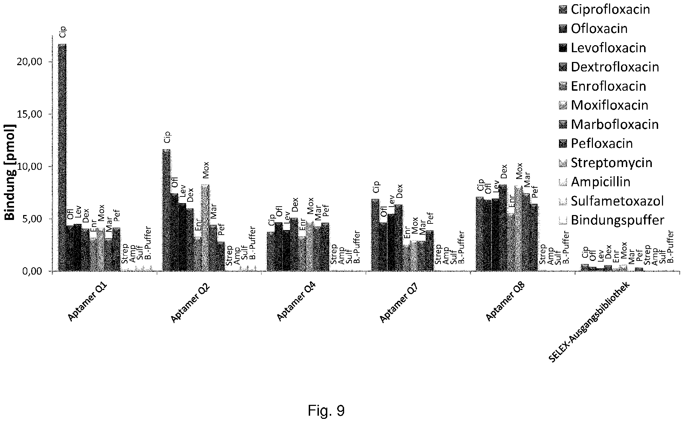

Fig. 9 zeigt die Bindung erfindungsgemäßer Fluorchinolon-Aptamere an verschiedene Antibiotika. Es wurde ein Bindungstest mit Vertretern verschiedener Antibiotikaklassen durchgeführt, nämlich Fluorchinolone, Aminoglykoside, β-Lactame, Sulfonamide. Die Ergebnisse zweigen eine sehr gute Aptamerbindung an Fluorchinolone, keine Bindung an andere Antibiotika und keine unspezifische Targetbindung durch ssDNA (SELEX-Ausgangsbibliothek).

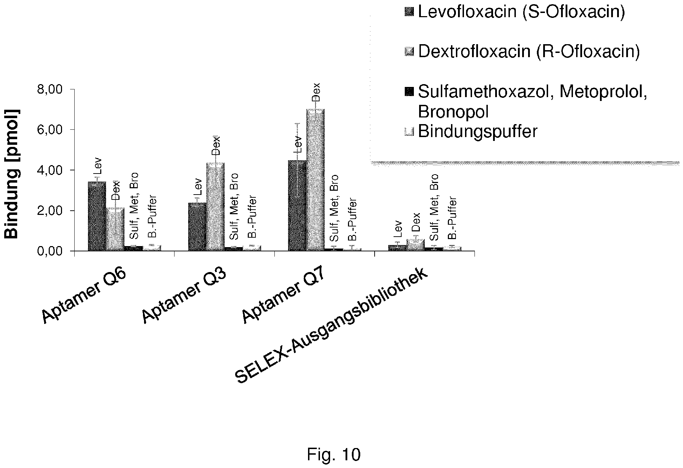

Fig. 10 zeigt die Bindung ausgewählter Aptamere an Ofloxacin-Enantiomere und Negativkontrollen. Die Ergebnisse zeigen ein unterschiedliches Bindungsvermögen einzelner Aptamere zu den Ofloxacin-Enantiomeren Levofloxacin und Dextrofloxacin. Es zeigt sich keine absolute Spezifität für eines der Enantiomere. Ferner ist keine Bindung zu Arzneimittel-Vertretern anderer Wirkstoffklassen sichtbar. In diesem Beispiel wurden Sulfamethoxazol, Metoprolol und Bronopol als Vertreter anderer Wirkstoffklassen getestet.

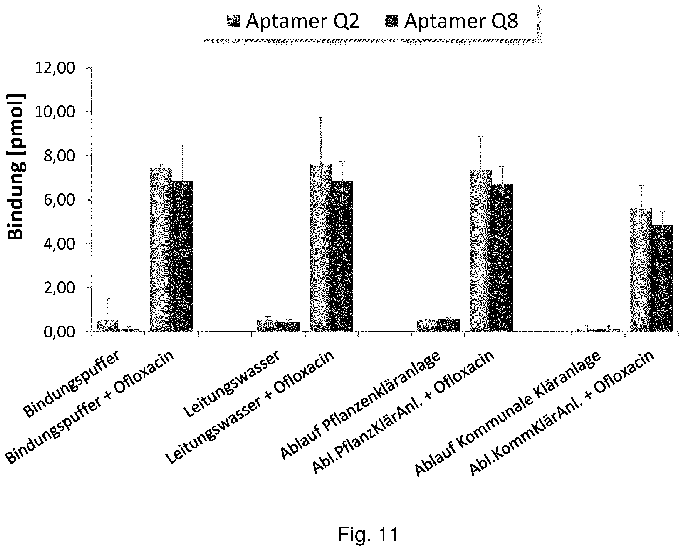

Fig. 11 zeigt die Aptamer-Target-Bindung in Realproben. Als Vergleich ist links in Fig. 11 die Aptamer-Target-Bindung in Bindungspuffer als Nicht-Realprobe dargestellt. Die Realproben wurden jeweils mit 1 mM Ofloxacin versetzt. Die Realproben waren:
- Leitungswasser der Stadt Leipzig, DE
- Der Ablauf einer Pflanzenkläranlage (horizontal, belüftet, bepflanzt mit Phragmites australis)
- Der Ablauf der kommunalen Kläranlage der Kommune Langenreichenbach, DE (16 000 Einwohner)

Die Ergebnisse zeigen keine Funktionseinschränkung, also die volle Funktionsfähigkeit, der Aptamer-Target-Bindung in Realproben im Vergleich zu reinem Bindungspuffer.

### BEISPIEL 5: Aufbau eines Biosensors und Durchführung eines Nachweises auf Fluorchinolon

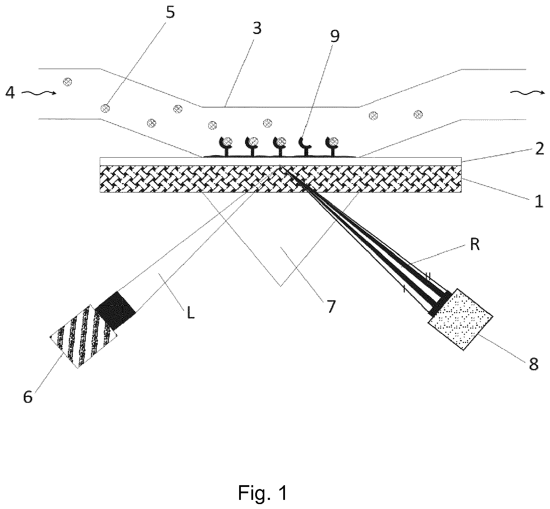

### Bezugszeichenliste zu Fig. 1:

- 1: Sensorchip (Glaschip)
- 2: Goldfilm
- 3: Flusszelle
- 4: Probelösung
- 5: Target
- 6: Lichtquelle
- 7: Prisma
- 8: Detektor
- 9: Aptamer
- L: monochromatisches polarisiertes Licht
- R: reflektiertes Licht

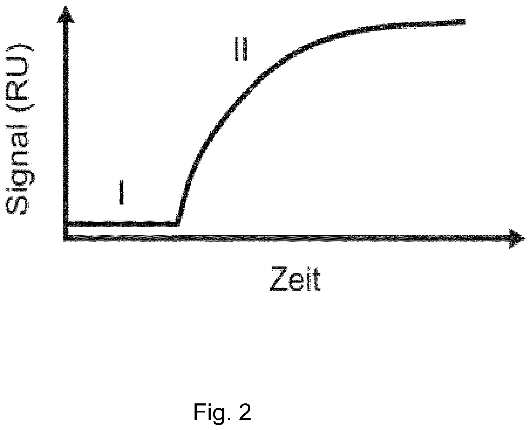

Das Biacore®-System ist ein kommerziell erhältliches Biosensor-System, welches den markierungsfreien Nachweis von biomolekularen Wechselwirkungen in Echtzeit ermöglicht und dafür das physikalische Prinzip der Oberflächenplasmonenresonanz-Spektroskopie (Surface Plasmon Resonance, SPR) nutzt, wie in der Fig. 1 dargestellt. Der Biosensorchip des Biacore-Systems besteht aus einem Glas-Chip 1, der mit einem dünnen Goldfilm 2 beschichtet ist und die Sensoroberfläche darstellt. Eine spezielle Funktionalisierung dieser Sensoroberfläche, beispielsweise mit Carboxymethyldextran (CM), erlaubt die kovalente Immobilisierung der biologischen Rezeptormoleküle. Im Falle der Aptamere 9 wird bevorzugt das Biotin-Streptavidin-Kopplungssystem genutzt, um die biotinylierten Aptamere auf die Streptavidin-beschichtete Sensoroberfläche des Biosensorchips zu immobilisieren. Die anschließende Interaktion zwischen Target und Aptamer findet in einer Flusszelle 3 statt, wobei durch ein integriertes, kontinuierliches Flusssystem die Lösung 4 mit Target 5 über die Sensoroberfläche geleitet wird. Die optische Detektionseinheit des Biacore-Systems besteht aus einer Lichtquelle 6 (Leuchtdioden), einem Prisma 7 und einem Detektor 8 (Diodenarray-Detektor). Monochromatisches, polarisiertes Licht L wird unter den Bedingungen der Totalreflexion von einem Medium mit hohem Brechungsindex (Sensorchip mit Goldfilm) in ein Medium mit niedrigem Brechungsindex (Sensorschicht, Pufferlösung) eingestrahlt. Dabei tritt bei einem bestimmten Einfallswinkel eine Abschwächung des reflektierten Lichtes auf. Dieser Winkel, der Resonanzwinkel, reagiert sehr sensitiv auf Veränderungen des Brechungsindexes der Sensorschicht. Die Anbindung des Targets 5 an das immobilisierte Aptamer 9 auf der Sensoroberfläche resultiert in einem Massezuwachs in der Sensorschicht, welcher zu einer Änderung des Brechungsindexes dieser Schicht und damit zu einer Verschiebung des Resonanzwinkels führt. Diese Änderungen werden als messbares Signal, ausgedrückt in Resonanz-Einheiten (RU), ausgegeben und sind proportional zu der Menge an gebundenem Target 5 auf der Sensoroberfläche. Während der Bindungsanalyse werden die Änderungen des Resonanzwinkels kontinuierlich aufgezeichnet und aufgetragen gegen die Zeit als Sensorgramm dargestellt, das in der Fig. 2 schematisch dargestellt ist. Im Abschnitt II der Signalkurve ist eine Verschiebung des Resonanzwinkels und die Anbindung von Aptamer erkennbar. Die Verschiebung des Resonanzwinkels ist auch in der Fig. 1 gezeigt, anhand eines mit "I" und eines mit "II" bezeichneten reflektierten Lichtstrahles.

Der Aptamer-Target-Komplex kann unter geeigneten Pufferbedingungen wieder gelöst werden, so dass die Sensoroberfläche regeneriert wird und für eine erneute Bindung mit Targetmolekülen zu Verfügung steht.

An der Oberfläche des oben beschriebenen Biosensors wird erfindungsmäßes Aptamer immobilisiert. Eine Fluorchinolon-Lösung (z.B. Ciprofloxacin, Ofloxacin, Levofloxacin) wird über die Sensoroberfläche geleitet, wobei dasFluorchinolon an das Aptamer bindet. Die Anbindung von Fluorchinolon wird als Verschiebung des Resonanzwinkels detektiert.

## Patentansprüche

1. DNA-Aptamer, das an eine oder mehrere Verbindungen aus der Gruppe der Fluorchinolone bindet und das ausgewählt ist aus der Gruppe bestehend aus
a) einem Aptamer umfassend, oder bestehend aus, eine/einer Nukleinsäuresequenz, die ausgewählt ist aus der Gruppe bestehend aus SEQ ID NO: 14, 20, 36, 30, 37, 33, 25, 22, 39, 27, 41, 43 und 52,
b) einem Aptamer, dessen Nukleinsäuresequenz eine Identität von mindestens 98% mit der Nukleinsäuresequenz eines Aptamers aus a) aufweist
c) einem Aptamer bei dem gegenüber einem Aptamer aus a) bei der Sequenz mit der SEQ ID NO: 14, 20, 36, 30, 37, 33, 25, 22, 39, 27, 41, 43 oder 52 bis zu 2 Nukleotide substituiert, deletiert und/oder insertiert sind,
d) einem Aptamer bei dem gegenüber einem Aptamer aus a) bei der Sequenz mit einer der SEQ ID NO: 14, 20, 36, 30, 37, 33, 25, 22, 39, 27, 41, 43 und 52 bis zu 20 Nukleotide am 3'-Ende und/oder am 5'-Ende angefügt sind,
e) einem Fragment eines Aptamers nach a), b) oder c), das mindestens 30 Nukleotide aufweist, und
f) einem Derivat eines Aptamers nach a), b), c), d) oder e), wobei ein Aptamer nach a), b), c), d) oder e) an der Nukleobase, an der Pentose oder am Phosphat-Rückgrat chemisch modifiziert ist und eine chemische Struktur aufweist, die in natürlicher DNA oder RNA nicht vorkommt.

2. Aptamer nach Anspruch 1, wobei das Aptamer nach Punkt b) - f) eines oder mehrere der Motive ATACCAGCTTATTCAATT (SEQ ID NO: 83), ACAATCGTAATCAGTTAG (SEQ ID NO: 84) und TGAGGCTCGATC (SEQ ID NO: 85) in unveränderter Form aufweist.

3. Aptamersonde zur Detektion einer oder mehrerer Verbindungen aus der Gruppe der Fluorchinolone, umfassend ein oder mehrere verschiedene Aptamere wie in einem der Ansprüche 1-2 beschrieben und ein Markierungsmittel.

4. Biosensor, aufweisend ein oder mehrere verschiedene Aptamere wie in einem der Ansprüche 1-2 beschrieben.

5. Feste Phase, insbesondere zur Verwendung beim Nachweis, Anreicherung, Abtrennung und/oder Isolierung einer oder mehrerer Verbindungen aus der Gruppe der Fluorchinolone, an die ein oder mehrerere verschiedene Aptamere nach einem der Ansprüche 1-2 immobilisiert ist/sind.

6. Teststreifen, insbesondere zur Verwendung in Lateralfluss-Assays, der ein oder mehrerere verschiedene Aptamere nach einem der Ansprüche 1-2 enthält.

7. Lateralfluss-Assay-Vorrichtung, die einen Teststreifen nach Anspruch 6 aufweist.

8. Kit, umfassend ein oder mehrere verschiedene Aptamere wie in einem der Ansprüche 1-2 beschrieben.

9. Messgerät zum Nachweis von Verbindungen aus der Gruppe der Fluorchinolone, aufweisend ein oder mehrere verschiedene Aptamere nach Anspruch 1-2, eine Aptamersonde nach Anspruch 3, einen Biosensor nach Anspruch 4 oder einen Teststreifen nach Anspruch 6.

10. Verwendung des Aptamers wie in einem der Ansprüche 1-2 beschrieben zum Nachweis, Anreicherung, Abtrennung und/oder Isolierung einer oder mehrerer Verbindungen aus der Gruppe der Fluorchinolone.

11. Verfahren zum Nachweis einer oder mehrerer Verbindungen aus der Gruppe der Fluorchinolone, bei dem
a) ein oder mehrere verschiedene Aptamere wie in einem der Ansprüche 1-2 beschrieben mit einer Probe, die eine oder mehrere Verbindungen aus der Gruppe der Fluorchinolone enthält, in Kontakt gebracht wird/werden und
b) eine Bindung zwischen dem/den Aptamer(en) und der/den Verbindung(en) aus der Gruppe der Fluorchinolone nachgewiesen wird.

12. Verfahren nach Anspruch 11, das ein in Form eines kompetitiven Assays oder eines Sandwich-Assays durchgeführt wird.

13. Verfahren zur Anreicherung, Abtrennung und/oder Isolierung einer oder mehrerer Verbindungen aus der Gruppe der Fluorchinolone, bei dem
a) ein oder mehrere verschiedene Aptamere wie in einem der Ansprüche 1-2 beschrieben mit einer Probe, die eine oder mehrere Verbindungen aus der Gruppe der Fluorchinolone enthält, in Kontakt gebracht wird/werden, wobei ein Komplex oder Komplexe aus dem/den Aptamer(en) und der/den Verbindung(en) aus der Gruppe der Fluorchinolone gebildet wird,
b) der/die Komplex(e) und die übrige Probe voneinander getrennt werden, und
c) optional die Verbindung(en) aus der Gruppe der Fluorchinolone aus dem Komplex isoliert wird/werden.

14. Verfahren nach einem der Ansprüche 11-13, bei dem das/die Aptamer(e) an einer festen Phase immobilisiert ist/sind.

15. Verwendung einer Aptamersonde nach Anspruch 3, eines Biosensors nach Anspruch 4, einer festen Phase nach Anspruch 5, eines Teststreifens nach Anspruch 6, einer Lateralfluss-Assay-Vorrichtung nach Anspruch 7, eines Kits nach Anspruch 8, oder eines Messgeräts nach Anspruch 9 zum Nachweis einer oder mehrerer Verbindungen aus der Gruppe der Fluorchinolone oder zur Anreicherung, Abtrennung und/oder Isolierung einer oder mehrerer Verbindungen aus der Gruppe der Fluorchinolone.

## Claims

1. A DNA aptamer that binds to one or more compounds from the group of the fluoroquinolones and is selected from the group consisting of
a) an aptamer comprising, or consisting of, a nucleic acid sequence selected from the group consisting of SEQ ID NO: 14, 20, 36, 30, 37, 33, 25, 22, 39, 27, 41, 43 and 52;
b) an aptamer having a nucleic acid sequence which is at least 98% identical to the nucleic acid sequence of an aptamer from a);
c) an aptamer in which up to 2 nucleotides are substituted, deleted and/or inserted in the sequence having SEQ ID NO: 14, 20, 36, 30, 37, 33, 25, 22, 39, 27, 41, 43 or 52 compared to an aptamer from a)
d) an aptamer in which up to 20 nucleotides are added at the 3' end and/or at the 5' end in the sequence having one of the SEQ ID NOs: 14, 20, 36, 30, 37, 33, 25, 22, 39, 27, 41, 43 and 52 compared to an aptamer from a);
e) a fragment of an aptamer according to a), b) or c), comprising at least 30 nucleotides; and
f) a derivative of an aptamer according to a), b), c), d) or e), wherein an aptamer according to a), b), c), d) or e) is chemically modified on the nucleobase, on the pentose or on the phosphate backbone and has a chemical structure that does not occur in natural DNA or RNA.

2. The aptamer according to claim 1, wherein the aptamer according to items b) to f) has one or more of the motifs ATACCAGCTTATTCAATT (SEQ ID NO: 83), ACAATCGTAATCAGTTAG (SEQ ID NO: 84) and TGAGGCTCGATC (SEQ ID NO: 85) in unmodified form.

3. An aptamer probe for detecting one or more compounds from the group of the fluoroquinolones, comprising one or more different aptamers as described in either claim 1 or 2 and a marking agent.

4. A biosensor, comprising one or more different aptamers as described in either claim 1 or 2.

5. A solid phase, in particular for use with the detection, enrichment, separation and/or isolation of one or more compounds from the group of the fluoroquinolones, on which one or more different aptamers according to either claim 1 or 2 are immobilized.

6. A test strip, in particular for use in lateral flow assays, comprising one or more different aptamers according to either claim 1 or 2.

7. A lateral flow assay device, comprising a test strip according to claim 6.

8. A kit, comprising one or more different aptamers as described in either claim 1 or 2.

9. A measuring device for detecting compounds from the group of the fluoroquinolones, comprising one or more different aptamers according to claims 1 to 2, an aptamer probe according to claim 3, a biosensor according to claim 4 or a test strip according to claim 6.

10. Use of the aptamer as described in either claim 1 or 2 for the detection, enrichment, separation and/or isolation of one or more compounds from the group of the fluoroquinolones.

11. A method for detecting one or more compounds from the group of the fluoroquinolones, in which
a) one or more different aptamers as described in either claim 1 or 2 are brought in contact with a sample comprising one or more compounds from the group of the fluoroquinolones; and
b) a bond between the aptamer(s) and the compound(s) from the group of the fluoroquinolones is detected.

12. The method according to claim 11, which is carried out in the form of a competitive assay or a sandwich assay.

13. A method for enriching, separating and/or isolating one or more compounds from the group of the fluoroquinolones, in which
a) one or more different aptamers as described in either claim 1 or 2 are brought in contact with a sample comprising one or more compounds from the group of the fluoroquinolones, wherein a complex or complexes is or are formed from the aptamer(s) and the compound(s) from the group of the fluoroquinolones;
b) the complex(es) and the remaining sample are separated from one another; and
c) optionally the compound(s) from the group of the fluoroquinolones is/are isolated from the complex.

14. The method according to any one of claims 11 to 13, in which the aptamer is, or the aptamers are, immobilized on a solid phase.

15. Use of an aptamer probe according to claim 3, of a biosensor according to claim 4, of a solid phase according to claim 5, of a test strip according to claim 6, of a lateral flow assay device according to claim 7, of a kit according to claim 8, or of a measuring device according to claim 9 for detecting one or more compounds from the group of the fluoroquinolones or for enriching, separating and/or isolating one or more compounds from the group of the fluoroquinolones.

## Revendications

1. Aptamère d'ADN, qui est lié à un ou à plusieurs composés issus du groupe des fluoroquinolones et qui est choisi parmi le groupe constitué
a) d'un aptamère comprenant, ou constitué d', une séquence d'acide nucléique, qui est choisie parmi le groupe constitué de SEQ ID NO : 14, 20, 36, 30, 37, 33, 25, 22, 39, 27, 41, 43 et 52,
b) d'un aptamère, dont la séquence d'acide nucléique présente une similitude d'au moins 98 % avec la séquence d'acide nucléique d'un aptamère de a),
c) d'un aptamère, dans lequel jusqu'à 2 nucléotides sont substitués, délétés et/ou insérés par rapport à un aptamère a) dans le cas de la séquence avec la SEQ ID NO : 14, 20, 36, 30, 37, 33, 25, 22, 39, 27, 41, 43 ou 52,
d) d'un aptamère, dans lequel jusqu'à 20 nucléotides sont ajoutés à l'extrémité 3' et/ou à l'extrémité 5' par rapport à un aptamère de a) dans le cas de la séquence avec une des séquences SED ID NO : 14, 20, 36, 30, 37, 33, 25, 22, 39, 27, 41, 43 et 52,
e) d'un fragment d'un aptamère selon a), b) ou c), qui présente au moins 30 nucléotides, et
f) d'un dérivé d'un aptamère selon a), b), c), d) ou e), dans lequel un aptamère selon a), b), c), d) ou e) est modifié chimiquement au niveau de la nucléobase, au niveau du pentose ou au niveau de résidus de phosphate et présente une structure chimique, qui est absente dans l'ADN ou l'ARN naturel.

2. Aptamère selon la revendication 1, dans lequel l'aptamère selon le point b) à f) présente un ou plusieurs des motifs ATACCAGCTTATTCAATT (SEQ ID NO : 83), ACAATCGTAATCAGTTAG (SEQ ID NO : 84) et TGAGGCTCGATC (SEQ ID NO : 85) sous une forme inchangée.

3. Sonde d'aptamère servant à détecter un ou plusieurs composés issus du groupe des fluoroquinolones, comprenant un ou plusieurs aptamères différents tels que décrits dans l'une quelque des revendications 1-2 et un agent de marquage.

4. Biocapteur, présentant un ou plusieurs aptamères différents tels que décrits dans l'une quelconque des revendications 1-2.

5. Phase solide, en particulier destinée à être utilisée pour démontrer la présence d'un ou plusieurs composés, pour enrichir, séparer et/ou isoler un ou plusieurs composés issus du groupe des fluoroquinolones, au niveau desquels un ou plusieurs aptamères différents selon l'une quelconque des revendications 1-2 est/sont immobilisé(s).

6. Bandelette de test, en particulier destinée à être utilisée dans des essais à flux latéral, laquelle contient un ou plusieurs aptamères différents selon l'une quelconque des revendications 1-2.

7. Dispositif d'essai à flux latéral, qui présente une bandelette de test selon la revendication 6.

8. Kit comprenant un ou plusieurs aptamères différents tels que décrits dans l'une quelconque des revendications 1-2.

9. Appareil de mesure servant à démontrer la présence de composés issus du groupe des fluoroquinolones, présentant un ou plusieurs aptamères différents selon la revendication 1-2, une sonde d'aptamère selon la revendication 3, un biocapteur selon la revendication 4 ou une bandelette de test selon la revendication 6.

10. Utilisation de l'aptamère tel que décrit dans l'une quelconque des revendications 1-2 servant à démontrer la présence d'un ou de plusieurs composés issus du groupe des fluoroquinolones, à enrichir, séparer et/ou isoler un ou plusieurs composés issus du groupe des fluoroquinolones.

11. Procédé servant à démontrer la présence d'un ou de plusieurs composés issus du groupe des fluoroquinolones, dans lequel
a) un ou plusieurs aptamères différents tels que décrits dans l'une quelconque des revendications 1-2 est/sont amenés en contact avec un échantillon, qui contient un ou plusieurs composés issus du groupe des fluoroquinolones, et
b) la présence d'une liaison entre l'aptamère/les aptamères et le composé/les composés issus du groupe des fluoroquinolones est démontrée.

12. Procédé selon la revendication 11, qui est mis en oeuvre sous la forme d'un essai compétitif ou d'un essai sandwich.

13. Procédé servant à enrichir, séparer et/ou isoler un ou plusieurs composés issus du groupe des fluoroquinolones, dans lequel
a) un ou plusieurs aptamères différents tels que décrits dans l'une quelconque des revendications 1-2 est/sont amenés en contact avec un échantillon, qui contient un ou plusieurs composés issus du groupe des fluoroquinolones, dans lequel un complexe ou des complexes composés du/des aptamère(s) et du/des composé(s) issu(s) du groupe des fluoroquinolones est/sont formé(s),
b) le/les complexe(s) et le reste de l'échantillon sont séparés les uns des autres, et
c) en option le/les composé(s) issu(s) du groupe des fluoroquinolones est/sont isolé(s) du complexe.

14. Procédé selon l'une quelconque des revendications 11-13, dans lequel le/les aptamère(s) est/sont immobilisé(s) au niveau d'une phase solide.

15. Utilisation d'une sonde d'aptamère selon la revendication 3, d'un biocapteur selon la revendication 4, d'une phase solide selon la revendication 5, d'une bandelette de test selon la revendication 6, d'un dispositif d'essai à flux latéral selon la revendication 7, d'un kit selon la revendication 8 ou d'un appareil de mesure selon la revendication 9 afin de démontrer la présence d'un ou de plusieurs composés issus du groupe des fluoroquinolones ou afin d'enrichir, de séparer et/ou d'isoler un ou plusieurs composés issus du groupe des fluoroquinolones.


### SEQUENCE LISTING

<110> Helmholtz-Zentrum für Umweltforschung GmbH - UFZ
<120> Fluorchinolon-spezifische Aptamere
<130> P14.059EP
<150> DE102013204057.1
   <151> 2013-03-08
<160> 87
<170> PatentIn version 3.5
<210> 1
   <211> 97
   <212> DNA
   <213> Artificial Sequence
<220>
   <223> DNA-Aptamer
<400> 1
   ataccagctt attcaattcg atggtaagtg aggttcgtcc ctttaataaa ctcgattagg      60
   atctcgtgag gtgtgctcta caatcgtaat cagttag                                97
<210> 2
   <211> 96
   <212> DNA
   <213> Artificial Sequence
<220>
   <223> DNA-Aptamer
<400> 2
   ataccagctt attcaattcg atggtaagtg aggttcgtcc ctttaataaa ctcgattagg      60
   atctcgtgag gtgtgctcta caatcgtaat cagtag                                 96
<210> 3
   <211> 97
   <212> DNA
   <213> Artificial Sequence
<220>
   <223> DNA-Aptamer
<220>
   <221> misc_feature
   <222> (96)..(96)
   <223> n is a, c, g, t or u
<400> 3
   ataccagctt attcaattcg atggtaagtg aggttcgtcc ctttaataaa ctcgattagg 60
   atctcgtgag gtgtgctcta caatcgtaat cagttng 97
<210> 4
   <211> 97
   <212> DNA
   <213> Artificial Sequence
<220>
   <223> DNA-Aptamer
<400> 4
   ataccagctt attcaattcg atggtaagtg aggttcgtcc ctttaataaa ctcgattagg 60
   atctcgtgag gtgtgctcca caatcgtaat cagttag 97
<210> 5
   <211> 97
   <212> DNA
   <213> Artificial Sequence
<220>
   <223> DNA-Aptamer
<400> 5
   ataccagctt attcaattcg atggtaagtg aggttcgtcc ctttaataaa ctcgattagg 60
   atctcgtgag gtgtgcttta caatcgtaat cagttag 97
<210> 6
   <211> 97
   <212> DNA
   <213> Artificial Sequence
<220>
   <223> DNA-Aptamer
<400> 6
   ataccagctt attcaattcg atggtaagtg aggttcgtcc ctttaataaa ctcgattaag 60
   atctcgtgag gtgtgctcta caatcgtaat cagttag 97
<210> 7
   <211> 97
   <212> DNA
   <213> Artificial Sequence
<220>
   <223> DNA-Aptamer
<400> 7
   ataccagctt attcaattcg atggtaagtg aggttcgtcc ctttaataaa ctcgattagg 60
   atctcgtgag gtgtgttcca caatcgtaat cagttag 97
<210> 8
   <211> 96
   <212> DNA
   <213> Artificial Sequence
<220>
   <223> DNA-Aptamer
<400> 8
   ataccagctt attcaattcg atggtattga ggttcgtccc tttaataaac tcgattagga 60
   tctcgtgagg tgtgctctac aatcgtaatc agttag 96
<210> 9
   <211> 97
   <212> DNA
   <213> Artificial Sequence
<220>
   <223> DNA-Aptamer
<220>
   <221> misc_feature
   <222> (96)..(96)
   <223> n is a, c, g, t or u
<400> 9
   ataccagctt attcaattag atggtaagtg aggttcgtcc ctttaataaa ctcgattagg 60
   atctcgtgag gtgtgctcta caatcgtaat cagttng 97
<210> 10
   <211> 99
   <212> DNA
   <213> Artificial Sequence
<220>
   <223> DNA-Aptamer
<400> 10
   ataccagctt attcaattcg atggtaagtg aggttcgtcc cttttaataa aactcgatta 60
   ggatctcgtg aggtgtgctc tacaatcgta atcagttag 99
<210> 11
   <211> 98
   <212> DNA
   <213> Artificial Sequence
<220>
   <223> DNA-Aptamer
<400> 11
   ataccagctt attcaattcg atggtaagtg aggttcgtcc ctttaataaa ctcgattagg 60
   atctcgtgag gtgtgctcta caatccgtaa tcagttag 98
<210> 12
   <211> 97
   <212> DNA
   <213> Artificial Sequence
<220>
   <223> DNA-Aptamer
<400> 12
   ataccagctt attcaattcg atggtaagtg aggttcgtcc ctttaataaa ctcgattagg 60
   atctcgtgag gtgtgctcta caatcgtaat cagttag 97
<210> 13
   <211> 97
   <212> DNA
   <213> Artificial Sequence
<220>
   <223> DNA-Aptamer
<400> 13
   ataccagctt attcaattgg atggtaagtg aggttcgtcc ctttaataaa ctcgattagg 60
   atctcgtgag gtgtgctcta caatcgtaat cagttag 97
<210> 14
   <211> 98
   <212> DNA
   <213> Artificial Sequence
<220>
   <223> DNA-Aptamer
<220>
   <221> misc_feature
   <222> (19)..(19)
   <223> n is a, c, g, t or u
<220>
   <221> mutation
   <222> (27)..(27)
   <223> deletion
<220>
   <221> mutation
   <222> (38)..(38)
   <223> deletion
<400> 14
   ataccagctt attcaattng atggtaaktg aggytcgatc cctttaataa actcgattar 60
   gatctcgtga ggtgtgytyy acaatcgtaa tcagttag 98
<210> 15
   <211> 98
   <212> DNA
   <213> Artificial Sequence
<220>
   <223> DNA-Aptamer
<400> 15
   ataccagctt attcaattgc agggtatctg aggcttgatc tactaaatgt cgtggggcat 60
   tgctattggc gttgatacgt acaatcgtaa tcagttag 98
<210> 16
   <211> 98
   <212> DNA
   <213> Artificial Sequence
<220>
   <223> DNA-Aptamer
<400> 16
   ataccagctt attcaattgc agggtatctg aggcttgatc tactaaatgt cgtgaggcat 60
   tgctattggc gttgacacgt acaatcgtaa tcagttag 98
<210> 17
   <211> 98
   <212> DNA
   <213> Artificial Sequence
<220>
   <223> DNA-Aptamer
<400> 17
   ataccagctt attcaattgc agggtatctg aggcttgatc tactaaatgt cgtggggcat 60
   tgctattgac gttgacacgt acaatcgtaa tcagttag 98
<210> 18
   <211> 100
   <212> DNA
   <213> Artificial Sequence
<220>
   <223> DNA-Aptamer
<400> 18
   ataccagctt attcaattgc agggtatctg aggcttgatc tactaaaatg tcgtggaggc 60
   attgctattg gcgttgacac gtacaatcgt aatcagttag 100
<210> 19
   <211> 98
   <212> DNA
   <213> Artificial Sequence
<220>
   <223> DNA-Aptamer
<400> 19
   ataccagctt attcaattgc agggtatctg aggcttgatc tactaaatgt cgtggggcat 60
   tactattggc gttgacacgt acaatcgtaa tcagttag 98
<210> 20
   <211> 98
   <212> DNA
   <213> Artificial Sequence
<220>
   <223> DNA-Aptamer
<400> 20
   ataccagctt attcaattgc agggtatctg aggctygatc tactaaatgt cgtgrggcat 60
   trctattgrc gttgayacgt acaatcgtaa tcagttag 98
<210> 21
   <211> 98
   <212> DNA
   <213> Artificial Sequence
<220>
   <223> DNA-Aptamer
<400> 21
   ataccagctt attcaattag ttgtgtattg aggtttgatc taggcatagt caacagagca 60
   cgatcgatct ggcttgttct acaatcgtaa tcagttag 98
<210> 22
   <211> 98
   <212> DNA
   <213> Artificial Sequence
<220>
   <223> DNA-Aptamer
<400> 22
   ataccagctt attcaattag ttgtgtattg aggytygatc taggcatagt caacagagca 60
   cgatcgatct ggcttgttct acaatcgtaa tcagttag 98
<210> 23
   <211> 98
   <212> DNA
   <213> Artificial Sequence
<220>
   <223> DNA-Aptamer
<400> 23
   ataccagctt attcaattcc cgccagggtg aggttcgatc tgatggctgg ttctggtatt 60
   cgctatgttt ccccgtttgt acaatcgtaa tcagttag 98
<210> 24
   <211> 101
   <212> DNA
   <213> Artificial Sequence
<220>
   <223> DNA-Aptamer
<400> 24
   ataccagctt attcaattcc cgcccagggt gaggtttcga tctgatggct ggttctggta        60
   ttcgctatgt ttccccgttt tgtacaatcg taatcagtta g                           101
<210> 25
   <211> 98
   <212> DNA
   <213> Artificial Sequence
<220>
   <223> DNA-Aptamer
<400> 25
   ataccagctt attcaattcc cgccagggtg aggytcgatc tgatggctgg ttctggtatt        60
   cgctatgttt ccccgtttgt acaatcgtaa tcagttag                                98
<210> 26
   <211> 98
   <212> DNA
   <213> Artificial Sequence
<220>
   <223> DNA-Aptamer
<400> 26
   ataccagctt attcaattgg ccaggtattg aggttcgatc tgtggctcgt aagctgtctg        60
   ccatgctagc attatcgggt acaatcgtaa tcagttag                                98
<210> 27
   <211> 98
   <212> DNA
   <213> Artificial Sequence
<220>
   <223> DNA-Aptamer
<400> 27
   ataccagctt attcaattgg ccaggtattg aggytcgatc tgtggctcgt aagctgtctg        60
   ccatgctagc attatcgggt acaatcgtaa tcagttag                                98
<210> 28
   <211> 97
   <212> DNA
   <213> Artificial Sequence
<220>
   <223> DNA-Aptamer
<400> 28
   ataccagctt attcaattgg ccacaatttg aggctgatcg cattttgctc gattgaggct 60
   gttactgagg cgttcttgta caatcgtaat cagttag 97
<210> 29
   <211> 97
   <212> DNA
   <213> Artificial Sequence
<220>
   <223> DNA-Aptamer
<400> 29
   ataccagctt attcaattgt ccacaatttg aggctgatcg cattttgctc gattgaggct 60
   gttactgagg cgttcttgta caatcgtaat cagttag 97
<210> 30
   <211> 98
   <212> DNA
   <213> Artificial Sequence
<220>
   <223> DNA-Aptamer
<220>
   <221> mutation
   <222> (36)..(36)
   <223> deletion
<400> 30
   ataccagctt attcaattgk ccacaatttg aggctcgatc gcattttgct cgattgaggc 60
   tgttactgag gcgttcttgt acaatcgtaa tcagttag 98
<210> 31
   <211> 98
   <212> DNA
   <213> Artificial Sequence
<220>
   <223> DNA-Aptamer
<400> 31
   ataccagctt attcaattaa gcgggggatg aggttcgatc tagtttaggc tcgttacata 60
   gtgtttcaag tgttcggtcg acaatcgtaa tcagttag 98
<210> 32
   <211> 100
   <212> DNA
   <213> Artificial Sequence
<220>
   <223> DNA-Aptamer
<400> 32
   ataccagctt attcaattaa gcgggggagt gaggttcgat ctagtttagg tctcgttaca 60
   tagtgtttca agtgttcggt cgacaatcgt aatcagttag 100
<210> 33
   <211> 98
   <212> DNA
   <213> Artificial Sequence
<220>
   <223> DNA-Aptamer
<400> 33
   ataccagctt attcaattaa gcgggggatg aggytcgatc tagtttaggc tcgttacata 60
   gtgtttcaag tgttcggtcg acaatcgtaa tcagttag 98
<210> 34
   <211> 98
   <212> DNA
   <213> Artificial Sequence
<220>
   <223> DNA-Aptamer
<400> 34
   ataccagctt attcaattca ggaggctctg aggttcgatc ctactggctg atagcttggt 60
   gtcggttgca ttcaccgtgg acaatcgtaa tcagttag 98
<210> 35
   <211> 98
   <212> DNA
   <213> Artificial Sequence
<220>
   <223> DNA-Aptamer
<400> 35
   ataccagctt attcaattca ggaggctctg aggttcgatc ctactggctg atagcttggt 60
   gtcggttgca ttcaccgtgc acaatcgtaa tcagttag 98
<210> 36
   <211> 98
   <212> DNA
   <213> Artificial Sequence
<220>
   <223> DNA-Aptamer
<400> 36
   ataccagctt attcaattca ggaggctctg aggytcgatc ctactggctg atagcttggt 60
   gtcggttgca ttcaccgtgs acaatcgtaa tcagttag 98
<210> 37
   <211> 98
   <212> DNA
   <213> Artificial Sequence
<220>
   <223> DNA-Aptamer
<400> 37
   ataccagctt attcaattag aggtgagatg aggctcgatc tccaaccttc tgtgaaatgc 60
   ggccggaagg gtatgtttcg acaatcgtaa tcagttag 98
<210> 38
   <211> 97
   <212> DNA
   <213> Artificial Sequence
<220>
   <223> DNA-Aptamer
<400> 38
   ataccagctt attcaattca ggtggtgtta ggcttgatct gccgatctgt cgaggctcgg 60
   gtttaagctc cgcttttgta caatcgtaat cagttag 97
<210> 39
   <211> 98
   <212> DNA
   <213> Artificial Sequence
<220>
   <223> DNA-Aptamer
<220>
   <221> mutation
   <222> (30)..(30)
   <223> deletion
<400> 39
   ataccagctt attcaattca ggtggtgttg aggctygatc tgccgatctg tcgaggctcg 60
   ggtttaagct ccgcttttgt acaatcgtaa tcagttag 98
<210> 40
   <211> 98
   <212> DNA
   <213> Artificial Sequence
<220>
   <223> DNA-Aptamer
<400> 40
   ataccagctt attcaattca caaatgactg aggcttgatc atgtggttcg atctgagtat 60
   ctactgcatt tgaatggtgt acaatcgtaa tcagttag 98
<210> 41
   <211> 98
   <212> DNA
   <213> Artificial Sequence
<220>
   <223> DNA-Aptamer
<400> 41
   ataccagctt attcaattca caaatgactg aggctygatc atgtggttcg atctgagtat 60
   ctactgcatt tgaatggtgt acaatcgtaa tcagttag 98
<210> 42
   <211> 98
   <212> DNA
   <213> Artificial Sequence
<220>
   <223> DNA-Aptamer
<400> 42
   ataccagctt attcaattag ggtcgatatg aggcttgatc ttatcttgtt actggtcgaa 60
   ctgtttgtta ctgcgtgcat acaatcgtaa tcagttag 98
<210> 43
   <211> 98
   <212> DNA
   <213> Artificial Sequence
<220>
   <223> DNA-Aptamer
<400> 43
   ataccagctt attcaattag ggtcgatatg aggctygatc ttatcttgtt actggtcgaa 60
   ctgtttgtta ctgcgtgcat acaatcgtaa tcagttag 98
<210> 44
   <211> 98
   <212> DNA
   <213> Artificial Sequence
<220>
   <223> DNA-Aptamer
<400> 44
   ataccagctt attcaattgg tcgaaaagtg aggcttgatc taagtagtgg cctaacattg 60
   atttcgtcgt gaggcattct acaatcgtaa tcagttag 98
<210> 45
   <211> 98
   <212> DNA
   <213> Artificial Sequence
<220>
   <223> DNA-Aptamer
<400> 45
   ataccagctt attcaattgg tcgaaaagtg aggcttgatc taagtagtgg cctaacattg 60
   atttcgtcgt gaggtattct acaatcgtaa tcagttag 98
<210> 46
   <211> 98
   <212> DNA
   <213> Artificial Sequence
<220>
   <223> DNA-Aptamer
<400> 46
   ataccagctt attcaattgg tcgaaaagtg aggctygatc taagtagtgg cctaacattg 60
   atttcgtcgt gaggyattct acaatcgtaa tcagttag 98
<210> 47
   <211> 98
   <212> DNA
   <213> Artificial Sequence
<220>
   <223> DNA-Aptamer
<400> 47
   ataccagctt attcaattgt atccagagtg aggatcgatc aggttgtgag gctcggatat 60
   catctgtcat tctctggcat acaatcgtaa tcagttag 98
<210> 48
   <211> 98
   <212> DNA
   <213> Artificial Sequence
<220>
   <223> DNA-Aptamer
<400> 48
   ataccagctt attcaattgt atccagagtg aggmtcgatc aggttgtgag gctcggatat 60
   catctgtcat tctctggcat acaatcgtaa tcagttag 98
<210> 49
   <211> 98
   <212> DNA
   <213> Artificial Sequence
<220>
   <223> DNA-Aptamer
<400> 49
   ataccagctt attcaattgc aggtggtatg agactcgatc actgttattt gtctttggct 60
   cgatcccgat ccggactggc acaatcgtaa tcagttag 98
<210> 50
   <211> 98
   <212> DNA
   <213> Artificial Sequence
<220>
   <223> DNA-Aptamer
<400> 50
   ataccagctt attcaattac aggtggtatg agactcgatc actgttattt gtctttggct 60
   cgatcccgat ccggactggt acaatcgtaa tcagttag 98
<210> 51
   <211> 98
   <212> DNA
   <213> Artificial Sequence
<220>
   <223> DNA-Aptamer
<400> 51
   ataccagctt attcaattrc aggtggtatg agrctcgatc actgttattt gtctttggct 60
   cgatcccgat ccggactggy acaatcgtaa tcagttag 98
<210> 52
   <211> 98
   <212> DNA
   <213> Artificial Sequence
<220>
   <223> DNA-Aptamer
<400> 52
   ataccagctt attcaattcc atattgtatg aggctcgatc aagtatggac tttgaggcgt 60
   ggatcttctg atttctttgc acaatcgtaa tcagttag 98
<210> 53
   <211> 98
   <212> DNA
   <213> Artificial Sequence
<220>
   <223> DNA-Aptamer
<400> 53
   ataccagctt attcaattcc cattgggatg aggcttgatc agtttgtgag gctactggct 60
   tggtcgtatg cactggttcg acaatcgtaa tcagttag 98
<210> 54
   <211> 98
   <212> DNA
   <213> Artificial Sequence
<220>
   <223> DNA-Aptamer
<400> 54
   ataccagctt attcaattcc cattgggatg aggctygatc agtttgtgag gctactggct 60
   tggtcgtatg cactggttcg acaatcgtaa tcagttag 98
<210> 55
   <211> 98
   <212> DNA
   <213> Artificial Sequence
<220>
   <223> DNA-Aptamer
<400> 55
   ataccagctt attcaattcc gaccaggatg aggcttgatc gtgtgagagt gagtggaggc 60
   gtggattatg ttgttattgt acaatcgtaa tcagttag 98
<210> 56
   <211> 99
   <212> DNA
   <213> Artificial Sequence
<220>
   <223> DNA-Aptamer
<400> 56
   ataccagctt attcaattcc gaccaggatg aggcttgact cgtgtgagag tgagtggagg 60
   cgtggattat gttgttattg tacaatcgta atcagttag 99
<210> 57
   <211> 98
   <212> DNA
   <213> Artificial Sequence
<220>
   <223> DNA-Aptamer
<400> 57
   ataccagctt attcaattcc gaccaggatg aggctygatc gtgtgagagt gagtggaggc 60
   gtggattatg ttgttattgt acaatcgtaa tcagttag 98
<210> 58
   <211> 98
   <212> DNA
   <213> Artificial Sequence
<220>
   <223> DNA-Aptamer
<400> 58
   ataccagctt attcaattgt atccagagtg aggatcgatt aggttgtgag gctcggatat 60
   catctgtcat tctctggcat acaatcgtaa tcagttag 98
<210> 59
   <211> 99
   <212> DNA
   <213> Artificial Sequence
<220>
   <223> DNA-Aptamer
<400> 59
   ataccagctt attcaattgt atccagagtg gaggatcgat caggttgtga ggctcggata 60
   tcatctgtca ttctctggca tacaatcgta atcagttag 99
<210> 60
   <211> 98
   <212> DNA
   <213> Artificial Sequence
<220>
   <223> DNA-Aptamer
<400> 60
   ataccagctt attcaattgt atccagagtg aggmtcgaty agtttgtgag gctcggatat 60
   catctgtcat tctctggcat acaatcgtaa tcagttag 98
<210> 61
   <211> 98
   <212> DNA
   <213> Artificial Sequence
<220>
   <223> DNA-Aptamer
<400> 61
   ataccagctt attcaattag ggtcgatctg aggctccatc acttctaata tgtagcgtct 60
   acgtggttgt tcctagtcgg acaatcgtaa tcagttag 98
<210> 62
   <211> 98
   <212> DNA
   <213> Artificial Sequence
<220>
   <223> DNA-Aptamer
<400> 62
   ataccagctt attcaattag ggtcgatctg aggctcsatc acttctaata tgtagcgtct 60
   acgtggttgt tcctagtcgg acaatcgtaa tcagttag 98
<210> 63
   <211> 98
   <212> DNA
   <213> Artificial Sequence
<220>
   <223> DNA-Aptamer
<400> 63
   ataccagctt attcaattgg aggcacgatg aggctcgatc gcctgtcaac tgataaagcc 60
   tgtgctgctg tcgttttggt acaatcgtaa tcagttag 98
<210> 64
   <211> 99
   <212> DNA
   <213> Artificial Sequence
<220>
   <223> DNA-Aptamer
<400> 64
   ataccagctt attcaattgg aggtacgatg aggctcgatc gcctgtcaac tgataaagcc 60
   tgtgctgctg tcgtttttgg tacaatcgta atcagttag 99
<210> 65
   <211> 98
   <212> DNA
   <213> Artificial Sequence
<220>
   <223> DNA-Aptamer
<400> 65
   ataccagctt attcaattac aggtggtatg aggctcgatc actgttattt gtctttggct 60
   cgatcccgat ccggactggc acaatcgtaa tcagttag 98
<210> 66
   <211> 98
   <212> DNA
   <213> Artificial Sequence
<220>
   <223> DNA-Aptamer
<400> 66
   ataccagctt attcaattag tccaatactg aggcttgatc tggcgagata aataactgtg        60
   ctactattcg ttgcgtcccc acaatcgtaa tcagttag                                98
<210> 67
   <211> 98
   <212> DNA
   <213> Artificial Sequence
<220>
   <223> DNA-Aptamer
<400> 67
   ataccagctt attcaattag tccaatactg aggctygatc tggcgagata aataactgtg        60
   ctactattcg ttgcgtcccc acaatcgtaa tcagttag                                98
<210> 68
   <211> 98
   <212> DNA
   <213> Artificial Sequence
<220>
   <223> DNA-Aptamer
<400> 68
   ataccagctt attcaattcg cgttgaattg aggcttgatc tgaatggctc gtgtatctat        60
   ttggtattgg ttttgtttgt acaatcgtaa tcagttag                                98
<210> 69
   <211> 98
   <212> DNA
   <213> Artificial Sequence
<220>
   <223> DNA-Aptamer
<400> 69
   ataccagctt attcaattcg cgttgaattg aggctygatc tgaatggctc gtgtatctat        60
   ttggtattgg ttttgtttgt acaatcgtaa tcagttag                                98
<210> 70
   <211> 98
   <212> DNA
   <213> Artificial Sequence
<220>
   <223> DNA-Aptamer
<400> 70
   ataccagctt attcaattcc cgccagggtg aggttcgatc tgatggctgg ttctggtatt 60
   cgctatgttt ccccgtttgt acaatcgtaa tcagttag 98
<210> 71
   <211> 98
   <212> DNA
   <213> Artificial Sequence
<220>
   <223> DNA-Aptamer
<400> 71
   ataccagctt attcaattcg cgccagggtg aggttcgatc tgatggctgg ttctggtatt 60
   cgctatgttt ccccgtttgt acaatcgtaa tcagttag 98
<210> 72
   <211> 98
   <212> DNA
   <213> Artificial Sequence
<220>
   <223> DNA-Aptamer
<400> 72
   ataccagctt attcaattcs cgccagggtg aggytcgatc tgatggctgg ttctggtatt 60
   cgctatgttt ccccgtttgt acaatcgtaa tcagttag 98
<210> 73
   <211> 100
   <212> DNA
   <213> Artificial Sequence
<220>
   <223> DNA-Aptamer
<400> 73
   ataccagctt attcaattgg tctggcattg aggctcaatc tgttgctcga tttggagtgg 60
   ctcgtagtta gggctctttt ctacaatcgt aatcagttag 100
<210> 74
   <211> 100
   <212> DNA
   <213> Artificial Sequence
<220>
   <223> DNA-Aptamer
<400> 74
   ataccagctt attcaattgg tctggcattg aggctcratc tgttgctcga tttggagtgg        60
   ctcgtagtta gggctctttt ctacaatcgt aatcagttag                             100
<210> 75
   <211> 99
   <212> DNA
   <213> Artificial Sequence
<220>
   <223> DNA-Aptamer
<220>
   <221> misc_feature
   <222> (59)..(59)
   <223> n is a, c, g, t or u
<400> 75
   ataccagctt attcaattgg atgggaggtg aggcttgatc tatgagggtc gtggtggcnt        60
   ttattatggc tgtactcatg gacaatcgta atcagttag                               99
<210> 76
   <211> 99
   <212> DNA
   <213> Artificial Sequence
<220>
   <223> DNA-Aptamer
<220>
   <221> misc_feature
   <222> (59)..(59)
   <223> n is a, c, g, t or u
<400> 76
   ataccagctt attcaattgg atgggaggtg aggctygatc tatgagggtc gtggtggcnt        60
   ttattatggc tgtactcatg gacaatcgta atcagttag                               99
<210> 77
   <211> 100
   <212> DNA
   <213> Artificial Sequence
<220>
   <223> DNA-Aptamer
<400> 77
   ataccagctt attcaattcg agggggagat gaggctcgat cacctttcga ttaattgctc 60
   gatctagatg tgtacgtttg ctacaatcgt aatcagttag 100
<210> 78
   <211> 101
   <212> DNA
   <213> Artificial Sequence
<220>
   <223> DNA-Aptamer
<400> 78
   ataccagctt attcaattca cagcaatttt gaggttcgat cgcggaggat aggctcggtc 60
   aatcctgggg tagtttttgt tgtacaatcg taatcagtta g 101
<210> 79
   <211> 100
   <212> DNA
   <213> Artificial Sequence
<220>
   <223> DNA-Aptamer
<400> 79
   ataccagctt attcaattgc agggtcgata tgaggcttga tctactaaat gtcgtgaggc 60
   attgctattg gcgttgacac gtacaatcgt aatcagttag 100
<210> 80
   <211> 100
   <212> DNA
   <213> Artificial Sequence
<220>
   <223> DNA-Aptamer
<400> 80
   ataccagctt attcaattgc agggtcgata tgaggctyga tctactaaat gtcgtgaggc 60
   attgctattg gcgttgacac gtacaatcgt aatcagttag 100
<210> 81
   <211> 98
   <212> DNA
   <213> Artificial Sequence
<220>
   <223> DNA-Aptamer
<400> 81
   ataccagctt attcaattgg gatggtattg aggcttgatc tactaaatgt cgtggggcat 60
   tgctattggc gttgatacgt acaatcgtaa tcagttag 98
<210> 82
   <211> 98
   <212> DNA
   <213> Artificial Sequence
<220>
   <223> DNA-Aptamer
<400> 82
   ataccagctt attcaattgg gatggtattg aggctygatc tactaaatgt cgtggggcat 60
   tgctattggc gttgatacgt acaatcgtaa tcagttag 98
<210> 83
   <211> 18
   <212> DNA
   <213> Artificial Sequence
<220>
   <223> Sense Primersequenz
<400> 83
   ataccagctt attcaatt 18
<210> 84
   <211> 18
   <212> DNA
   <213> Artificial Sequence
<220>
   <223> komplementäre Sequenz zu Antisense-Primersequenz
<400> 84
   acaatcgtaa tcagttag 18
<210> 85
   <211> 12
   <212> DNA
   <213> Artificial Sequence
<220>
   <223> komplementäre Sequenz zu Fängersequenz für Capture SELEX
<400> 85
   tgaggctcga tc 12
<210> 86
   <211> 18
   <212> DNA
   <213> Artificial Sequence
<220>
   <223> Antisense-Primersequenz
<400> 86
   ctaactgatt acgattgt 18
<210> 87
   <211> 12
   <212> DNA
   <213> Artificial Sequence
<220>
   <223> Fängersequenz für Capture SELEX
<400> 87
   gatcgagcct ca 12